# EUROPEAN PATENT SPECIFICATION

(11) **EP 3 019 005 B1**
(45) Date of publication and mention of the grant of the patent: **20.02.2019**
(21) Application number: 14822448.8
(22) Date of filing: 09.07.2014
(51) Int. Cl.: A01K 67/027

(54) **MRAP2 KNOCKOUTS**
MRAP2-KNOCKOUTS
INACTIVATIONS DE MRAP2

(30) Priority: 10.07.2013 US 201361844666 P
(43) Date of publication of application: 18.05.2016
(73) Proprietor: EffStock, LLC, Boston, MA 02116 (US)
(72) Inventor: MAJZOUB Joseph A., Boston, MA 02116 (US); ASAI Masato, Nagoya, Aichi 468-0075 (JP)
(74) Representative: Sweetinburgh, Mark Roger
(86) International application number: PCT/US2014/045934
(87) International publication number: WO 2015/006437

(56) References cited:
- US-A1- 2008 299 042
- US-A1- 2010 120 627
- L. F. CHAN ET AL: "MRAP and MRAP2 are bidirectional regulators of the melanocortin receptor family", PROCEEDINGS OF THE NATIONAL ACADEMY OF SCIENCES, vol. 106, no. 15, 14 April 2009 (2009-04-14), pages 6146-6151, XP055306434, US ISSN: 0027-8424, DOI: 10.1073/pnas.0809918106
- GI-WOOK HWANG ET AL: "siRNA-mediated knockdown of the melanocortin 2 receptor accessory protein 2 (MRAP2) gene confers resistance to methylmercury on HEK293 cells", JOURNAL OF TOXICOLOGICAL SCIENCES., vol. 35, no. 6, 2010, pages 947-950, XP055318504, JP ISSN: 0388-1350, DOI: 10.2131/jts.35.947
- J. A. SEBAG ET AL: "Opposite Effects of the Melanocortin-2 (MC2) Receptor Accessory Protein MRAP on MC2 and MC5 Receptor Dimerization and Trafficking", JOURNAL OF BIOLOGICAL CHEMISTRY, vol. 284, no. 34, 17 June 2009 (2009-06-17), pages 22641-22648, XP055306427, US ISSN: 0021-9258, DOI: 10.1074/jbc.M109.022400
- R. J. GORRIGAN ET AL: "Localisation of the melanocortin-2-receptor and its accessory proteins in the developing and adult adrenal gland", JOURNAL OF MOLECULAR ENDOCRINOLOGY., vol. 46, no. 3, 2 March 2011 (2011-03-02), pages 227-232, XP055306435, GB ISSN: 0952-5041, DOI: 10.1530/JME-11-0011
- TOM R. WEBB ET AL: "Minireview: The Melanocortin 2 Receptor Accessory Proteins", MOLECULAR ENDOCRINOLOGY, vol. 24, no. 3, March 2010 (2010-03), pages 475-484, XP055318885, US ISSN: 0888-8809, DOI: 10.1210/me.2009-0283
- LI F CHAN ET AL: "Effects of melanocortins on adrenal gland physiology", EUROPEAN JOURNAL OF PHARMACOLOGY, ELSEVIER SCIENCE, NL, vol. 660, no. 1, 25 November 2010 (2010-11-25), pages 171-180, XP028208937, ISSN: 0014-2999, DOI: 10.1016/J.EJPHAR.2010.11.041 [retrieved on 2011-01-03]
- CHUANXIAN WEI ET AL: "TALEN or Cas9 - Rapid, Efficient and Specific Choices for Genome Modifications", JOURNAL OF GENETICS AND GENOMICS, vol. 40, no. 6, June 2013 (2013-06), pages 281-289, XP055071686, ISSN: 1673-8527, DOI: 10.1016/j.jgg.2013.03.013
- J. HAUSCHILD ET AL: "Efficient generation of a biallelic knockout in pigs using zinc-finger nucleases", PROCEEDINGS OF THE NATIONAL ACADEMY OF SCIENCES, vol. 108, no. 29, 19 July 2011 (2011-07-19), pages 12013-12017, XP055007649, ISSN: 0027-8424, DOI: 10.1073/pnas.1106422108
- M. ASAI ET AL: "Loss of Function of the Melanocortin 2 Receptor Accessory Protein 2 Is Associated with Mammalian Obesity", SCIENCE, vol. 341, no. 6143, 19 July 2013 (2013-07-19), pages 275-278, XP055318468, US ISSN: 0036-8075, DOI: 10.1126/science.1233000
- SHWETHA RAMACHANDRAPPA ET AL: "The melanocortin receptors and their accessory proteins", FRONTIERS IN ENDOCRINOLOGY, vol. 4, February 2013 (2013-02), XP055318577, DOI: 10.3389/fendo.2013.00009
- Randall S Prather ET AL: "Genetically Modified Pigs for Medicine and Agriculture" In: "Biotechnology and Genetic Engineering Reviews", 2008, Nottingham University Press, XP055050381, vol. 25, pages 245-266, * the whole document *
- SEBAG ET AL.: 'Opposite Effects of the Melanocortin-2 ( MC 2) Receptor Accessory Protein MRAP on MC 2 and MC 5 Receptor Dimerization and Trafficking' J. BIOL. CHEM. vol. 284, 17 June 2009, pages 22641 - 22648, XP055306427
- LIU ET AL.: 'A Highly Efficient Recombineering-Based Method for Generating Conditional Knockout Mutations' GENOME RESEARCH vol. 13, 01 March 2003, pages 476 - 484, XP002376686
- CHAN ET AL.: 'MRAP and MRAP2 are bidirectional regulators of the melanocortin receptor family' PNAS vol. 16, no. 15, 14 April 2009, pages 6146 - 6151, XP055306434
- GORRIGAN ET AL.: 'Localisation of the melanocortin-2-receptor and its accessory proteins in the developing and adult adrenal gland' JOURNAL OF MOLECULAR ENDOCRINOLOGY vol. 46, 09 June 2011, pages 227 - 232, XP055306435

### SEQUENCE LISTING

<110> MAJZOUB, JOSEPH A. ASAI, MASATO
<120> MRAP2 KNOCKOUTS
<130> 068570-078611-PCT
<140>
   <141>
<150> 61/844,666
   <151> 2013-07-10
<160> 69
<170> PatentIn version 3.5
<210> 1
   <211> 205
   <212> PRT
   <213> Homo sapiens
<400> 1
   Met Ser Ala Gln Arg Leu Ile Ser Asn Arg Thr Ser Gln Gln Ser Ala
   1               5                   10                  15
   Ser Asn Ser Asp Tyr Thr Trp Glu Tyr Glu Tyr Tyr Glu Ile Gly Pro
               20                  25                  30
   Val Ser Phe Glu Gly Leu Lys Ala His Lys Tyr Ser Ile Val Ile Gly
           35                  40                  45
   Phe Trp Val Gly Leu Ala Val Phe Val Ile Phe Met Phe Phe Val Leu
       50                  55                  60
   Thr Leu Leu Thr Lys Thr Gly Ala Pro His Gln Asp Asn Ala Glu Ser
   65                  70                  75                  80
   Ser Glu Lys Arg Phe Arg Met Asn Ser Phe Val Ser Asp Phe Gly Arg
                   85                  90                  95
   Pro Leu Glu Pro Asp Lys Val Phe Ser Arg Gln Gly Asn Glu Glu Ser
               100                 105                 110
   Arg Ser Leu Phe His Cys Tyr Ile Asn Glu Val Glu Arg Leu Asp Arg
           115                 120                 125
   Ala Lys Ala Cys His Gln Thr Thr Ala Leu Asp Ser Asp Val Gln Leu
       130                 135                 140
   Gln Glu Ala Ile Arg Ser Ser Gly Gln Pro Glu Glu Glu Leu Asn Arg
   145                 150                 155                 160
   Leu Met Lys Phe Asp Ile Pro Asn Phe Val Asn Thr Asp Gln Asn Tyr
                   165                 170                 175
   Phe Gly Glu Asp Asp Leu Leu Ile Ser Glu Pro Pro Ile Val Leu Glu
               180                 185                 190
   Thr Lys Pro Leu Ser Gln Thr Ser His Lys Asp Leu Asp
           195                 200                 205
<210> 2
   <211> 2229
   <212> DNA
   <213> Homo sapiens
<400> 2
   tccggcgccc cgcgccgcct ccgctgcggg tcgggagcgc gcgtctccgc cgcacctcgg      60
   atctaggagc tactcgcccg gccctgggcg gtgggaggcg gcggcggcgg cggcgctcgc     120
   gcacctcgga ggagccagga gccggaacca gggccgagcc cgcgggccgg ggctagccag     180
   ccggtcggag atgtccgccc agaggttaat ttctaacaga acctcccagc aatcggcatc     240
   taattctgat tacacctggg aatatgaata ttatgagatt ggaccagttt cctttgaagg     300
   actgaaggct cataaatatt ccattgtgat tggattttgg gttggtcttg cagtcttcgt     360
   gatttttatg ttttttgtgc tgaccttgct gaccaagaca ggagccccac accaagacaa     420
   tgcagagtcc tcagagaaga gattcagaat gaacagcttt gtgtcagact ttggaagacc     480
   tctggagcca gataaagtat tttctcgcca aggcaacgag gagtccaggt ctctctttca     540
   ctgctacatc aatgaggtgg aacgcttgga cagagccaaa gcttgtcacc agaccacagc     600
   ccttgacagt gacgtccaac tccaggaagc catcagaagc agtgggcagc cagaggagga     660
   gctgaacagg ctcatgaagt ttgacatccc caactttgtg aacacagacc agaactactt     720
   tggggaggat gatcttctga tttctgaacc acctattgtt ctggaaacta agccactttc     780
   ccagacctca cacaaagacc tggattgaga aacatgctct gtaaagggtc ttcctgaaga     840
   tgtggattct atctttatgt agcaagaaat ctacatccac caaaattgtg tgtgtttggg     900
   ggagagagag acatagagat agagacagag aggcagagaa gagacccctt tagaagagag     960
   ctgagctgat taagctgagt ggttttttgt tttgttttgt ttttgctttt taatacattt    1020
   ggagctttgg gagtattaaa gtatttacac caagcttgtc caacccgtgg catgtgtccc    1080
   aggacagctt tgaatgtggc ccaatacaaa ttttaaactt tattaaaaca tgagttttgt    1140
   tttttttttt gctatttttt ttaaagctcg tcagctatcg ttagtgttag tgtactttat    1200
   gtgtggccca agacaactct tcttccagtg tggcacaggg aagctaaaag attggacacc    1260
   tctgatttat actagctcgt tttgcttgtt gaaaaatttg gccaaatacc tattgtcagc    1320
   attcttgggt gaggattagc ctaccatgtt ctaatctggc cctgccacta ctatgctcta     1380
   cctttggtga gttgctttac ctctctgggc tgccccattt ttaactgtag gttgacaggt     1440
   ctagagtgat ccatcccacc tctaatattt tgtgaattta tgactttgcc ttcagatgag     1500
   gctgagctat acataaaaca gtataaacta gggtactgcc tcgtatctct tgtaggctct     1560
   ctcaaatctc tgtaccttcc acttaaccct aattgagcca agctttagtc aggggatctg     1620
   gttgtctacc agaatgtcag gagactcatc ttacacagtc atggtggcca atgtttctgg     1680
   tgggttgtgc tgaaacagct cttctgagaa cttccaacca cccatgctct aacctggaga     1740
   cagccatccc ctgcctcaga ataagtacca attcgtagta catgtatggt actcttgtcc     1800
   ccaagaaatg ttaggaagct tgtcagctga atgagaggag gtgccttctg ggtatctctg     1860
   tgttggtgta tctgtgccat tggctacaga acaagaaaaa tactatttgc catgctatta     1920
   ccttggcaga tgtgtaggtg atagtcatct ggctttgagc tgagatggtc agtgggttgt     1980
   aaattcccca ctagcagata ttcagggtgg cctgagttat gtaaacaagt gagcaacaca     2040
   gctttaattt catggaggaa tcaaagctgc acactggtat taaaacaact tgattttgcg     2100
   cacacagttg catgcatggc aagctgttaa cctctgggtg gcattttcat tatgaatttg     2160
   ttcaccacct gtcttgctta agctacaaaa taaatgcatt tgactgcaca gaaaaaaaaa     2220
   aaaaaaaaa                                                             2229
<210> 3
   <211> 206
   <212> PRT
   <213> Sus scrofa
<400> 3
   Met Ser Ser Gln Arg Leu Ile Ser Asn Arg Thr Ser Gln Gln Ser Thr
   1               5                   10                  15
   Ser Asn Ser Asp Tyr Thr Trp Glu Tyr Glu His Tyr Glu Ile Gly Pro
               20                  25                  30
   Val Ser Phe Glu Gly Leu Lys Ala His Lys Tyr Ser Ile Val Ile Gly
           35                  40                  45
   Phe Trp Val Gly Leu Ala Val Phe Val Ile Phe Met Phe Phe Val Leu
       50                  55                  60
   Thr Leu Leu Thr Lys Thr Gly Ala Pro His Gln Asp Asn Ala Glu Ser
   65                  70                  75                  80
   Ser Glu Lys Arg Phe Arg Met Asn Ser Phe Val Ser Asp Phe Gly Arg
                   85                  90                  95
   Pro Leu Glu Pro Asp Lys Val Phe Ser Arg Gln Gly Asn Glu Glu Ser
               100                 105                 110
   Arg Ser Leu Phe His Cys Tyr Ile Asn Glu Val Asp Gln Leu Asp Lys
           115                 120                 125
   Ala Lys Ala Cys Phe Gln Thr Thr Ala Leu Asp Ser Asn Val Gln Leu
       130                 135                 140
   Gln Glu Ala Ile Arg Arg Ser Gly Gln Pro Glu Glu Glu Leu Asn Arg
   145                 150                 155                 160
   Leu Met Lys Phe Asp Ile Pro Asn Phe Val Asn Thr Asp Gln Asn Ser
                   165                 170                 175
   Ser Phe Gly Glu Asp Asp Leu Leu Ile Ser Glu Pro Pro Ile Val Leu
               180                 185                 190
   Glu Asn Lys Pro Val Ala Gln Thr Pro His Lys Asp Leu Asp
           195                 200                 205
<210> 4
   <211> 2180
   <212> DNA
   <213> Sus scrofa
<400> 4
   ttgaactcga agccattgct gaggattttt cctaatcccc atcaatgctg cctcactccc        60
   tcttgctcag ccctttgggc tcctgtcacc caaagttggt cagagagacg cactgctcag       120
   ccgagaggac aaacaattct ccccatccct ttcccttctt cgcggcaggc tctccttcag       180
   acggtgactg agcgccacgt gggcacagag ggttccgcag agctcgcctc ctcccggccc       240
   ccgcgccctg ggactgggga ggcagccgcc tccgccgcgg gtggggaacg cgcgtccccg       300
   ccgcgctgca gcgccgggag cgaccagccg gcagggctgc tgaaagcggc ggtggcggcg       360
   ctcacgcacc tcgcaggagc tggtggaaat gtcttcccag aggttaattt ctaacagaac       420
   atcccagcaa tctacatcta attctgatta cacctgggaa tatgagcatt atgagattgg       480
   acctgtttcc tttgaaggac taaaggctca caaatattcc attgtaattg gattttgggt       540
   tggtcttgcg gtcttcgtga ttttcatgtt ttttgtgctg accttgctga ccaagacagg       600
   agctccacac caagacaatg cagagtcttc agagaagaga ttccgaatga acagctttgt       660
   gtcagatttt ggaagaccac tggagccaga caaggtgttt tctcgacagg gcaatgagga       720
   atccaggtcc ctctttcatt gttacattaa tgaagtggac cagttggaca aggccaaagc       780
   ttgtttccag accacggccc tcgatagtaa tgttcagctc caagaagcca tcagacgcag       840
   tgggcagccg gaggaggagc tcaacaggct catgaaattt gacattccca actttgtcaa 900
   cacagaccag aactcctcct ttggggaaga cgatctcctg atatcggaac cacctattgt 960
   tctagaaaat aagccagttg cccagacccc acacaaagac ttggattgag aagcatgttt 1020
   tgtaaagtgt atacctgaag aggctggtct gtctttgtat agtacgaaat ctccattcac 1080
   caaaattgtg tgcgtttgta tatgagagac agagacagag agaccaaaag gcaaaatgac 1140
   agagaagaga gccccctggg aagagagctg attcagctgt ttttacgccc tcaaacacat 1200
   atggggcttt tgggaggggg agaaccaagt agttacactg tctcatcttc cttgttgttg 1260
   aagaacttgg ccacatactc ggcatcatca ctcttgaatg gggttgacat atggccttcc 1320
   tgtctgccac tgctatgctc ttcattagtg agttgctttt tctctctggg ctgccgtctt 1380
   ttttactgta aggagaggag tatggagtag atgatttgtc tcaactcgaa taatttgccg 1440
   atttatgact gtgttcagat gaggctggga tatacccaaa ggagtataaa ctagggcacc 1500
   gtatcctagc tcttgctata agatccccca gttgactttc cattcaaccc ttaactgagc 1560
   ccagcttttg tcagggtccc ggtgtctatc aggatgtcag gagactcatc tctcatggtt 1620
   tctgtggcaa catttctatt ggttgggctg aaatggctct tctgagaccc tccagccacc 1680
   cacgctcaaa gctgaggaca gccctgcccc tcctctcctg ggatgagtac caatccacaa 1740
   tacattgtgt gacagtcttg ttcccagaaa catatggaag gtcatcagcc gcgtgaggag 1800
   aggtgccttc tgtgtctctc tgttggtgta actgtgtctg gtcacactgc tatagtattg 1860
   gctagtgaac aagaaaagca ctatttgcca tgctttctgt tcacttggca gatgtgtagg 1920
   tgatggtggt gtggcttatg tgtgggcttt gagctgggat gttgaatgag ttagaattac 1980
   tccatatgca aatattcaga gtggcctggc acaacacaga gctgattcat gaatcaaaac 2040
   tgcatgttgt tttggaaaca acttggtttt gcagtcatgt ggcatgcatg gccagctgct 2100
   aatctctggt tggtgttttc attatggatt tcattatgtc ttgcttaagc tgaaaaataa 2160
   atgcatttat tgcaccagaa 2180
<210> 5
   <211> 29
   <212> DNA
   <213> Artificial Sequence
<220>
   <223> Description of Artificial Sequence: Synthetic primer
<400> 5
   gcggccgcca aacaaaaaaa ggtatttgg 29
<210> 6
   <211> 22
   <212> DNA
   <213> Artificial Sequence
<220>
   <223> Description of Artificial Sequence: Synthetic primer
<400> 6
   actagtctac agtatgtatt ac 22
<210> 7
   <211> 22
   <212> DNA
   <213> Artificial Sequence
<220>
   <223> Description of Artificial Sequence: Synthetic primer
<400> 7
   actagttact atagtatcat ta 22
<210> 8
   <211> 27
   <212> DNA
   <213> Artificial Sequence
<220>
   <223> Description of Artificial Sequence: Synthetic primer
<400> 8
   ggatccgttc ttggtcttgc tcagaaa 27
<210> 9
   <211> 26
   <212> DNA
   <213> Artificial Sequence
<220>
   <223> Description of Artificial Sequence: Synthetic primer
<400> 9
   gtcgacatgt gaagcgtgga ccaaaa 26
<210> 10
   <211> 26
   <212> DNA
   <213> Artificial Sequence
<220>
   <223> Description of Artificial Sequence: Synthetic primer
<400> 10
   gaattcattc atgttcatgt tgacac 26
<210> 11
   <211> 26
   <212> DNA
   <213> Artificial Sequence
<220>
   <223> Description of Artificial Sequence: Synthetic primer
<400> 11
   ggatccgaag tggagtaaat tcaatc 26
<210> 12
   <211> 28
   <212> DNA
   <213> Artificial Sequence
<220>
   <223> Description of Artificial Sequence: Synthetic primer
<400> 12
   gcggccgcgc acaggtgccc cgcccacc 28
<210> 13
   <211> 26
   <212> DNA
   <213> Artificial Sequence
<220>
   <223> Description of Artificial Sequence: Synthetic primer
<400> 13
   gtcgaccgcg atgtttcttt agcata 26
<210> 14
   <211> 26
   <212> DNA
   <213> Artificial Sequence
<220>
   <223> Description of Artificial Sequence: Synthetic primer
<400> 14
   gaattctcac gagggcaaaa accagg 26
<210> 15
   <211> 26
   <212> DNA
   <213> Artificial Sequence
<220>
   <223> Description of Artificial Sequence: Synthetic primer
<400> 15
   ggatccaatt aggtgttttg gttctt 26
<210> 16
   <211> 28
   <212> DNA
   <213> Artificial Sequence
<220>
   <223> Description of Artificial Sequence: Synthetic primer
<400> 16
   gcggccgcct tttcttacta ttaaaaat 28
<210> 17
   <211> 19
   <212> DNA
   <213> Artificial Sequence
<220>
   <223> Description of Artificial Sequence: Synthetic primer
<400> 17
   gcaaggcgat taagttggg 19
<210> 18
   <211> 19
   <212> DNA
   <213> Artificial Sequence
<220>
   <223> Description of Artificial Sequence: Synthetic primer
<400> 18
   ggaaacattc caggcctgg 19
<210> 19
   <211> 35
   <212> DNA
   <213> Artificial Sequence
<220>
   <223> Description of Artificial Sequence: Synthetic primer
<400> 19
   cttagctgtc cctgtggctg tcccctaaag ttggc 35
<210> 20
   <211> 98
   <212> DNA
   <213> Artificial Sequence
<220>
   <223> Description of Artificial Sequence: Synthetic primer
<400> 20
   ggatcccctc gagggaccta ataacttcgt atagcataca ttatacgaag ttatattaag 60
   ggttattgaa tatgatcgga attgggctgc aggaattc 98
<210> 21
   <211> 44
   <212> DNA
   <213> Artificial Sequence
<220>
   <223> Description of Artificial Sequence: Synthetic primer
<400> 21
   tgccactccc actgtccttt cctaataaaa tgaggaaatt gcat 44
<210> 22
   <211> 50
   <212> DNA
   <213> Artificial Sequence
<220>
   <223> Description of Artificial Sequence: Synthetic primer
<400> 22
   accaacatcc ctgtgatgca cctgtagcct gctggactgg ctgaacagaa 50
<210> 23
   <211> 28
   <212> DNA
   <213> Artificial Sequence
<220>
   <223> Description of Artificial Sequence: Synthetic primer
<400> 23
   atgtgaagcg tggaccaaaa tgctgaga 28
<210> 24
   <211> 28
   <212> DNA
   <213> Artificial Sequence
<220>
   <223> Description of Artificial Sequence: Synthetic primer
<400> 24
   ttctctagct ccggacatct caagcaca 28
<210> 25
   <211> 26
   <212> DNA
   <213> Artificial Sequence
<220>
   <223> Description of Artificial Sequence: Synthetic primer
<400> 25
   gtcgacatgt gaagcgtgga ccaaaa 26
<210> 26
   <211> 28
   <212> DNA
   <213> Artificial Sequence
<220>
   <223> Description of Artificial Sequence: Synthetic primer
<400> 26
   gcggccgcgc acaggtgccc cgcccacc 28
<210> 27
   <211> 26
   <212> DNA
   <213> Artificial Sequence
<220>
   <223> Description of Artificial Sequence: Synthetic primer
<400> 27
   gtcgaccgcg atgtttcttt agcata 26
<210> 28
   <211> 28
   <212> DNA
   <213> Artificial Sequence
<220>
   <223> Description of Artificial Sequence: Synthetic primer
<400> 28
   gcggccgcct tttcttacta ttaaaaat 28
<210> 29
   <211> 35
   <212> DNA
   <213> Artificial Sequence
<220>
   <223> Description of Artificial Sequence: Synthetic primer
<400> 29
   cttagctgtc cctgtggctg tcccctaaag ttggc 35
<210> 30
   <211> 89
   <212> DNA
   <213> Artificial Sequence
<220>
   <223> Description of Artificial Sequence: Synthetic primer
<400> 30
   ggatccacct aataacttcg tatagcatac attatacgaa gttatattaa gggttattga 60
   atatgatcgg aattgggctg caggaattc 89
<210> 31
   <211> 89
   <212> DNA
   <213> Artificial Sequence
<220>
   <223> Description of Artificial Sequence: Synthetic primer
<400> 31
   gaattcctgc agcccaattc cgatcatatt caataaccct taatataact tcgtataatg 60
   tatgctatac gaagttatta ggtggatcc 89
<210> 32
   <211> 50
   <212> DNA
   <213> Artificial Sequence
<220>
   <223> Description of Artificial Sequence: Synthetic primer
<400> 32
   accaacatcc ctgtgatgca cctgtagcct gctggactgg ctgaacagaa 50
<210> 33
   <211> 35
   <212> DNA
   <213> Artificial Sequence
<220>
   <223> Description of Artificial Sequence: Synthetic primer
<400> 33
   cttagctgtc cctgtggctg tcccctaaag ttggc 35
<210> 34
   <211> 97
   <212> DNA
   <213> Artificial Sequence
<220>
   <223> Description of Artificial Sequence: Synthetic primer
<400> 34
   ggatcccctc gagggaccta ataacttcgt atagcataca ttatacgaag ttatattaag 60
   ggttattgaa tatatcggaa ttgggctgca ggaattc 97
<210> 35
   <211> 103
   <212> DNA
   <213> Artificial Sequence
<220>
   <223> Description of Artificial Sequence: Synthetic primer
<400> 35
   gaattccgaa gttcctattc tctagaaagt ataggaactt catcagtcag gtacataata 60
   taacttcgta taagtatgct atacgaagtt attaggtgga tcc 103
<210> 36
   <211> 50
   <212> DNA
   <213> Artificial Sequence
<220>
   <223> Description of Artificial Sequence: Synthetic primer
<400> 36
   accaacatcc ctgtgatgca cctgtagcct gctggactgg ctgaacagaa 50
<210> 37
   <211> 22
   <212> DNA
   <213> Artificial Sequence
<220>
   <223> Description of Artificial Sequence: Synthetic primer
<400> 37
   tcagcctccg tgtcagagct gg 22
<210> 38
   <211> 27
   <212> DNA
   <213> Artificial Sequence
<220>
   <223> Description of Artificial Sequence: Synthetic primer
<400> 38
   ggcatggatt gctacccaga tgcaggt 27
<210> 39
   <211> 27
   <212> DNA
   <213> Artificial Sequence
<220>
   <223> Description of Artificial Sequence: Synthetic primer
<400> 39
   ggctcaactc tgcaaacacg tcagcgt 27
<210> 40
   <211> 27
   <212> DNA
   <213> Artificial Sequence
<220>
   <223> Description of Artificial Sequence: Synthetic primer
<400> 40
   tgctgacttt gctgacgaag acaggtg 27
<210> 41
   <211> 44
   <212> DNA
   <213> Artificial Sequence
<220>
   <223> Description of Artificial Sequence: Synthetic primer
<400> 41
   tggactcctc gttgccctga cgtaggccat gaggcccatc cagt 44
<210> 42
   <211> 28
   <212> DNA
   <213> Artificial Sequence
<220>
   <223> Description of Artificial Sequence: Synthetic primer
<400> 42
   aggcgggtcg ggatcatcat aagttgta 28
<210> 43
   <211> 29
   <212> DNA
   <213> Artificial Sequence
<220>
   <223> Description of Artificial Sequence: Synthetic primer
<400> 43
   cctcgccatc aggaacatgt ggacataga 29
<210> 44
   <211> 21
   <212> DNA
   <213> Artificial Sequence
<220>
   <223> Description of Artificial Sequence: Synthetic primer
<400> 44
   agtccctgcc ctttgtacac a 21
<210> 45
   <211> 19
   <212> DNA
   <213> Artificial Sequence
<220>
   <223> Description of Artificial Sequence: Synthetic primer
<400> 45
   cgatccgagg gcctcacta 19
<210> 46
   <211> 20
   <212> DNA
   <213> Artificial Sequence
<220>
   <223> Description of Artificial Sequence: Synthetic primer
<400> 46
   gtctgcccag aggctggctt 20
<210> 47
   <211> 20
   <212> DNA
   <213> Artificial Sequence
<220>
   <223> Description of Artificial Sequence: Synthetic primer
<400> 47
   cctctctgag gactccgcgt 20
<210> 48
   <211> 20
   <212> DNA
   <213> Artificial Sequence
<220>
   <223> Description of Artificial Sequence: Synthetic primer
<400> 48
   agcaccggag gagctgaacc 20
<210> 49
   <211> 25
   <212> DNA
   <213> Artificial Sequence
<220>
   <223> Description of Artificial Sequence: Synthetic primer
<400> 49
   cagtccttca aaggaaactg gtccg 25
<210> 50
   <211> 20
   <212> DNA
   <213> Artificial Sequence
<220>
   <223> Description of Artificial Sequence: Synthetic primer
<400> 50
   ggattggcct ctacgactca 20
<210> 51
   <211> 20
   <212> DNA
   <213> Artificial Sequence
<220>
   <223> Description of Artificial Sequence: Synthetic primer
<400> 51
   taagccggct gagatcttgt 20
<210> 52
   <211> 20
   <212> DNA
   <213> Artificial Sequence
<220>
   <223> Description of Artificial Sequence: Synthetic primer
<400> 52
   tgtgtaaggc tgcacgagtc 20
<210> 53
   <211> 20
   <212> DNA
   <213> Artificial Sequence
<220>
   <223> Description of Artificial Sequence: Synthetic primer
<400> 53
   ggcagtagca aaaggcattg 20
<210> 54
   <211> 20
   <212> DNA
   <213> Artificial Sequence
<220>
   <223> Description of Artificial Sequence: Synthetic primer
<400> 54
   gagttcaaga gggagctgga 20
<210> 55
   <211> 20
   <212> DNA
   <213> Artificial Sequence
<220>
   <223> Description of Artificial Sequence: Synthetic primer
<400> 55
   ggtcatgaag ccaccgtaac 20
<210> 56
   <211> 20
   <212> DNA
   <213> Artificial Sequence
<220>
   <223> Description of Artificial Sequence: Synthetic probe
<400> 56
   tttgctgacg aagacaggtg 20
<210> 57
   <211> 20
   <212> DNA
   <213> Artificial Sequence
<220>
   <223> Description of Artificial Sequence: Synthetic probe
<400> 57
   cagtccaggt ctatgcgtga 20
<210> 58
   <211> 32
   <212> DNA
   <213> Artificial Sequence
<220>
   <223> Description of Artificial Sequence: Synthetic primer
<400> 58
   aagcttgcca ccatggagat gtctgcccag ag 32
<210> 59
   <211> 28
   <212> DNA
   <213> Artificial Sequence
<220>
   <223> Description of Artificial Sequence: Synthetic primer
<400> 59
   gcggccgctc agtccaggtc tatgcgtg 28
<210> 60
   <211> 49
   <212> DNA
   <213> Artificial Sequence
<220>
   <223> Description of Artificial Sequence: Synthetic primer
<400> 60
   tttcctttga aggactgaag gctcataaat caacgcggag tcctcagag 49
<210> 61
   <211> 49
   <212> DNA
   <213> Artificial Sequence
<220>
   <223> Description of Artificial Sequence: Synthetic primer
<400> 61
   ctctgaggac tccgcgttga tttatgagcc ttcagtcctt caaaggaaa 49
<210> 62
   <211> 32
   <212> DNA
   <213> Artificial Sequence
<220>
   <223> Description of Artificial Sequence: Synthetic primer
<400> 62
   aagcttgcca ccatgaactc cacccaccac ca 32
<210> 63
   <211> 28
   <212> DNA
   <213> Artificial Sequence
<220>
   <223> Description of Artificial Sequence: Synthetic primer
<400> 63
   gcggccgctt aatacctgct agacaact 28
<210> 64
   <211> 10
   <212> PRT
   <213> Mus musculus
<400> 64
   Met Ser Ala Gln Arg Leu Ala Ser Asn Arg
   1 5 10
<210> 65
   <211> 16
   <212> PRT
   <213> Rattus sp.
<400> 65
   Leu Leu Glu Asn Lys Pro Val Ser Gln Thr Ala Arg Thr Asp Leu Asp
   1 5 10 15
<210> 66
   <211> 21
   <212> DNA
   <213> Artificial Sequence
<220>
   <223> Description of Artificial Sequence: Synthetic oligonucleotide
<220>
   <221> CDS
   <222> (1)..(21)
<400> 66
   gct cat aaa tat tcc att gtg 21
   Ala His Lys Tyr Ser Ile Val
   1 5
<210> 67
   <211> 7
   <212> PRT
   <213> Artificial Sequence
<220>
   <223> Description of Artificial Sequence: Synthetic peptide
<400> 67
   Ala His Lys Tyr Ser Ile Val
   1               5
<210> 68
   <211> 21
   <212> DNA
   <213> Artificial Sequence
<220>
   <223> Description of Artificial Sequence: Synthetic oligonucleotide
<220>
   <221> CDS
   <222> (1)..(21)
<400> 68
   gct cat aaa tca acg cgg agt                                   21
   Ala His Lys Ser Thr Arg Ser
   1               5
<210> 69
   <211> 7
   <212> PRT
   <213> Artificial Sequence
<220>
   <223> Description of Artificial Sequence: Synthetic peptide
<400> 69
   Ala His Lys Ser Thr Arg Ser
   1               5

## Description

### CROSS-REFERENCE TO RELATED APPLICATIONS

This application claims benefit under 35 U.S.C. § 119(e) of U.S. Provisional Application No. 61/844,666 filed July 10, 2013.

### GOVERNMENT SUPPORT

This invention was made with federal funding under Grant Nos. T32DK07699 and NIHP30-HD18655 awarded by the National Institutes of Health. The U.S. government has certain rights in the invention.

### SEQUENCE LISTING

The instant application contains a Sequence Listing which has been submitted electronically in ASCII format and is hereby incorporated by reference in its entirety. Said ASCII copy, created on June 27, 2014, is named 068570-078611-PCT_SL.txt and is 25,182 bytes in size.

### TECHNICAL FIELD

The technology described herein relates to knock-out and transgenic animals.

### BACKGROUND

When raising animals for purposes such as food or breeding, a significant contributor to the costs associated is the time it takes the animal to mature to the necessary size and/or weight. The faster the animal reaches the necessary size and/or weight, the lower the costs associated with raising the animal and the fewer resources are consumed in producing a functional animal. Animal husbandry often seeks to minimize this time by controlling feed, environment, and verterinary care in such a way as to encourage rapid growth. Additionally, animal feed is the major cost of bringing animals to market, and therefore increased 'feed efficiency (weight of animal growth/weight of food consumed)' is sought to decrease the cost of livestock.

### SUMMARY

The invention is defined in the claims. As described herein, the inventor has discovered that by inhibiting the level and/or activity of Mrap2 in an animal, the animal gains weight at a very rapid rate as compared to a wildtype animal. In fact, the animal can reach mature adult size as much as 50% faster than a wildtype animal. Further, the animal lacking Mrap2 activity can grow just as quickly as a wildtype animal when restricted to a smaller diet. Accordingly, described herein are cells with modifications of Mrap2, animals comprising those cells, and methods of using such animals in order to provide more efficient production of animal products.

In one aspect, described herein is a non-human mammalian cell comprising a modification of the Mrap2 gene, wherein the cell does not express a detectable level of functional Mrap2. In some embodiments described herein, the modification comprises a deletion of the Mrap2 coding sequence. In some embodiments described herein, the modification comprises a deletion of exon 3 of the Mrap2 gene. In some embodiments described herein, the modification comprises a deletion of the transmembrane domain of Mrap2. In some embodiments described herein, the modification comprises a deletion of the intracellular domain of Mrap2. In some embodiments described herein, the modification comprises a mutation of at least one amino acid (or at least one nucleotide encoding such an amino acid, *e.g.,* a point mutation, a SNP, etc.) of SEQ ID NO. 1.

In some embodiments described herein, the cell is heterozygous for the modification. In some embodiments described herein, the cell is homozygous for the modification. In some embodiments described herein, the non-human mammalian cell is of a species selected from the group consisting of pig; cow; sheep; rabbit; and goat.

In some embodiments, the modification is produced by the action of a CRISPR, TALENs, or ZFN nuclease. In some embodiments the modification is produced by the action of a recombinase. In some embodiments, the recombinase is cre recombinase. In some embodiments, the recombinase is under the control of a tissue-specific promoter. In some embodiments, the promoter is specific for neurons or brain tissue. In some embodiments, the promoter is a Sim1 promoter.

In some embodiments, the modification is an engineered modification, e.g.. a modification that is not naturally-ocurring. As used herein, "engineered" refers to the aspect of having been manipulated by the hand of man. For example, a polynucleotide can be considered to be "engineered" when two or more sequences, that are not linked together in that order in nature, are manipulated by the hand of man to be directly linked to one another in the engineered polynucleotide. By way of further example, a polynucleotide can be considered to be "engineered" when the sequence of the polynucleotide is altered (e.g. by deletion) such that the sequence varies from that found in nature. As is common practice and is understood by those in the art, progeny and copies of an engineered polynucleotide are typically still referred to as "engineered" even though the actual manipulation was performed on a prior entity.

In one aspect, described herein is a non-human mammal comprising a cell as described herein. In one aspect, described herein is a non-human mammal consisting of cells as described herein.

In one aspect, described herein is a knockout non-human mammal, comprising a modification of the Mrap2 gene, wherein the mammal does not express a detectable level of functional Mrap2.

In some embodiments, the modification comprises a deletion of the Mrap2 coding sequence. In some embodiments, the modification comprises a deletion of exon 3 of the Mrap2 gene. In some embodiments, the modification comprises a deletion of the transmembrane domain of Mrap2. In some embodiments, the modification comprises a deletion of the intracellular domain of Mrap2. In some embodiments, the modification comprises a mutation of at least one amino acid (or at least one nucleotide encoding such an amino acid, *e.g.,* a point mutation, a SNP, etc.) of SEQ ID NO. 1. In some embodiments described herein, the mammal is heterozygous for the modification. In some embodiments described herein, the mammal is homozygous for the modification. In some embodiments, the non-human mammal is of a species selected from the group consisting of pig; cow; bison; horse; sheep; rabbit; and goat.

In some embodiments, the modification is produced by the action of a CRISPR, TALENs, or ZFN nuclease. In some embodiments the modification is produced by the action of a recombinase. In some embodiments, the recombinase is cre recombinase. In some embodiments, the recombinase is under the control of a tissue-specific promoter. In some embodiments, the promoter is specific for neurons or brain tissue. In some embodiments, the promoter is a Sim1 promoter.

In one aspect, described herein is a method of producing a mammal for use of the carcass, the method comprising: providing species-appropriate food to a mammal described herein and butchering the mammal when it reaches the desired size for use. In some embodiments described herein, the mammal is butchered as much as 80% earlier than a wild-type mammal. In some embodiments described herein, the mammal is butchered as much as 50% earlier than a wild-type mammal. In some embodiments described herein, the mammal is provided feed substantially *ad libitum.* The mammal is a pig. Other non-limiting examples described herein of a mammal for use with the methods described herein include a cow, a horse, a sheep, a goat, a rabbit, a bison, etc.

In some embodiments described herein, the mammal is butchered at from about 75 to about 125 days of age. In other embodiments described herein, the mammal is butchered between 4-8 months of age (*e.g.,* at 4 months, 5 months, 6 months, 7 months, 8 months or any time period between). In some embodiments described herein, the mammal is provided a restricted level of food. In some embodiments described herein, the restricted level of food is from about 60% to about 95% of the level provided to a wild-type mammal. In some embodiments described herein, the restricted level of food is from about 80% to about 95% of the level provided to a wild-type mammal. The mammal is a pig. In alternative embodiments described herein, the mammal is a cow, a horse, a sheep, a rabbit, a goat, a bison etc.

### BRIEF DESCRIPTION OF THE DRAWINGS

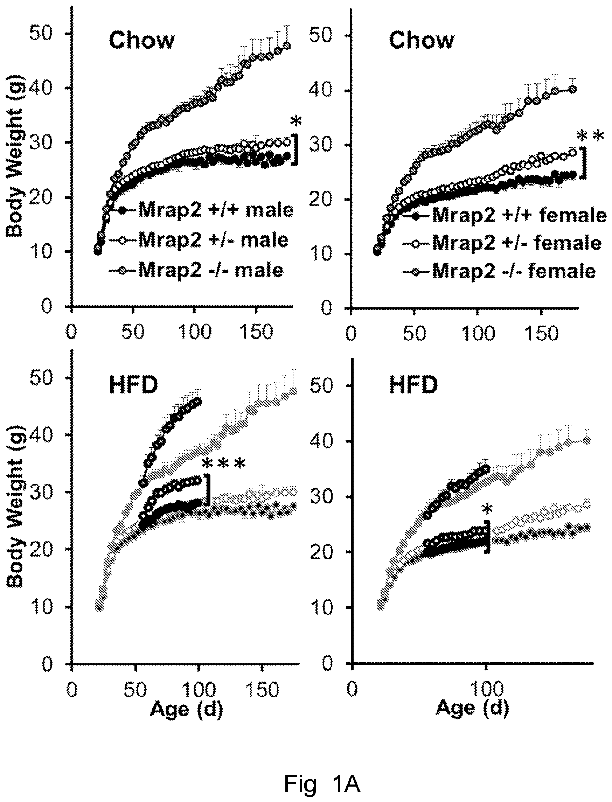

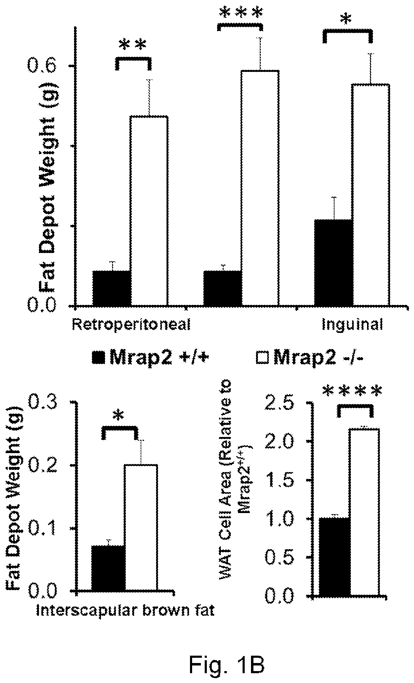

Figs. 1A-1B depict the phenotype of *Mrap2*^{*-*/*-*} mice. Fig. 1A depicts weight curves for *Mrap*^{*+*/}*⁺ vs. Mrap*^{*+*/-} *vs. Mrap2*^{-/-} mice on standard chow (Chow, upper panels: male n= 9 vs. 28 vs. 15, female n=12 vs. 18 vs. 10) or high fat diets (HFD, ages 56-95 days, lower panels, superimposed on standard chow curves: male n= 10 vs. 8 vs. 10, female n=7 vs. 12 vs. 7). For both genders the weight curves of *Mrap*^{*+*/*+*} and *Mrap*^{*+*/-} mice on standard chow differ significantly at older (161-175 days) ages, and at younger ages (56-95 days) on a high fat diet. ). *p=.02, **p=.001, ***p=.0003. Fig. 1B depicts graphs of fat depots on standard chow diet. Upper panel: White adipose tissue (WAT) weights in *Mrap*^{*+*/}*⁺ vs. Mrap2*^{-/-} (males and females, ages 117-122 days, n=5 vs. 4, respectively). Lower left panel: BAT weight in *Mrap*^{*+*/}*⁺ vs. Mrap2*^{-/-} mice (males and females, ages 117-122 days, n=5 vs. 4). Lower right panel: WAT cell size in in *Mrap*^{*+*/}*⁺ vs. Mrap2*^{-/-} mice (females, 50 cells counted from each mouse). *p=.009, **p=.003, ***p=.0003, ****p<.00001.

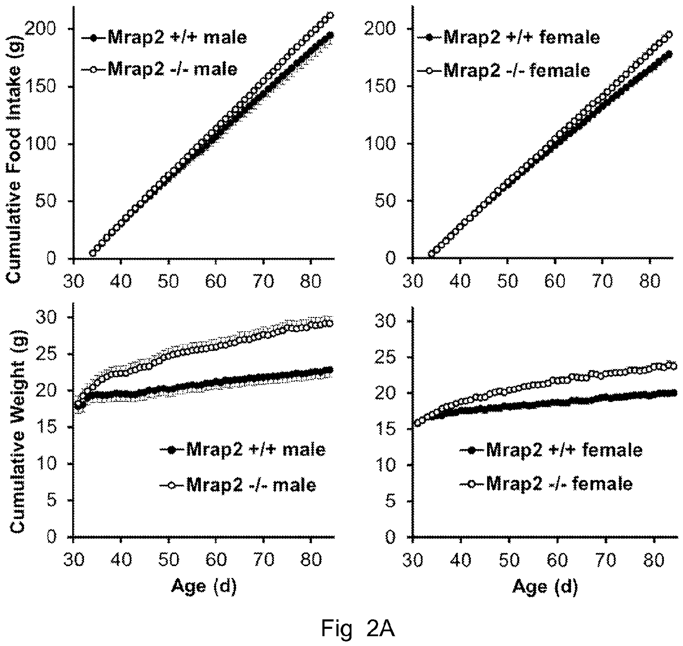

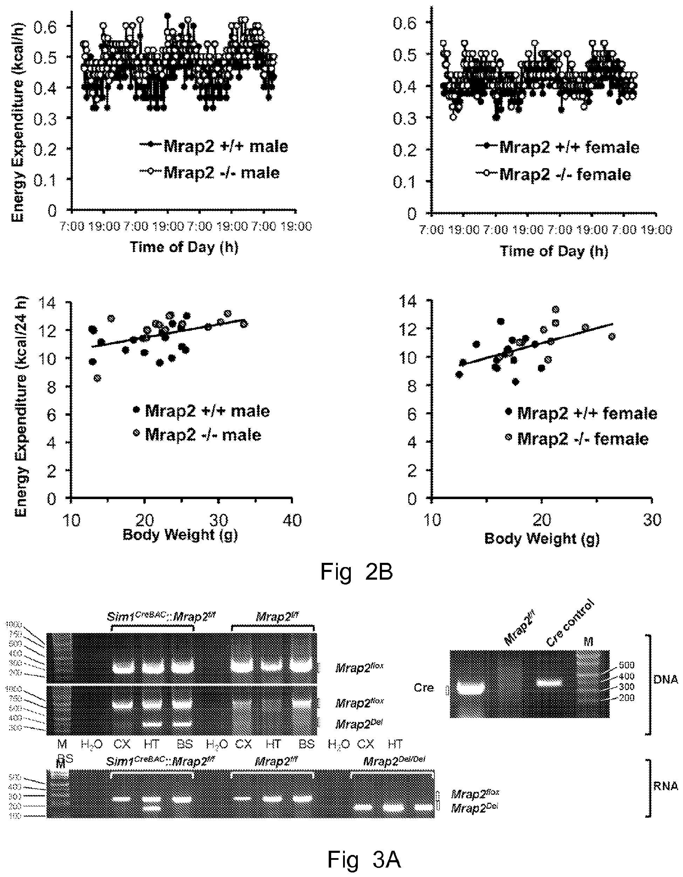

Figs. 2A-2B depict the energy balance in *Mrap2*^{-/-} mice. Fig. 2A depicts graphs of cumulative food intake (upper panels) and weight (lower panels) in *ad libitum* fed *Mrap*^{*+*/}*⁺ vs. Mrap2*^{-/-} males (n=10 *vs*. 11) and females (n=11 vs. 8). Fig. 2B depicts graphs of energy expenditure in *ad libitum-fed Mrap*^{+/+} *vs*. *Mrap2*^{-/-} mice. Upper panels, continuous measurement over 3 days, males (n=3 *vs*. 4), females (n=4 vs. 3), ages 30-34 days. Lower panels: body weight vs. energy expenditure, integrated over 24 h, males (n=18 *vs*. 14, ages 30-45 days), females (n=16 *vs*. 11, ages 30-42 days). Analysis by ANCOVA showed no differences between genotypes (males, p=.38, females, p=.67).

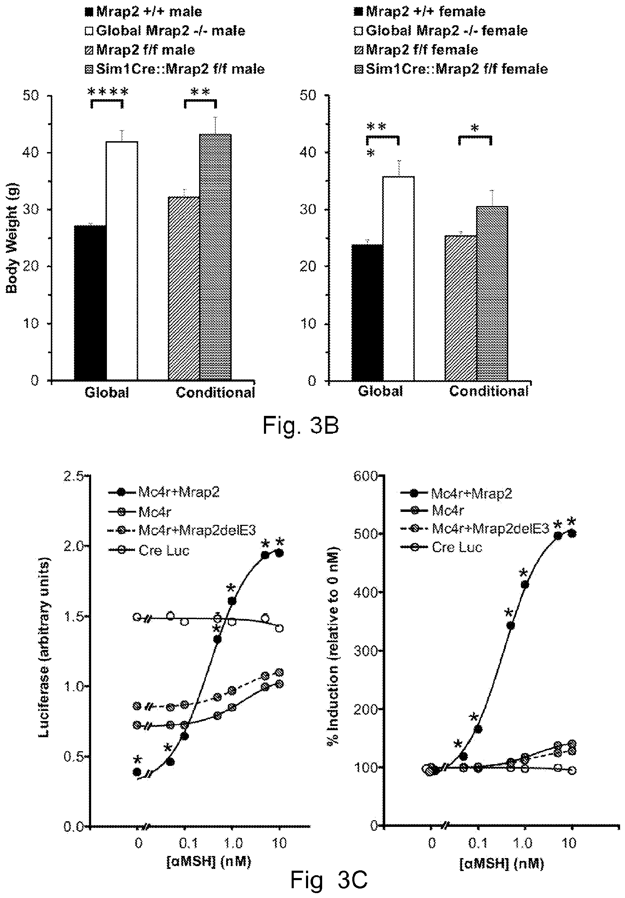

Figs. 3A-3C depict the interaction between Mrap2 and Mc4r. Fig. 3A depicts electrophoresis gels demonstrating the conditional deletion of *Mrap2* in Simlneurons. Top right, Cre DNA analysis by PCR. HT DNA from *Sim1^{CreBAC}*::*Mrap2*^{*f*/*f*} mice contains *Cre* (374 bp), but from *Mrap2*^{*f*/*f*} mice does not. Molecular weight marker (M) on right (bp). Top left, *Mrap2* DNA analysis in *Sim1^{CreBAC}*::*Mrap2*^{*f*/*f*} and *Mrap2*^{*f*/*f*} mice by PCR. Both genotypes contain floxed, intact *Mrap2* DNA in CX, HT, and BS (314 bp in upper electropherogram, and 1013 bp in lower electropherogram, molecular weight markers on left). Only *Sim1^{CreBAC}*::*Mrap2*^{*f*/*f*} mice contain *Mrap2^{Del}* (400 bp, lower electropherogram), and only in HT and BS, but not in CX, consistent with fluorescent reporter data (Fig. S3A). No PCR products are present without added DNA (H₂O). Bottom, Mrap2 mRNA expression in *Sim1^{CreBAC}*::*Mrap2*^{*f*/*f*} and *Mrap2*^{*f*/*f*} mice by RT-PCR. Both genotypes express floxed, intact Mrap2 mRNA in CX, HT, and BS (247 bp). Only *Sim1^{CreBAC}*::*Mrap2*^{*f*/*f*} mice express Mrap*2^{Del}* mRNA (147 bp), and only in HT. Global *Mrap2*^{*Del*/Del} mice express Mrap*2^{Del}* mRNA in all 3 sites. Fig. 3B depicts graphs of body weights of *Mrap2*^{+/+} (male n=6, female n=11), *Mrap2*^{-/-} (male n=11, female n=7), *Mrap2*^{*f*/*f*} (male n=8, female n=12) and conditional *Sim1^{CreBAC}*::*Mrap2*^{*f*/*f*} (male n=8, female n=7) mice, all age 133 days. *p=.04, **p=.007, ***p=.0002, ****p<.0001. Fig. 3C depicts graphs of the effect of Mrap2 on Mc4r signaling. Left panel: Level of cAMP reporter activity (CRELuc) in CHO cells alone, or co-transfected with *Mc4r,* with or without *Mrap2* or the *Mrap2* knockout construct, *Mrap2delE3,* 5 h following exposure to 0-10 nM αMSH (n=3/group). Right panel: cAMP activity of these same constructs, expressed as percent induction following 0-10 nM αMSH, relative to 0 nM αMSH. *p<.0001, Mc4r+Mrap2 *vs*. Mc4r at same [αMSH], by ANOVA. For most data points, error bars are obscured by symbols.

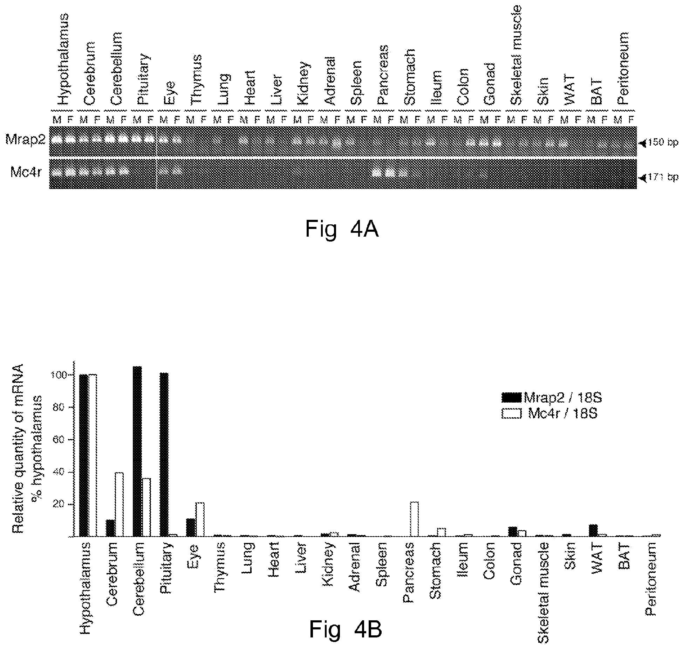

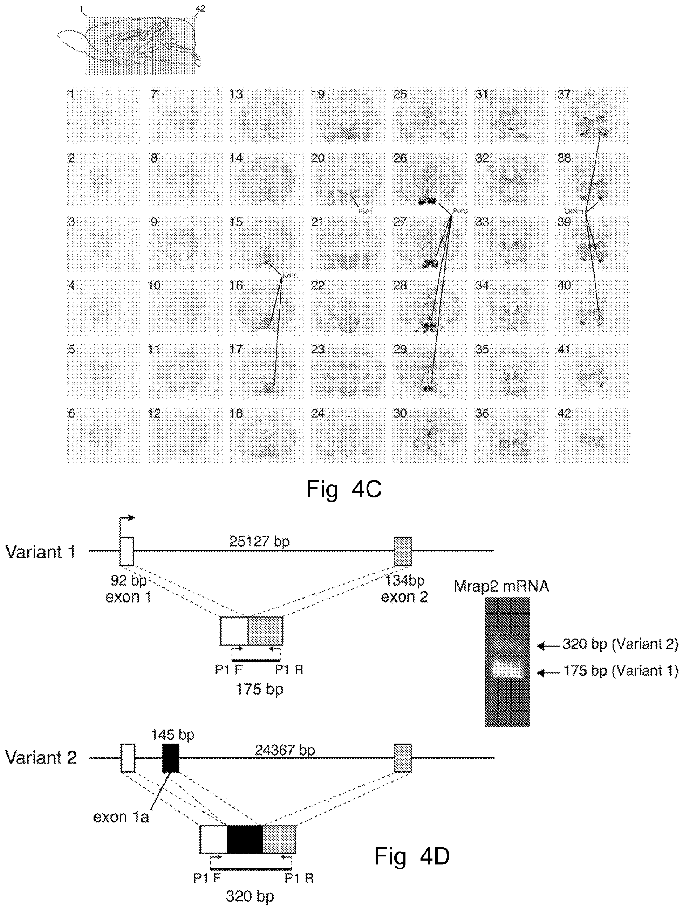

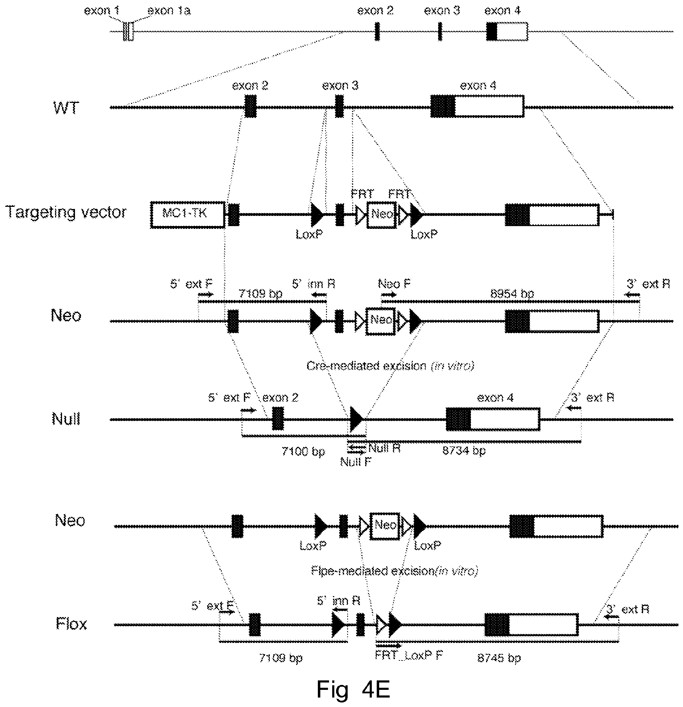

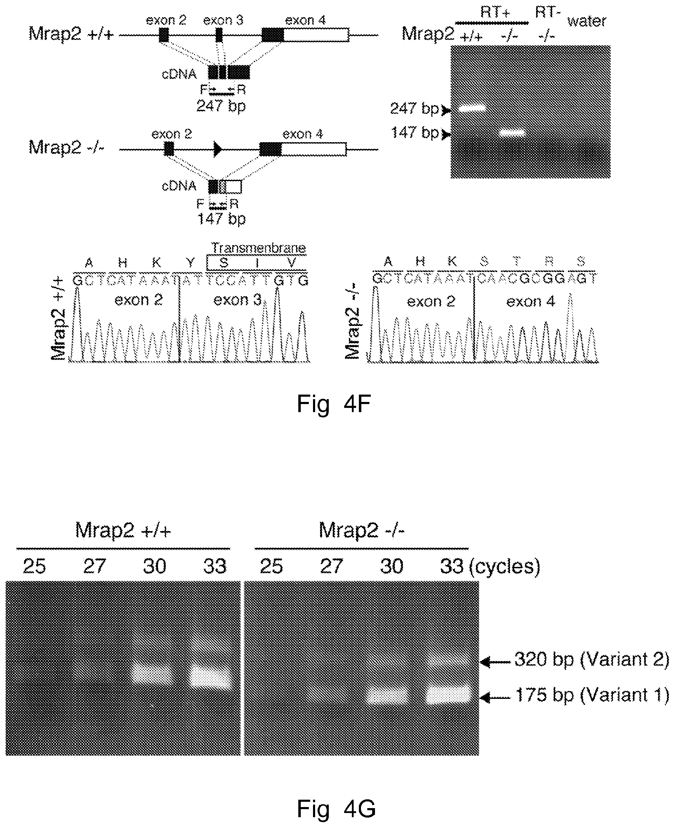

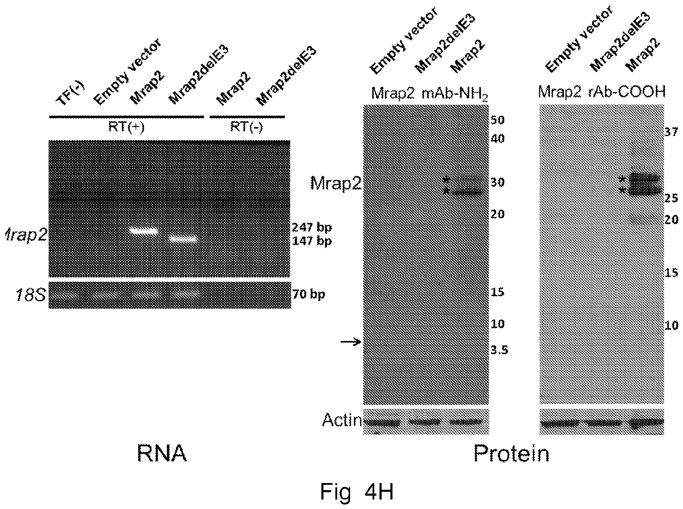

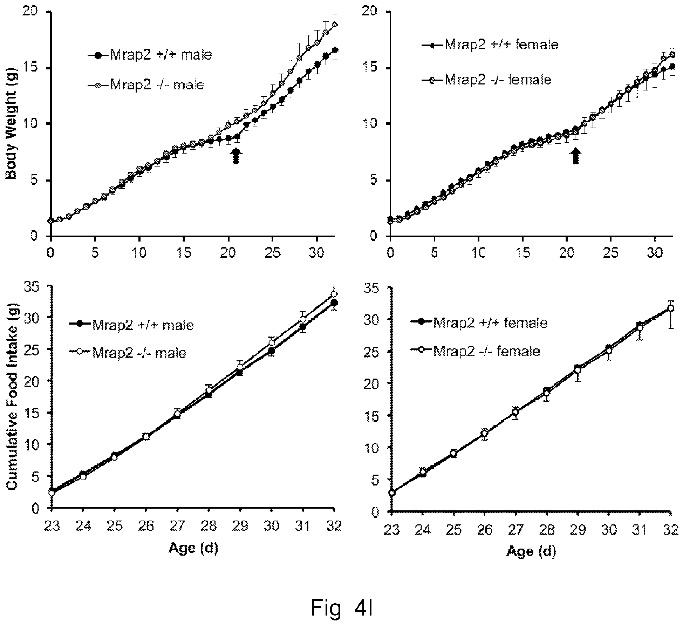

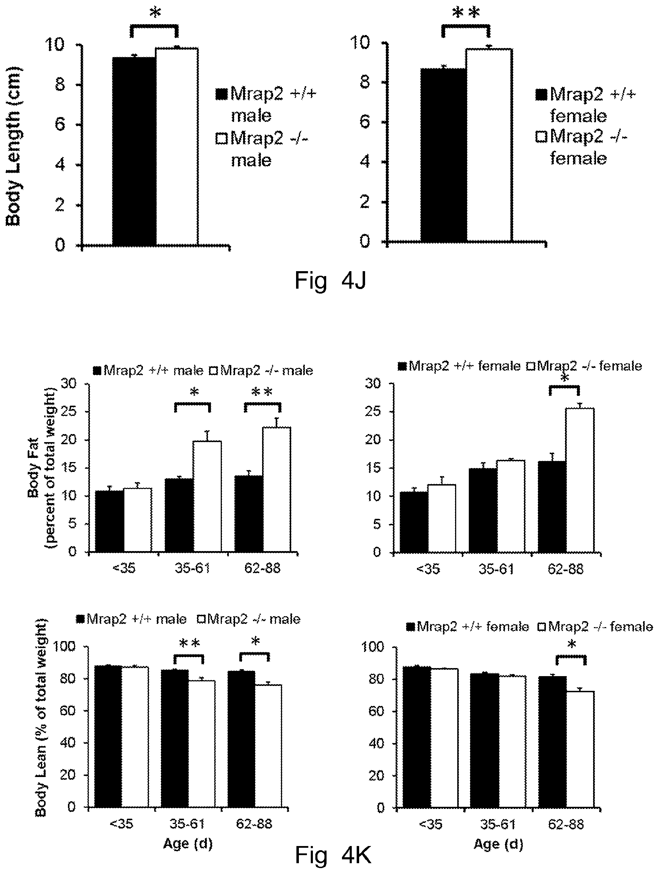

Figs. 4A-4K demonstrate Mrap2 mRNA expression and creation of *Mrap2*^{-/-} mice. Fig. 4A depicts images of gels of Mrap2 (150 base pairs (bp), upper panel) and Mc4r (171 bp, lower panel) mRNA in brain and peripheral tissues of male (M) and female (F) mice, by RT-PCR. Fig. 4B depicts a graphical comparison of Mrap2 mRNA and Mc4r mRNA in male mouse tissues, relative to their amounts in hypothalamus, by qRT-PCR corrected for 18s RNA. Both mRNAs are expressed principally in the brain. Fig. 4C depicts images of expression of Mrap2 mRNA in coronal sections of mouse brain, by in situ hybridization, with major sites of expression in medial preoptic nucleus (MPN), paraventricular nucleus (PVN) of the hypothalamus, pons, laternal reticular nucleus, magnocellular region (LRNm), and diffusely throughout the cerebellum. Fig. 4D depicts the detection of minor Mrap2 mRNA variant containing 145 bp exon 1a in hypothalamus in wild-type mice. Fig. 4E depicts a schematic of a strategy for creating *Mrap2*^{-/-} (null) mice. A targeting vector containing a neomycin resistance (neo) gene cassette flanked by FRT sites (open triangles) and a thymidine kinase (TK) gene, along with *Mrap2* exon 3 flanked with loxP sites (solid triangles), was incorporated by homologous recombination into the genomic *Mrap2* locus of 129Sv embryonic stem cells. Exon 3 and the neo cassette were removed using Cre-mediated excision *in vitro* to create *Mrap2* null ES cells, and the neo cassette alone was removed using Flpe-mediated excision *in vitro* to create *Mrap2* floxed ES cells. ES cells were implanted into C56BL/6J pseudopregnant mice and male offspring were bred to 129S6/SvEvTac females to create *Mrap2* null or *Mrap2* floxed mice on a pure 129 background. Fig. 4F depicts hypothalamic Mrap2 mRNA in *Mrap2*^{-/-} mice. Upper left panel, diagram of *Mrap2* gene and mRNA in *Mrap*^{+/+} and *Mrap2*^{-/-} mice. In *Mrap2*^{-/-} mice, the 100-base pair (bp) exon 3 (encoding the transmembrane domain) is replaced with a LoxP site (triangle), such that *Mrap2*^{-/-} mRNA (Mrap2*^{Del}* mRNA) is 100 nucleotides shorter than *Mrap*^{*+*/*+*} mRNA. Upper right panel, RT-PCR of hypothalamic Mrap2 mRNA in *Mrap2*^{+/+} or *Mrap2*^{-/-} mice or no RNA control (water), in presence (RT+) or absence (RT-) of reverse transcriptase. RT-PCR of cDNA reverse-transcribed from *Mrap2*^{-/-} mouse hypothalamic RNA is 147 bp *vs.* 247 bp long. Lower panel, hypothalamic Mrap2 mRNA sequence across either the exon 2-exon 3 junction of *Mrap2*^{+/+} mice (left) or across the exon 2-exon 4 junction of *Mrap2*^{-/-} mice (right). In the latter, the transmembrane domain of exon 3 is replaced by the out-of-frame exon 4 encoding 11 aberrant amino acids followed by a stop codon. Sequences disclosed as SEQ ID NOS 67, 69, 66 and 68, respectively, in order of appearance. Fig. 4G demonstrates that *Mrap2*^{-/-} mice express a normal amount of mutant hypothalamic Mrap2 mRNA. Using primers (P1F, P1R) within exons 1 and 2 (and which do not detect the exon 3 deletion in *Mrap2*^{-/-} mRNA), semiquantiative RT-PCR reveals similar amounts of Mrap2 mRNA (including major variant 1 and minor variant 2) in *Mrap2*^{+/+} and *Mrap2*^{-/-} mouse hypothalami. Fig. 4H depicts the levels of Mrap2 RNA and protein in HEK293 cells. Cells were transfected with nothing [TF(-)], empty vector, *Mrap2,* or *Mrap2delE3* expression constructs, followed by RTPCR of Mrap2 mRNA and 18S rRNA (left panel), or western blot of Mrap2 protein (middle and right panels), respectively. WT Mrap2 cDNA, 247 bp, mutant Mrap2delE3 cDNA, 147 bp (as in Fig. 1A). Middle panel western blot probed with Mrap2 mAb-NH₂, directed against the amino-terminus, right panel western blot probed with Mrap2 rAb-COOH, directed against the carboxyl-terminus. Mrap2 protein predicted size, 23.7 kD, mutant Mrap2delE3 protein predicted size, 5.9 kD. Asterisks denote positions of migration of Mrap2, and horizontal arrow denotes predicted position of migration of mutant Mrap2delE3. Molecular weight markers (kD) to right of each blot. cDNAs transcribed from *Mrap2* and *Mrap2delE3* mRNAs are detected only with transfection of their respective constructs and in the presence of reverse transcriptase [RT(+)]. Mrap2, but not Mrap2delE3 protein, is detected by both antibodies. Fig. 4I depicts graphs of body weight (upper panels, ages 0-32 days) and cumulative food intake (lower panels, ages 23-32 days) in young *Mrap2*^{+/+} *vs. Mrap2*^{-/-} male (n=6 *vs*. 3) and female (n=3 *vs*. 6) mice, weaned at 21 days (upward arrow). There is no significant difference between genotypes in body weight or food intake in either gender, although *Mrap2*^{-/-} male mice have a trend towards greater weight and food intake with advancing age. Fig. 4J depicts graphs of body length of *Mrap*^{+*l*+} *vs*. *Mrap2*^{-/-} (age 84 days, male n=9 *vs*. 15, female n=12 *vs*. 10). *p=.015, **p=.01 Fig. 4K depicts fat mass percentage (upper panels) and lean mass percentage (lower panels) of *Mrap*^{+/+} *vs. Mrap2*^{-/-} (male n=35 *vs*. 33, female n=34 *vs.* 40) mice. *p=.001, **p=.0001.

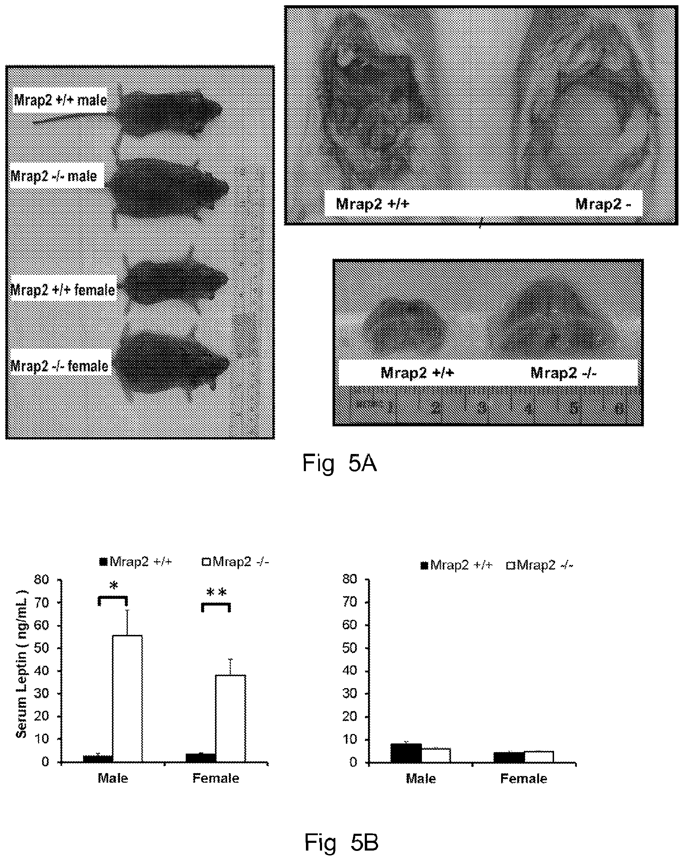

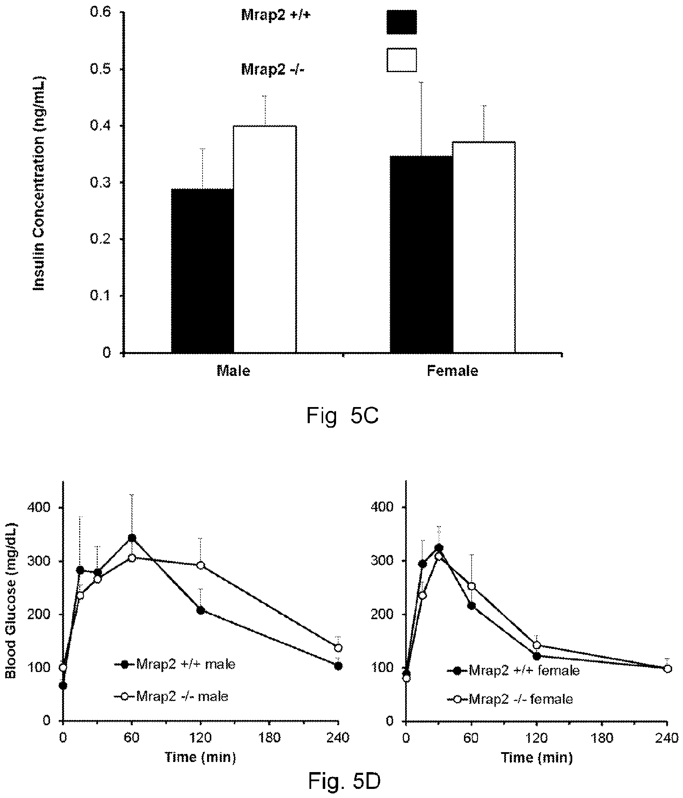

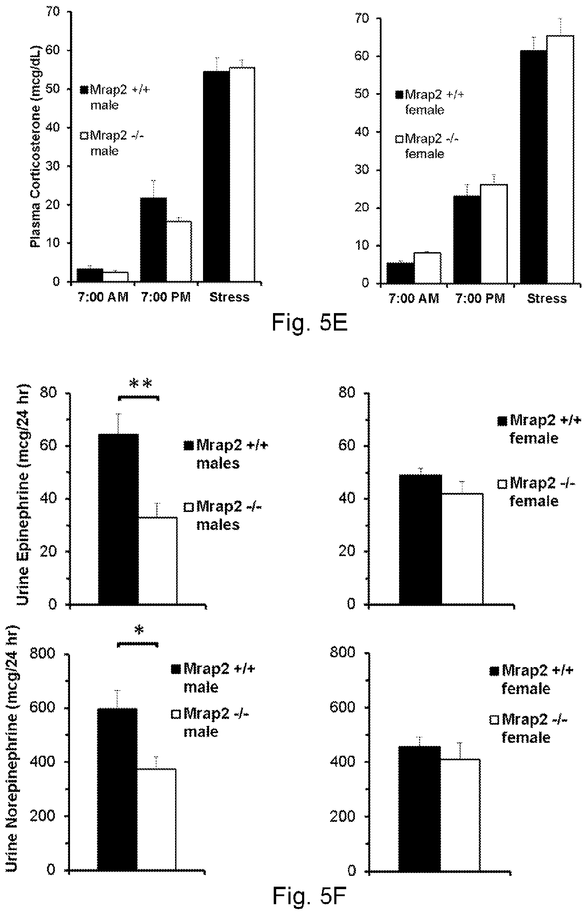

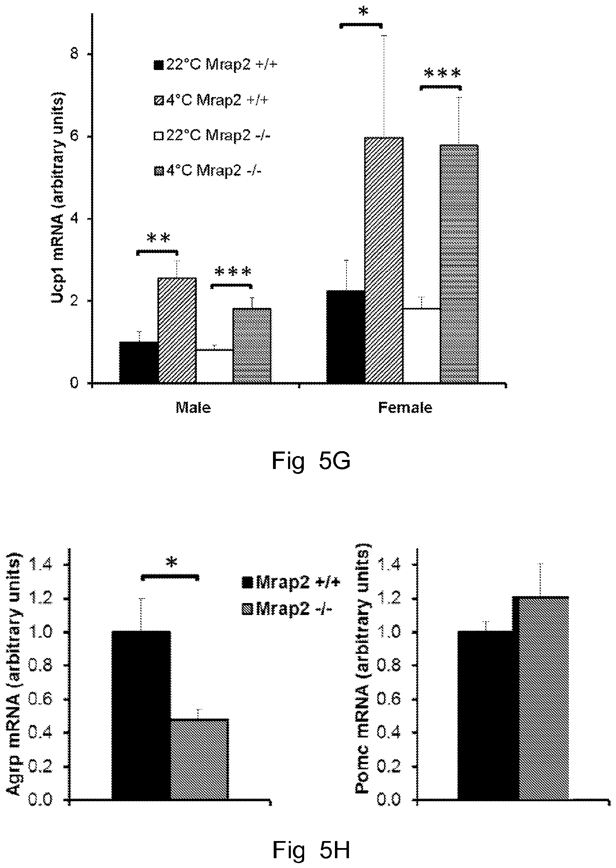

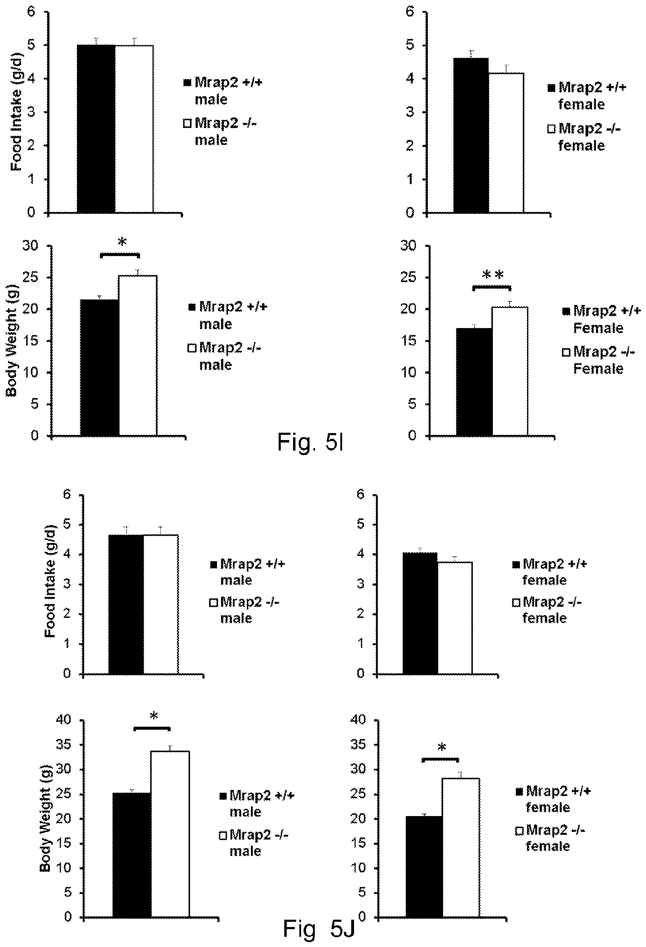

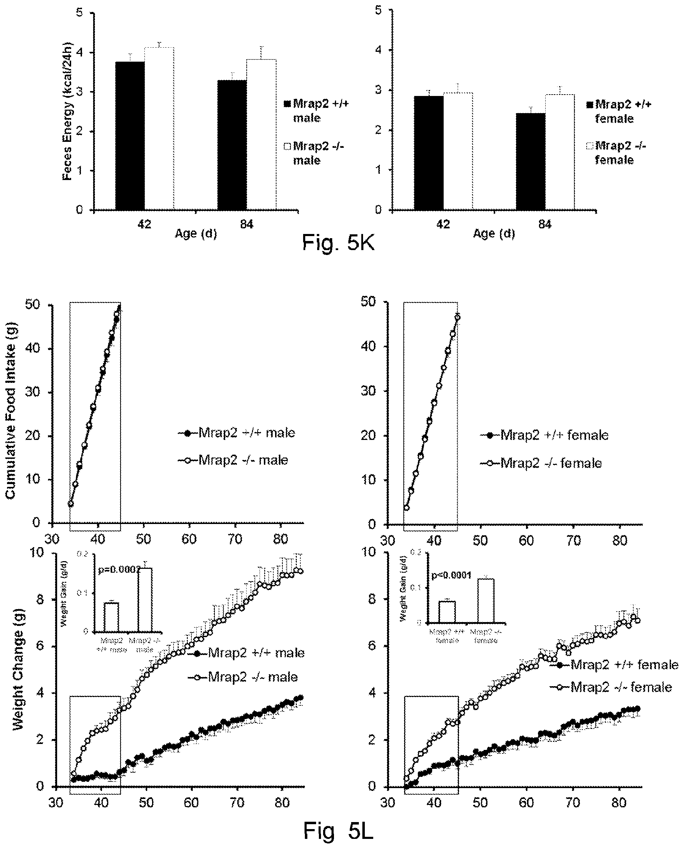

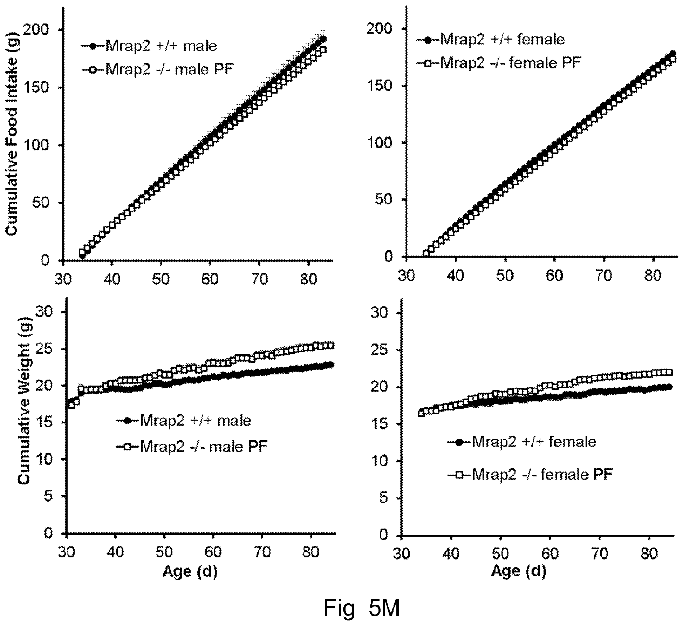

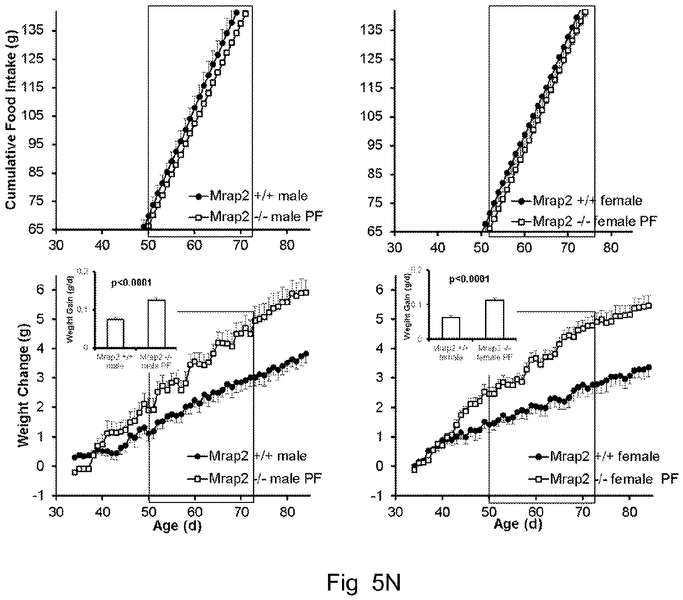

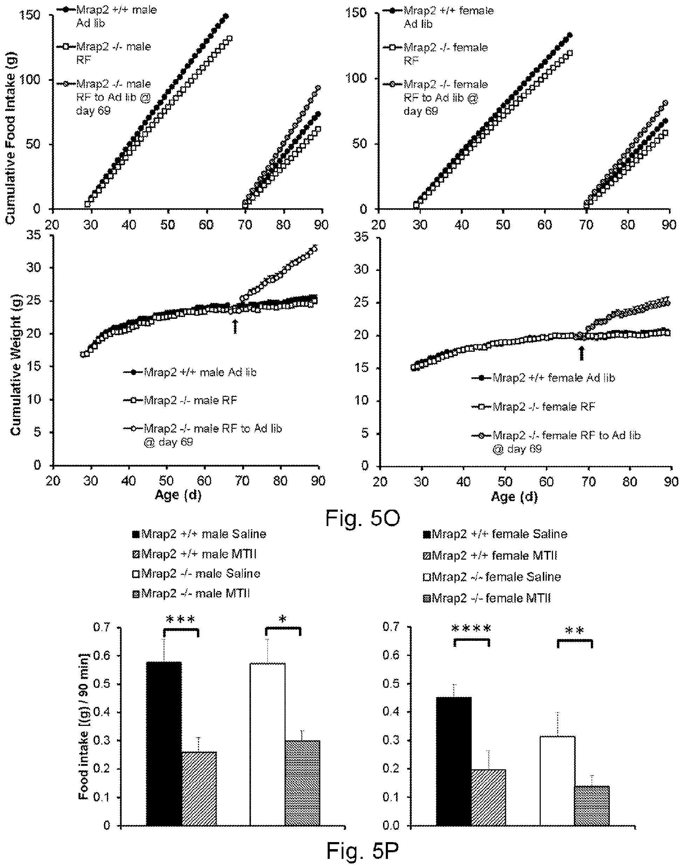

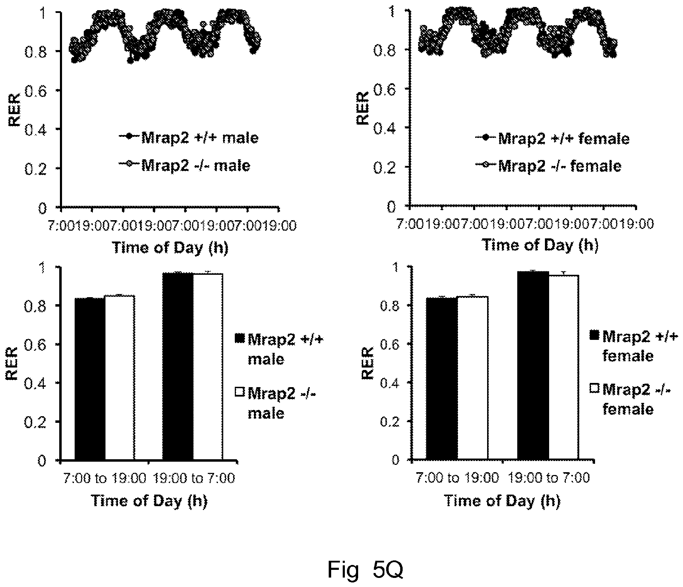

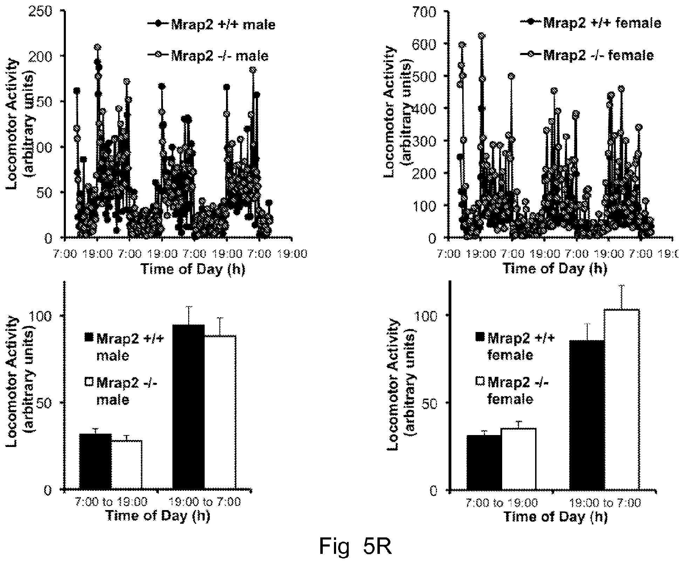

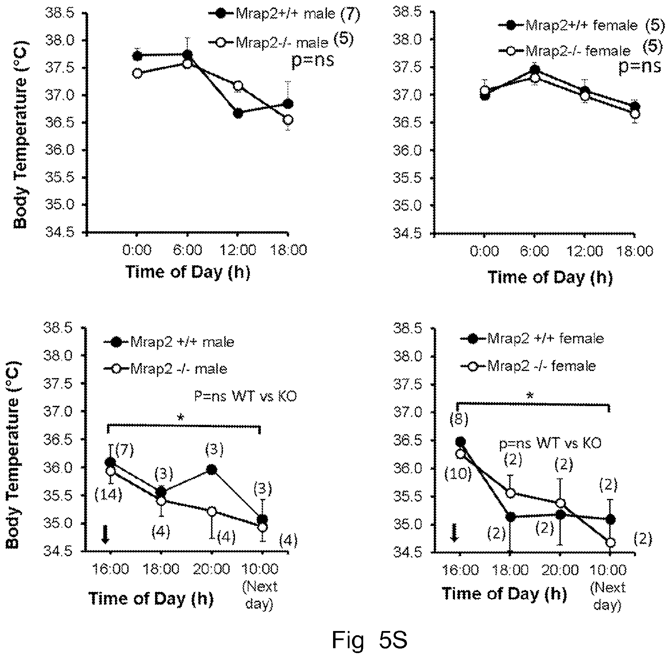

Figs. 5A-5S demonstrate the phenotype of *Mrap2*^{-/-} mice. Fig. 5A depicts images of the appearance of male and female *Mrap*^{*+*/*+*} and *Mrap2*^{-/-} mice. Left panel: External appearance on standard chow (age 175 days). Upper right: mesenteric fat in female *Mrap*^{*+*/*+*} and *Mrap2*^{-/-} mice (age 175 days). Lower right: Brown adipose tissue (BAT) in female *Mrap*^{*+*/*+*} and *Mrap2*^{-/-} mice (age 175 days). Fig. 5B depicts the level of serum leptin in *Mrap2*^{+/+} and *Mrap2*^{-/-} mice. Left panel: *Mrap2*^{+/+} *vs. Mrap2*^{-/-} *ad libitum* fed male (n=4 vs. 6) and female (n=8 *vs.* 4) mice (ages 302-349 days). Right panel: *Mrap2*^{+/+} *vs. Mrap2*^{-/-} food-restricted, weight-matched male (n=4 *vs.* 6, weight=27.4±0.6 *vs*. 24.8±0.2 g), and female (n=4 *vs*. 6, weight=21.5±0.6 vs. 20.8±0.2 g) mice (ages 135-151 days). *p=.005, **p<.00001. Fig. 5C depicts the level of fasting serum insulin in *Mrap2*^{+/+} and *Mrap2*^{-/-} male (n=4 *vs.* 6) and female (n=6 *vs.* 8) mice (ages 88-194 days). There was no significant difference between genotypes of either gender. Fig. 5D depicts the results of a glucose tolerance test (intraperitoneal) in *Mrap2*^{+/+} and *Mrap2*^{-/-} male (n=4 vs. 6) and female (n=6 vs. 8) mice (ages 88-194 days). There was no significant difference between genotypes of either gender. Fig. 5E depicts levels of blood corticosterone in morning, evening, and following 30 minutes of restraint stress in *Mrap2*^{+/+} and *Mrap2*^{-/-} male (n=4 vs. 3) and female (n=6 vs. 7) mice (ages 124-178 days). With all 3 conditions, there were no significant differences between genotypes of either gender. (G) Urine catecholamine excretion in *Mrap2*^{+/+} *vs. Mrap2*^{-/-} male (n=8 vs. 8) and female (n=9 vs. 10) mice (age 90 days). In males but not in females, epinephrine and norepinephrine differed significantly between the genotypes. *p=.02, **p<.006. Fig. 5F depicts the uncoupling protein (Ucpl) mRNA content in BAT from in *Mrap2*^{+/+} *vs. Mrap2*^{-/-} mice (ages 3-9 months) at 22°C (male n=4 vs. 4, female n=6 vs. 3) and after 18 h at 4°C (male n=8 vs. 7, female n=7 vs. 5). *p=.1, **p=.02, ***p=.003. Fig. 5H depicts Agrp and Pomc mRNA content in hypothalami of *ad libitum* standard chow diet-fed *Mrap2*^{+/+} *vs. Mrap2*^{-/-} male mice (age 40 days, n=5 vs. 6), collected at 1600 h. Agrp but not Pomc mRNA differed significantly between genotypes, *p=.03. Fig. 5I depicts graphs of food intake (upper panels) and body weight (lower panels) averaged over a 4-day period in *Mrap2*^{+/+} *vs. Mrap2*^{-/-} male (n=9 vs. 7) and female (n=13 vs. 11) mice (age 42 days). For both genders, weight but not food intake differed significantly between the two genotypes. *p=.01, **p=.004. Fig. 5J depicts graphs of food intake (upper panels) and body weight (lower panels) averaged over a 4-day period in *Mrap2*^{+/+} *vs. Mrap2*^{-/-} male (n=14 *vs*. 19) and female (n=18 *vs.* 15) mice (age 84 days). For both genders, weight but not food intake differed significantly between the two genotypes. *p<.00001. Fig. 5K depicts graphs of fecal energy content in *Mrap2*^{+/+} and *Mrap2*^{-/-} male (left panel, n=5 vs. 4) and female (right panel, n=7 *vs*. 5) mice at ages 42 and 84 days. At both ages, there was no significant difference between genotypes of either gender. Fig. 5L depicts graphs of cumulative food intake (upper panels) and weight gain (lower panels) from 34-44 days of age in *ad libitum* fed *Mrap*^{+/+} *vs. Mrap2*^{-/-} males (n=10 vs. 11) and females (n=11 vs. 8). Inset in lower panel documents significantly greater weight gain in *Mrap2*^{-/-} vs*. Mrap*^{+/+} mice. Fig. 5M depicts graphs of cumulative food intake (upper panels) and weight (lower panels) in *ad libitum* fed *Mrap*^{+/+} *vs.* pair-fed (PF) *Mrap2*^{-/-} mice males (n=10 *vs*. 11) and females (n=11 *vs*. 10). Fig. 5N depicts graphs of cumulative food intake (upper panels) and weight gain (lower panels) from 50-72 days of age in pair-fed *Mrap*^{*+*/}*⁺ vs. Mrap2*^{-/-} males (n=10 vs. 11) and females (n=11 vs. 10). Inset in lower panel documents significantly greater weight gain in *Mrap2*^{-/-} vs. *Mrap*^{*+*/*+*} mice. Fig. 5O depicts graphs of cumulative food intake (upper panels) and weight (lower panels) in *ad libitum* fed *Mrap*^{*+*/}*⁺ vs.* restricted-fed (RF) *Mrap2*^{-/-} males (n=16 vs. 30) and females (n=16 vs. 21). At 69 days of age (upward arrow) 10 RF *Mrap2*^{-/-} males and 7 RF *Mrap2*^{-/-} females were released to *ad libitum* feeding, and the remainder continued on RF. Fig. 5P depicts graphs of food intake following saline or MTII (2 µg/g body weight, i.p.) in *Mrap*^{+/+} *vs. Mrap2*^{-/-} males (n=4 vs. 4) and females (n=6 vs. 7), ages 38-45 days. *p=.056, **p=.019, ***p=.016, ****p<.008. Fig. 5Q depicts graphs of respiratory exchange ratio (RER) in *Mrap*^{+/+} *vs. Mrap2*^{-/-} males (n=3 vs. 5) and females (n=4 vs. 3), ages 27-31 days. Upper panels, continuous measurement over 3 days. Lower panels, integrated measurement over 12 h periods, daytime vs. nighttime. Fig. 5R depicts graphs of locomotor activity in *Mrap*^{+/+} *vs. Mrap2*^{-/-} males (n=18 vs. 14) and females (n=20 vs. 22), ages 31-43 days. Upper panels, continuous measurement over 3 days. Lower panels, integrated measurement over 12 h periods, daytime vs. nighttime. For both genders, there were no significant differences between genotypes. Fig. 5S depicts graphs of core body temperature in *Mrap*^{*+*/}*⁺ vs. Mrap2*^{-/-} mice (ages 243-261 days). Upper panels: Diurnal temperature rhythm at 22°C in male (n=7 vs. 5) and female (n=5 vs. 5) mice. Lower panels: Body temperature at 16:00 h, 22°C (male n=7 vs. 14, female n= 8 *vs*. 10) and following placement at 4°C (downward arrow, male n=3 vs. 4, female n=2 vs. 2). For both genders, there were no significant temperature differences between genotypes in either diurnal rhythm or response to 4°C.

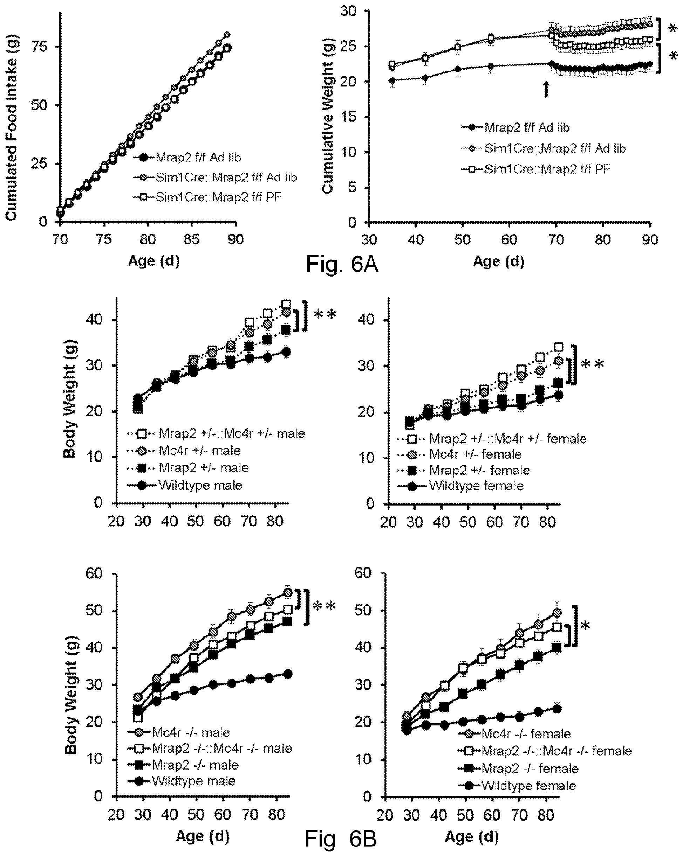

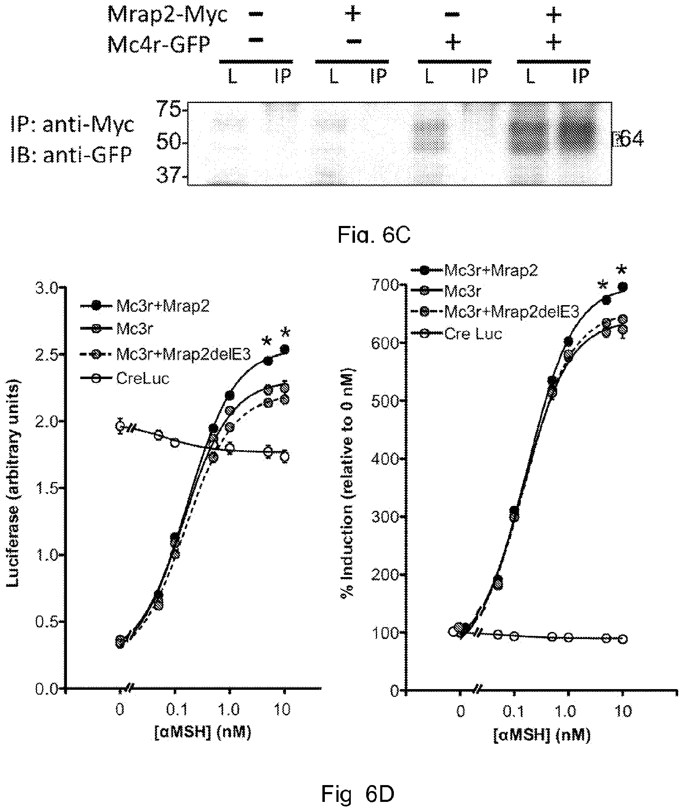

Figs. 6A-6D demonstrate the interaction between Mrap2 and Mc4r. Fig. 6A depicts graphs of food intake (left panel) and body weight (right panel) in female *Mrap2*^{*flox*/}*^{flox} vs. Sim1^{Cre}*::*Mrap2*^{*flox*/*flox*} mice. *Sim1^{Cre}*::*Mrap2*^{*flox*/*flox*} mice were either *ad libitum*-fed or pair-fed starting at age 70 days (upward arrow) to *ad libitum-fed Mrap2*^{*flox*/*flox*} mice. Body weights were significantly different between all three conditions. *p<.001 by ANOVA. Fig. 6B depicts graphs of body weights in mice with combined knockout of *Mrap2* and *Mc4r.* Top panels: Body weights in mixed *Mrap*^{+/-} ::*Mc4r*^{+/-} genotypes (male *Mrap*^{+/-}::*Mc4r*^{+/-}, n=10; *Mrap*^{+/+}::*M*c*4r*^{+/-}, n=10; *Mrap*^{+/-}::*Mc4r*^{+/+}, n=8; *Mrap*^{+/+}::*Mc4r*^{+/+}, n=9; female *Mrap*^{+/-}::*Mc4r*^{+/-}, n=9; *Mrap*^{+/+}::*Mc4r*^{+/-}, n=12; *Mrap*^{+/-}::*Mc4r*^{+/+}, n=8; *Mrap*^{+/+}::*Mc4r*^{+/+}, n=8). Bottom panels: Body weights in mixed *Mrap*^{-/-}::*Mc4r*^{-/-} genotypes (male *Mrap*^{-/-}::*Mc4r*^{-/-}, n=9; *Mrap*^{+/+}::*Mc4r*^{-/-}, n=8; *Mrap*^{-/-}::*Mc4r*^{+/+}, n=8; *Mrap*^{+/+}::*Mc4r*^{+/+}, n=9; female *Mrap*^{-/-}::*Mc4r*^{-/-}, n=7; *Mrap*^{+/+}::*Mc4r*^{-/-}, n=8; *Mrap*^{-/-}::*Mc4r*^{+/+}, n=9; *Mrap*^{+/+}::*Mc4r*^{+/+}, n=8). *p<.05, **p<.001 by ANOVA. Fig. 6C depicts the results of co-immunoprecipitation assay between Mrap2 and Mc4r. Transfection into CHO cells of Myc-tagged Mrap2 and GFP-tagged Mc4r was followed by anti-Myc immunoprecipitation and anti-GFP immunoblotting. L: input lysate; IP: immunoprecipitation. 64 kD Mc4r-GFP is present only in cell lysates transfected with Mc4r-GFP, and only in immunoprecipitates transfected with both constructs. Molecular weight markers (in kD) on left. Fig. 6D depicts graphs of the effect of Mrap2 on Mc3r signaling. Left panel: Level of cAMP reporter activity (CRELuc) in CHO cells alone, or co-transfected with *Mc3r,* with or without *Mrap2* or the *Mrap2* knockout construct, *Mrap2delE3,* 5 h following exposure to 0-10 nM αMSH (n=3/group). Right panel: cAMP activity of these same constructs, expressed as percent induction following 0-10 nM αMSH, relative to 0 nM αMSH. *p<.0001, Mc3r+Mrap2 *vs*. Mc3r at same [αMSH], by ANOVA. For most data points, error bars are obscured by symbols.

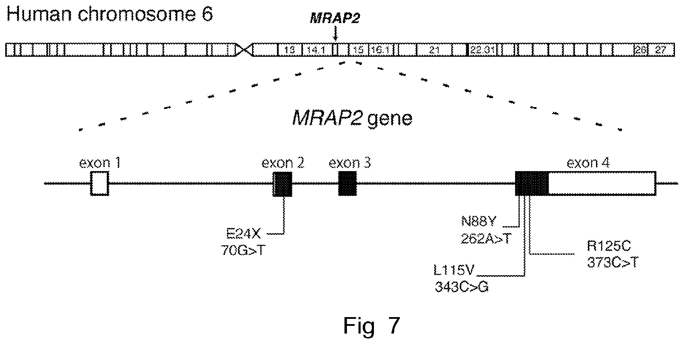

Fig. 7 depicts mutations in *MRAP2* associated with human obesity. The 4 *MRAP2* exons are depicted in rectangles, with coding regions in black. 4 missense variants associated with severe obesity are present in exons 2 and 4.

### DETAILED DESCRIPTION

As described herein, the inventors have discovered that knock-outs of Mrap2 modulate melanocortin receptor signaling in the brain such that the knock-out animal exhibits accelerated weight gain during development, reaching a mature weight much more rapidly than a wildtype animal. Provided herein are cells and pigs with reduced and/or undetectable levels of Mrap2 and/or Mrap2 activity. Such pigs will reach mature weight in less time, and accordingly, by consuming fewer resources, than wildtype animals, permitting more efficient production of various animal products (e.g. meat, fiber, milk, and other products).

As used herein, "Mrap2" or "melanocortin receptor accessory protein 2" refers to a transmembrane expressed in the brain, adrenal glands, and hypothalamus that interacts with the five melanocortin receptors (MCRs) to modulate their signaling. The Mrap2 gene comprises 3 exons, encoding a protein subject to N-linked glycosylation at the N-terminus (specifically, at N9). Mrap2 comprises an N-terminal domain (amino acids 1-44 of SEQ ID NO: 1), a transmembrane domain (amino acids 45-67 of SEQ ID NO: 1), and a C-terminal domain (amino acids 68-205 of SEQ ID NO: 1). The sequence of Mrap2 for a number of species is known, e.g. human Mrap2 (NCBI Gene ID No: 112609; polypeptide (NCBI Ref Seq: NP_612418, SEQ ID NO: 1); mRNA (NCBI Ref Seq: NM_138409; SEQ ID NO: 2)) and porcine Mrap2 (NCBI Gene ID No: 100515980; polypeptide (NCBI Ref Seq: XP_003353296, SEQ ID NO: 3); mRNA (NCBI Ref Seq: XM_003353248; SEQ ID NO: 4)). Further details of the structure and function of Mrap2 can be found, e.g. in Chan et al. PNAS 2009 106:6146-6151 and Hofland et al. J Clin Endocrinol Metab 2012 97:E747-E754.

The Mrap2 gene is considered any sequence associated with the Mrap2 locus. Thus, it would at least include the chromosomal nucleic acid contained within any organism that expresses a Mrap2, such as, the introns, exons, 5' upstream sequence involved with the Mrap2 coding and noncoding sequence, and 3' downstream sequence involved with the Mrap2 coding and non coding sequence. Exon 1 of Mrap2 consists of the nucleotides corresponding to positions 1-183 of SEQ ID NO: 2; exon 2 of Mrap2 consists of the nucleotides corresponding to positions 184-317 of SEQ ID NO: 2; exon 3 of Mrap2 consists of the nucleotides corresponding to positions 318-417 of SEQ ID NO: 2, and exon 4 of Mrap2 consists of the nucleotides corresponding to positions 418-2214 of SEQ ID NO: 2. The coding sequence of Mrap2 consists of the nucleotides corresponding to positions 191-808 of SEQ ID NO: 2.

Mrap2 functions by interacting with melanocortin receptors, particularly Mc4r in the brain. Via this interaction, it enhances the generation of cyclic AMP (cAMP) mediated by Mc4R. In some embodiments, the functionality of Mrap2 can be measured using a bioassy for Mc4r activity. For example, cells expressing Mc4r can be contacted with α-melanocyte stimulating hormone. The addition of α-melanocyte stimulating hormone will induce PKA activity (which can be measured, e.g. by directly assaying the level of cAMP, or by measuring the level of a reporter, e.g. pCRE-LUC, which is induced in response to cAMP production) if a functional Mc4r and a functional Mrap2 are present. A cell lacking a detectable level of functional Mrap2 will not display a level of induction of PKA activity that is greater than the level of activity prior to the addition of α-melanocyte stimulating hormone, e.g. a level of induction that is less than 3x, less than 2x, or less than 1.5x, of the level prior to addition of the hormone.

By transfecting cells with altered versions of Mrap2 and measuring the induction of PKA activity by α-melanocyte stimulating hormone, it is possible to determine if that alteration is a modification that renders Mrap2 non-functional.

In some embodiments, a modification rendering Mrap2 nonfunctional is a modification that results in an inhibition of expression of Mrap2, or in an inhition of the expression of Mrap2 lacking domains required for functionality, as described elsewhere herein. The level of Mrap2 expression can be measured by methods known in the art for determining the level of nucleic acid and/or polypeptide expression, including, but not limited to, RT-PCR, qRT-PCR, northern blotting, western blotting, immunochemistry, and the like. Primers suitable for amplification of a Mrap2 expression product are provided herein in Table 4. Antibodies suitable for the detection of various domains of Mrap2 are described in the Examples herein. In some embodiments, a commercial antibody for Mrap2 can be used with the methods described herein, e.g., an antibody specific for the transmembrane and/or C-terminal domains of Mrap2 (ab129397 from Abcam, Cambridge, MA).

As used herein, "functional" refers to a portion and/or variant of a polypeptide that retains at least a detectable level of the activity of the native polypeptide from which it is derived. Methods of detecting, e.g. Mrap2 activity and/or functionality are described elsewhere herein. In some embodiments, a functional fragment can retain at least 50% of the activity of the native polypeptide, e.g. 50% or more of the activity, 60% or more of the activity, 75% or more of the activity, or 90% or more of the activity of the native polypeptide. A "variant," as referred to herein, is a polypeptide substantially homologous to a native or reference polypeptide, but which has an amino acid sequence different from that of the native or reference polypeptide because of one or a plurality of deletions, insertions or substitutions.

As used herein, "detectable" refers to a level (e.g. a concentration or an activity) which can be distinguished, by a statistically significant amount, from the background or negative control levels. In some embodiments described herein, a reduced level of Mrap2 and/or Mrap2 activity is less than 10% of the level found in a wildtype cell and/or animal of the same type, e.g. 10% or less, 9% or less, 8% or less, 7% or less, 6% or less, 5% or less, 4% or less, 3% or less, 2% or less, or 1% or less.

A "modification" is a detectable change in the genetic material in the animal, which is transmitted to the animal's progeny. A modification is usually a change of one or more deoxyribonucleotides, the modification being obtained by, for example, adding, deleting, inverting, or substituting nucleotides. As described herein, a modification of Mrap2 renders the Mrap2 gene nonfuctional under at least some circumstances, producing a "knockout" animal. In some embodiments, Mrap2 is rendered nonfuctional in any cell comprising the modification. In some embodiments, Mrap2 is rendered nonfunctional in a specific cell type comprising the modification, e.g. in the case of tissue-specific recombinase modifications, as described below herein.

In some embodiments described herein, the genome of the cell and/or animal can comprise a modification of Mrap2, such that a detectable level of Mrap2 is not produced in the cell and/or animal. In some embodiments described herein, the genome of the cell and/or animal can comprise one or more deletions in one or more exons of Mrap2. In some embodiments, the genome of the cell and/or animal can comprise a stop codon inserted into the Mrap2 sequence such that the translation of the peptide terminates early, e.g. before the transmembrane domain and/or the C-terminal domain are translated.

A knock-out cell or animal as described herein can have a modification of Mrap2 present in one or both copies of Mrap2. In the latter case, in which a homozygous modification is present, the mutation is termed a "null" mutation. In the case where only one copy of Mrap2 is modified, the knockout animal is termed a "heterozygous knockout animal". The pigs for use in the method of the invention are typically homozygous for the disruption of both copies of Mrap2.

In some embodiments described herein, the genome of the transgenic non-human animal can further comprise a heterologous selectable marker gene, e.g. a marker that is introduced into the genome with the modification of Mrap2.

A cell or animal as described herein can be of any non-human species, e.g. a mammalian species. In some embodiments described herein, the cell or animal can be a species that is raised for agricultural purposes, e.g. for the use of a product (e.g. milk or fiber) produced by the animl or for the use of its carcass (e.g. for meat for consumption). In some embodiments described herein, the cell or animal can be a species and/or variety that is domesticated. In some embodiments, the cell or animal can be, e.g. a fish, bird or reptile. In some embodiments, the cell or animal can be a non-human mammal. Non-limiting examples of mammals disclosed herein can include, pigs, cows, sheep, goats, rabbits, alpaca, buffalo, bison, camel, cats, deer, donkey, dog, gayal, guinea pig, horse, llama, mule, reindeer, elk, yak. In the present invention the non-human animal is a pig.

A cell as described herein can be any type of cell, including but not limited to, an embryonic stem cell, an embryonic cell, a germline cell, a somatic cell, a brain cell, a neuron, a cell that can be cultured (e.g. an ES cell), a progenitor cell, a stem cell, or the like.

In some aspects, described herein is a transgenic Mrap2 knockout cell and/or animal, e.g. one in which the Mrap2 coding sequence is not modified, but where expression of functional Mrap2 is not detectable. In some embodiments, a modification can be introduced into the genome at a location other than at the Mrap2 gene. Such a transgenic Mrap2 knockout can comprise an antisense molecule targeting the Mrap2 gene.

In some embodiments, the modified Mrap2 gene comprises sites for recombination by a recombinase, e.g. wherein the sites are lox sites and the recombinase is cre recombinase. When the recombinase is present, the nucleic acid sequence between the recombinase sites will be excised from the genome, creating a deletion, e.g. of a portion of Mrap 2 which renders it non-functional as described elsewhere herein.

In some embodiments, the modification is produced by the action of a recombinase. In some embodiments, the recombinase is cre recombinase. In some embodiments, the recombinase is under the control of a tissue-specific promoter. In some embodiments, the promoter is specific for neurons or brain tissue, e.g. the Sim1 promoter. Promoters specific for neurons and/or brain tissue are known in the art, e.g. the promoters of myelin basic protein, prolactin, GFAP, NSE, nestin, synapsin I, preproenkephalin, and dopamine beta-hydroxylase. See, e.g Papadakis et al. Current Gene Therapy 2004 4:89-113.

In some aspects, described herein are animals comprising or consisting of cells as described herein. The animal is a pig.

In one aspect, described herein is an animal comprising a modification of the Mrap2 gene, wherein the mammal does not express a detectable level of functional Mrap2. In some embodiments described herein, the animal is a non-human mammal. In some embodiments described herein, the animal is a transgenic animal. In some embodiments described herein, the animal is a knockout animal.

Techniques for obtaining the cells and/or animals described herein are well known in the art. Non-limiting examples of methods of introducing a modification into the genome of a cell and/or animal can include microinjection, viral delivery, recombinase technologies, homologous recombination, TALENS, CRISPR, and/or ZFN, see, e.g. Clark and Whitelaw Nature Reviews Genetics 2003 4:825-833.

Zinc finger nucleases (ZFNs), the Cas9/ CRISPR system, and transcription-activator like effector nucleases (TALENs) are meganucleases. Meganucleases are found commonly in microbial species and have the unique property of having very long recognition sequences (>14bp) thus making them naturally very specific for cutting at a desired location. This can be exploited to make site-specific double-stranded breaks in, e.g. a genome. These nucleases can cut and create specific double-stranded breaks at a desired location(s) in the genome, which are then repaired by cellular endogenous processes such as, homologous recombination (HR), homology directed repair (HDR) and non-homologous end-joining (NHEJ). NHEJ directly joins the DNA ends in a double-stranded break, while HDR utilizes a homologous sequence as a template for regenerating the missing DNA sequence at the break point. Thus, by introducing a ZFN, CRISPR, and/or TALENs specific for Mrap2 into a cell, at least one double strand-break can be generated in Mrap2, resulting in an excision of at least part of the Mrap2 gene (i.e. introducing a modification as described herein) (see, e.g. Gaj et al. Trends in Biotechnology 2013 31:397-405; Carlson et al. PNAS 2012 109:17382-7; and Wang et al. Cell 2013 153:910-8. Alternatively, if a specifically-designed homologous donor DNA is provided in combination with, e.g., the ZFNs, this template can result in gene correction or insertion, as repair of the DSB can include a few nucleotides changed at the endogenous site or the addition of a new and/or modified gene at the break site. One of skill in the art can use these naturally occurring meganucleases, however the number of such naturally occurring meganucleases is limited.

To overcome this challenge, mutagenesis and high throughput screening methods have been used to create meganuclease variants that recognize unique sequences. For example, various meganucleases have been fused to create hybrid enzymes that recognize a new sequence. Alternatively, DNA interacting amino acids of the meganuclease can be altered to design sequence specific meganucleases (see e.g., US Patent 8,021,867). Meganucleases can be designed using the methods described in e.g., Certo, MT et al. Nature Methods (2012) 9:073-975; U.S. Patent Nos. 8,304,222; 8,021,867; 8,119,381; 8,124,369; 8,129,134; 8,133,697; 8,143,015; 8,143,016; 8,148,098; or 8,163,514. Alternatively, meganucleases with site specific cutting characteristics can be obtained using commercially available technologies e.g., Precision BioSciences' Directed Nuclease Editor™ genome editing technology.

ZFNs and TALENs restriction endonuclease technology utilizes a non-specific DNA cutting enzyme which is linked to a specific DNA sequence recognizing peptide(s) such as zinc fingers and transcription activator-like effectors (TALEs). Typically an endonuclease whose DNA recognition site and cleaving site are separate from each other is selected and its cleaving portion is separated and then linked to a sequence recognizing peptide, thereby yielding an endonuclease with very high specificity for a desired sequence. An exemplary restriction enzyme with such properties is FokI. Additionally FokI has the advantage of requiring dimerization to have nuclease activity and this means the specificity increases dramatically as each nuclease partner recognizes a unique DNA sequence. To enhance this effect, FokI nucleases have been engineered that can only function as heterodimers and have increased catalytic activity. The heterodimer functioning nucleases avoid the possibility of unwanted homodimer activity and thus increase specificity of the double-stranded break.

Although the nuclease portions of both ZFNs and TALENs have similar properties, the difference between these engineered nucleases is in their DNA recognition peptide. ZFNs rely on Cys2-His2 zinc fingers and TALENs on TALEs. Both of these DNA recognizing peptide domains have the characteristic that they are naturally found in combinations in their proteins. Cys2-His2 Zinc fingers typically happen in repeats that are 3 bp apart and are found in diverse combinations in a variety of nucleic acid interacting proteins such as transcription factors. TALEs on the other hand are found in repeats with a one-to-one recognition ratio between the amino acids and the recognized nucleotide pairs. Because both zinc fingers and TALEs happen in repeated patterns, different combinations can be tried to create a wide variety of sequence specificities. Approaches for making site-specific zinc finger endonucleases include, e.g., modular assembly (where Zinc fingers correlated with a triplet sequence are attached in a row to cover the required sequence), OPEN (low-stringency selection of peptide domains vs. triplet nucleotides followed by high-stringency selections of peptide combination vs. the final target in bacterial systems), and bacterial one-hybrid screening of zinc finger libraries, among others. ZFNs for use with the methods and compositions described herein can be obtained commercially from e.g., Sangamo Biosciences™ (Richmond, CA).

In some embodimetns, the Cas9/ CRISPR system can be used to create a modification in an Mrap2 gene as described herein. Clustered regularly interspaced short palindromic repeats (CRISPR)/CRISPR-associated (Cas) systems are useful for, e.g. RNA-programmable genome editing (see e.g., Marraffini and Sontheimer. Nature Reviews Genetics 2010 11:181-190; Sorek et al. Nature Reviews Microbiology 2008 6:181-6; Karginov and Hannon. Mol Cell 2010 1:7-19; Hale et al. Mol Cell 2010:45:292-302; Jinek et al. Science 2012 337:815-820; Bikard and Marraffini Curr Opin Immunol 2012 24:15-20; Bikard et al. Cell Host & Microbe 2012 12:177-186. A CRISPR guide RNA is used that can target a Cas enzyme to the desired location in the genome, where it generates a double strand break. This technique is known in the art and described, e.g. at Mali et al. Science 2013 339:823-6; and kits for the design and use of CRISPR-mediated genome editing are commercially available, e.g. the PRECISION X CAS9 SMART NUCLEASE™ System (Cat No. CAS900A-1) from System Biosciences, Mountain View, CA.

In some embodiments, a CRISPR, TALENs, or ZFN molecule (e.g. a peptide and/or peptide/nucleic acid complex) can be introduced into a cell, e.g. a cultured ES cell, such that the presence of the CRISPR, TALENs, or ZFN molecule is transient and will not be detectable in the progeny of, or an animal derived from, that cell. In some embodiments, a nucleic acid encoding a CRISPR, TALENs, or ZFN molecule (e.g. a peptide and/or multiple nucleic acids encoding the parts of a peptide/nucleic acid complex) can be introduced into a cell, e.g. a cultured ES cell, such that the nucleic acid is present in the cell transiently and the nucleic acid encoding the CRISPR, TALENs, or ZFN molecule as well as the CRISPR, TALENs, or ZFN molecule itself will not be detectable in the progeny of, or an animal derived from, that cell. In some embodiments, a nucleic acid encoding a CRISPR, TALENs, or ZFN molecule (e.g. a peptide and/or multiple nucleic acids encoding the parts of a peptide/nucleic acid complex) can be introduced into a cell, e.g. a cultured ES cell, such that the nucleic acid is maintained in the cell (e.g. incorporated into the genome) and the nucleic acid encoding the CRISPR, TALENs, or ZFN molecule and/or the CRISPR, TALENs, or ZFN molecule will be detectable in the progeny of, or an animal derived from, that cell.

By way of non-limiting example, a TALENs targeting the 5' end of exon 3 of Mrap2 and a TALENs targeting the 3' end of exon 3 of Mrap2 can be introduced into a cell, thereby causing a modification of Mrap2 in which exon 3 is deleted.

Alternatively, a modification in Mrap2 can be introduced into the genome of a cell using recombinant adeno-associated virus (rAAV) based genome engineering, which is a genome-editing platform centered around the use of rAAV vectors that enables insertion, deletion or substitution of DNA sequences into the genomes of live mammalian cells. The rAAV genome is a single-stranded deoxyribonucleic acid (ssDNA) molecule, either positive- or negative-sensed, which is about 4.7 kilobase long. These single-stranded DNA viral vectors have high transduction rates and have a unique property of stimulating endogenous homologous recombination in the absence of causing double strand DNA breaks in the genome. One of skill in the art can design a rAAV vector to target a desired genomic locus and perform both gross and/or subtle endogenous gene alterations in a cell, such as a deletion. rAAV genome editing has the advantage in that it targets a single allele and does not result in any off-target genomic alterations. rAAV genome editing technology is commercially available, for example, the rAAV GENESIS™ system from Horizon™ (Cambridge, UK).

In some embodiments of the various aspects described herein, a targeting vector can be used to introduce a modification of Mrap2. A "targeting vector" is a vector comprising sequences that can be inserted into the gene to be disrupted, e.g., by homologous recombination. The targeting vector generally has a 5' flanking region and a 3' flanking region homologous to segments of the gene of interest, surrounding a DNA sequence comprising a modification and/or a foreign DNA sequence to be inserted into the gene. For example, the foreign DNA sequence may encode a selectable marker, such as an antibiotics resistance gene. Examples for suitable selectable markers are the neomycin resistance gene (NEO) and the hygromycin β-phosphotransferase gene. The 5' flanking region and the 3' flanking region are homologous to regions within the gene surrounding the portion of the gene to be replaced with the unrelated DNA sequence. In some embodiments, the targeting vector does not comprise a selectable marker. DNA comprising the targeting vector and the native gene of interest are contacted under conditions that favor homologous recombination. For example, the targeting vector can be used to transform embryonic stem (ES) cells, in which they can subsequently undergo homologous recombination.

A typical targeting vector contains nucleic acid fragments of not less than about 0.5 kb nor more than about 10.0 kb from both the 5' and the 3' ends of the genomic locus which encodes the gene to be modified (e.g. Mrap2). These two fragments are separated by an intervening fragment of nucleic acid which encodes the modification to be introduced. When the resulting construct recombines homologously with the chromosome at this locus, it results in the introduction of the modification, e.g. a deletion of an exon or the insertion of a stop codon.

The homologous recombination of the above-described targeting vectors is sometimes rare and such a construct can insert nonhomologously into a random region of the genome where it has no effect on the gene which has been targeted for deletion, and where it can potentially recombine so as to disrupt another gene which was otherwise not intended to be altered. In some embodiments, such non-homologous recombination events can be selected against by modifying the above-mentioned targeting vectors so that they are flanked by negative selectable markers at either end (particularly through the use of two allelic variants of the thymidine kinase gene, the polypeptide product of which can be selected against in expressing cell lines in an appropriate tissue culture medium well known in the art-i.e. one containing a drug such as 5-bromodeoxyuridine). Non-homologous recombination between the resulting targeting vector comprising the negative selectable marker and the genome will usually result in the stable integration of one or both of these negative selectable marker genes and hence cells which have undergone non-homologous recombination can be selected against by growth in the appropriate selective media (e.g. media containing a drug such as 5-bromodeoxyuridine for example). Simultaneous selection for the positive selectable marker and against the negative selectable marker will result in a vast enrichment for clones in which the targeting vector has recombined homologously at the locus of the gene intended to be mutated.

In some embodiments, each targeting vector to be inserted into the cell is linearized. Linearization is accomplished by digesting the DNA with a suitable restriction endonuclease selected to cut only within the vector sequence and not the 5' or 3' homologous regions or the modification region.

Thus, a targeting vector refers to a nucleic acid that can be used to decrease or suppress expression of a protein encoded by endogenous DNA sequences in a cell. In a simple example, the knockout construct is comprised of a Mrap2 polynucleotide with a deletion in a critical portion of the polynucleotide (e.g. the transmembrane domain) so that a functional Mrap2 cannot be expressed therefrom. Alternatively, a number of termination codons can be added to the native polynucleotide to cause early termination of the protein or an intron junction can be inactivated. Proper homologous recombination can be confirmed by Southern blot analysis of restriction endonuclease digested DNA using, as a probe, a non-modifed region of the gene. Since the native gene will exhibit a restriction pattern different from that of the disrupted gene, the presence of a disrupted gene can be determined from the size of the restriction fragments that hybridize to the probe.

A targeting vector can comprise the whole or a fragment of the genomic sequence of a Mrap2 and optionally, a selection marker, e.g., a positive selection marker. Several kilobases of unaltered flanking DNA (both at the 5' and 3' ends) can be included in the vector (see e.g., Thomas and Capecchi, (1987) Cell, 51:503 for a description of homologous recombination vectors). In one aspect of the invention, the genomic sequence of the Mrap2 gene comprises at least part of an exon of Mrap2.

A selection marker of the invention can include a positive selection marker, a negative selection marker or include both a positive and negative selection marker. Examples of positive selection marker include but are not limited to, e.g., a neomycin resistance gene (neo), a hygromycin resistance gene, etc. In one embodiment, the positive selection marker is a neomycin resistance gene. In certain embodiments of the invention, the genomic sequence further comprises a negative selection marker. Examples of negative selection markers include but are not limited to, e.g., a diphtheria toxin gene, an HSV-thymidine kinase gene (HSV-TK), etc.

The term "modifier" is used herein to collectively refer to any molecule which can effect a modification of Mrap2, e.g. a targeting vector or a TALENs, CRISPR, or ZFN molecule, complex, and/or one or more nucleic acids encoding such a molecule or the parts of such a complex.

A modifier can be introduced into a cell by any technique that allows for the addition of the exogenous genetic material into nucleic genetic material can be utilized so long as it is not destructive to the cell, nuclear membrane, or other existing cellular or genetic structures. Such techniques include, but are not limited to transfection, scrape-loading or infection with a vector, pronuclear microinjection (U.S. Pat. Nos. 4,873,191, 4,736,866 and 4,870,009); retrovirus mediated transfer into germ lines (an der Putten, et al., Proc. Natl. Acad. Sci., USA, 82:6148-6152 (1985)); gene targeting in embryonic stem cells (Thompson, et al., Cell. 56:313-321 (1989)); nonspecific insertional inactivation using a gene trap vector (U.S. Pat. No. 6,436,707); electroporation of embryos (Lo, Mol. Cell. Biol., 3:1803-1814 (1983)); lipofection; and sperm-mediated gene transfer (Lavitrano, et al., Cell. 57:717-723 (1989)); These methods and compositions can largely be broken down into two classes: viral based delivery systems and non-viral based delivery systems. For example, the modifier can be delivered through a number of direct delivery systems such as, electroporation, lipofection, calcium phosphate precipitation, plasmids, viral vectors, viral nucleic acids, phage nucleic acids, phages, cosmids, or via transfer of material in cells or carriers such as cationic liposomes. Appropriate means for transfection, including viral vectors, chemical transfectants, or physico-mechanical methods such as electroporation and direct diffusion of DNA, are described by, for example, Wolff, J. A., et al., Science, 247, 1465-1468, (1990); and Wolff, J. A. Nature, 352, 815-818, (1991); each of which are incorporated by reference herein in its entirety. Such methods are well known in the art and readily adaptable for use with the compositions and methods described herein. The methods described herein can be used to deliver a modifier to any cell type, e.g. a germline cell, a zygote, an embryo, or a somatic cell. The cells can be cultured in vitro or present in vivo. Non-limiting examples are provided herein below.

In one example, a modifier inserted into the nucleic genetic material by microinjection. Microinjection of cells and cellular structures is known and is used in the art. Following introduction of the transgene nucleotide sequence into the embryo, the embryo may be incubated in vitro for varying amounts of time, or reimplanted into the surrogate host, or both. In vitro incubation to maturity is within the scope of this invention. One common method is to incubate the embryos in vitro for about 1-7 days, depending on the species, and then reimplant them into the surrogate host. In some embodiments, a zygote is microinjected. The use of zygotes as a target for modification of a host gene has an advantage in that in most cases the injected DNA will be incorporated into the host gene before the first cleavage (Brinster et al. (1985) PNAS 82:4438-4442). As a consequence, all cells of the transgenic animal will carry the incorporated nucleic acids of the targeting vector. This will in general also be reflected in the efficient transmission to offspring of the founder since 50% of the germ cells will harbor the modification. One route of introducing foreign DNA into a germ line entails the direct microinjection of linear DNA molecules into a pronucleus of a fertilized one-cell egg. Microinjected eggs are subsequently transferred into the oviducts of pseudopregnant foster mothers and allowed to develop. About 25% of the progeny inherit one or more copies of the micro-injected DNA. Techniques suitable for obtaining transgenic animals have been amply described. A suitable technique for obtaining completely ES cell derived transgenic non-human animals is described in WO 98/06834.

In some embodiments, a modifier can be introduced into a cell by electroporation. The cells and the targeting vector can be exposed to an electric pulse using an electroporation machine and following the manufacturer's guidelines for use. After electroporation, the cells are typically allowed to recover under suitable incubation conditions. The cells are then screened for the presence of the targeting vector as explained herein.

Retroviral infection can also be used to introduce a nucleic acid modifier (e.g. a targeting vector) or a nucleic acid encoding a modifier into a cell, e.g. a non-human animal cell.

In some embodiments, a retrovirus can be used to introduce the Mrap2 modification to a cell or cells, e.g. an embryo. For example, the developing non-human embryo can be cultured in vitro to the blastocyst stage. During this time, the blastomeres can be targets for retroviral infection (Jaenich, Proc. Natl. Acad. Sci. USA, 73:1260-1264 (1976)). Efficient infection of the blastomeres is obtained by enzymatic treatment to remove the zona pellucida (Manipulating the Mouse Embryo, Hogan, ed. (Cold Spring Harbor Laboratory Press, Cold Spring Harbor, N.Y., 1986)). The viral vector system used to introduce the modifier is typically a replication-defective retrovirus carrying the transgene (Jahner et al., Proc. Natl. Acad. Sci. USA, 82: 6972-6931 (1985); and, Van der Putten et al., Proc. Natl. Acad. Sci. USA, 82: 6148-6152 (1985)). Transfection is easily and efficiently obtained by culturing the blastomeres on a monolayer of virus-producing cells (Van der Putten et al., supra; and, Stewart et al., EMBO J., 6: 383-388 (1987)). Alternatively, infection can be performed at a later stage. Virus or virus-producing cells can be injected into the blastocoele (Jahner et al., Nature, 298: 623-628(1982)). Most of the founders will be mosaic for the transgene since incorporation occurs only in a subset of the cells that formed the transgenic non-human animal. In addition, it is also possible to introduce transgenes into the germ line by intrauterine retroviral infection of the mid-gestation embryo (Jahner et al. (1982), supra).

Other viral vectors can include, but are not limited to, adenoviral vectors (Mitani et al., Hum. Gene Ther. 5:941- 948, 1994), adeno-associated viral (AAV) vectors (Goodman et al., Blood 84:1492-1500, 1994), lentiviral vectors (Naidini et al., Science 272:263-267, 1996), pseudotyped retroviral vectors (Agrawal et al., Exper. Hematol. 24:738-747, 1996).

In some embodiments, a modifier can be introduced to a cell by the use of liposomes, e.g. cationic liposomes (e.g., DOTMA, DOPE, DC-cholesterol) or anionic liposomes. Liposomes can further comprise proteins to facilitate targeting a particular cell, if desired. Regarding liposomes, see, e.g., Brigham et al. Am. J. Resp. Cell. Mol. Biol. 1:95-100 (1989); Feigner et al. Proc. Natl. Acad. Sci USA 84:7413-7417 (1987); U.S. Pat. No.4,897,355. Commercially available liposome preparations include, e.g. as LIPOFECTIN, LIPOFECTAMINE (GIBCO-BRL, Inc., Gaithersburg, MD), SUPERFECT (Qiagen, Inc. Hilden, Germany) and TRANSFECTAM (Promega Biotec, Inc., Madison, WI), as well as other liposomes developed according to procedures standard in the art.

The number of copies of a modifier (e.g., the targeting vector or TALENs molecule) which are added to the cell is dependent upon the total amount of exogenous genetic material added and will be the amount which enables the genetic transformation to occur. Theoretically only one copy is required; however, generally, numerous copies are utilized, for example, 1,000-20,000 copies of a targeting vector, in order to insure that one copy is functional.

In some embodiments, cells contacted with a modifier are subsequently screened for accurate targeting to identify and isolate those which have been properly modified at the Mrap2 locus.

Once the cell comprising a modification of Mrap2 is produced through the methods described herein, an animal can be produced from this cell through either stem cell technology or cloning technology. For example, if the cell into which the nucleic acid was transfected was a stem cell for the organism (e.g. an embryonic stem cell), then this cell, after transfection and culturmg, can be used to produce an organism which will contain the gene modification in germ line cells, which can then in turn be used to produce another animal that possesses the gene modification or disruption in all of its cells. In other methods for production of an animal containing the gene modification or disruption in all of its cells, cloning technologies can be used. These technologies generally take the nucleus of the transfected cell and either through fusion or replacement fuse the transfected nucleus with an oocyte which can then be manipulated to produce an animal. The advantage of procedures that use cloning instead of ES technology is that cells other than ES cells can be transfected. For example, a fibroblast cell, which is very easy to culture can be used as the cell which is transfected and has a Mrap2 modification event take place, and then cells derived from this cell can be used to clone a whole animal.

Generally, cells (e.g. ES cells) used to produce the knockout animals will be of the same species as the knockout animal to be generated. Thus for example, pig embryonic stem cells will usually be used for generation of knockout pigs. Methods of isolating, culturing, and manipulating various cells types are known in the art. By way of non-limiting example, embryonic stem cells are generated and maintained using methods well known to the skilled artisan such as those described by Doetschman et al. (1985) J. Embryol. Exp. Mol. Biol. 87:27-45). The cells are cultured and prepared for knockout construct insertion using methods well known to the skilled artisan, such as those set forth by Robertson in: Teratocarcinomas and Embryonic Stem Cells: A Practical Approach, E. J. Robertson, ed. IRL Press, Washington, D.C. [1987]); by Bradley et al. (1986) Current Topics in Devel. Biol. 20:357-371); and by Hogan et al. (Manipulating the Mouse Embryo: A Laboratory Manual, Cold Spring Harbor Laboratory Press, Cold Spring Harbor, N.Y. (1986)).

In some embodiments, after cells comprising the modification of Mrap2 have been generated, and optionally, selected, the cells can be inserted into an embryo. Insertion may be accomplished in a variety of ways known to the skilled artisan, however the typical method is by microinjection. For microinjection, about 10-30 cells are collected into a micropipet and injected into embryos that are at the proper stage of development to permit integration of the ES cell containing the Mrap2 modification into the developing embryo. For instance, the ES cells can be microinjected into blastocytes. The suitable stage of development for the embryo used for insertion of ES cells is very species dependent, however for mice it is about 3.5 days. The embryos are obtained by perfusing the uterus of pregnant females. Suitable methods for accomplishing this are known to the skilled artisan.

In some embodiments, a modification of Mrap2 that renders it nonfunctional can be generated by a recombinase. For example, sites for a recombinase can be inserted into the native Mrap2 gene, such that they flank an area that can be deleted in order to render Mrap2 nonfunctional (e.g. exon 3). In the presence of the recombinase, the flanked area of Mrap2 will be deleted. This permits inducible or tissue-specific modification of Mrap2, e.g. in the brain only.

A widely used site-specific DNA recombination system uses the Cre recombinase, e.g., from bacteriophage P1, or the Flp recombinase from *S*. *cerevisiae,* which has also been adapted for use in animals. The loxP-Cre system utilizes the expression of the PI phage Cre recombinase to catalyze the excision of DNA located between flanking lox sites. By using gene-targeting techniques to produce binary transgene animals with modified endogenous genes that can be acted on by Cre or Flp recombinases expressed under the control of tissue-specific promoters, site-specific recombination may be employed to inactivate endogenous genes in a spatially or time controlled manner. See, e.g., U.S. Pat. Nos. 6,080,576, 5,434,066, and 4,959,317; and Joyner, A. L., et al. Laboratory Protocols for Conditional Gene Targeting, Oxford University Press, New York (1997). The cre-lox system, an approach based on the ability of transgenic mice, carrying the bacteriophage Cre gene, to promote recombination between, for example, 34 by repeats termed loxP sites, allows ablation of a given gene in a tissue specific and a developmentally regulated manner (Orban et al. (1992) PNAS 89:6861-6865). The Cre-lox system has been successfully applied for tissue-specific transgene expression (Orban PC, Chui D, Marth JD. Proc Natl Acad Sci U S A. 1992 Aug 1;89(15):6861-5.), for site specific gene targeting and for exchange of gene sequence by the "knock-in" method (Aguzzi A, Brandner S, Isenmann S, Steinbach JP, Sure U. Glia. 1995 Nov;15(3):348-64. Review).

The recombinase can be delivered at different stages. For example, a recombinase can be added to an embryonic stem cell containing a disrupted gene prior to the production of chimeras or implantation into an animal. In certain embodiments of the invention, the recombinase is delivered after the generation of an animal containing at least one gene allele with introduced recombinase sites. For example, the recombinase is delivered by cross breeding the animal containing a gene with recombinase sites with an animal expressing the recombinase. The animal expressing the recombinase may express it, e.g., ubiquitously, in a tissue-restricted manner, or in a temporal-restricted manner. Cre/Flp activity can also be controlled temporally by delivering cre/FLP-encoding transgenes in viral vectors, by administering exogenous steroids to the animals that carry a chimeric transgene consisting of the cre gene fused to a mutated ligand-binding domain, or by using transcriptional transactivation to control cre/FLP expression. In certain embodiments of the invention, mutated recombinase sites may be used. Tissue-specific, temporally-regulated, and inducible promoters for controlling the expression of, e.g. Cre recombinase are known in the art. By way of non-limiting example, a Sim1 promoter controlled Cre, which permits brain-specific expression of Cre recombinase is described in the Examples herein.

Animals with germline cells comprising the desired modification described herein can be selected, e.g. by genotyping or assaying Mrap2 levels or activity in the germline cells and/or progeny. Non-limiting examples of methods for such genotyping or assaying can include, RNA analysis (Northern blotting or RT-PCR, including qRT-PCR), assays for determing the activity of Mrap2 as described elsewhere herein, protein analysis (e.g. Western blotting), histological stains, flow cytometric analysis and the like. The extent of the contribution of the modified cells in an animal described herein can also be assessed visually by choosing animals strains for the modified cells (e.g. the ES cells that will be modified) and the blastocytes that have different coat colors.

Transgenic offspring can be screened for the presence and/or expression of the transgene by any suitable method. Screening is often accomplished by Southern blot or Northern blot analysis, using a probe that is complementary to at least a portion of the transgene. Typically, DNA is prepared from, e.g. tail tissue and analyzed by Southern analysis or PCR for the transgene. Alternatively, the tissues or cells believed to have a modification of Mrap2 are tested for the presence and expression of the modified Mrap2 using Southern analysis or PCR, although any tissues or cell types may be used for this analysis. See, e.g., southern hybridization. (Southern J. Mol. Biol. 98:503-517 (1975)), northern hybridization (see, e.g., Freeman et al. Proc. Natl. Acad. Sci. USA 80:4094-4098 (1983)), restriction endonuclease mapping (Sambrook et al. (2001) Molecular Cloning, A Laboratory Manual, 3rd Ed. Cold Spring Harbor Laboratory Press, New York), RNase protection assays (Current Protocols in Molecular Biology, John Wiley and Sons, New York, 1997), DNA sequence analysis, and polymerase chain reaction amplification (PCR; U.S. Pat. Nos. 4,683,202, 4,683,195, and 4,889,818; Gyllenstein et al. Proc Natl. Acad. Sci. USA 85:7652-7657 (1988); Ochman et al. Genetics 120:621-623 (1988); and, Loh et al. Science 243:217-220 (1989). Other methods of amplification commonly known in the art can be employed. The stringency of the hybridization conditions for northern or Southern blot analysis can be manipulated to ensure detection of nucleic acids with the desired degree of relatedness to the specific probes used. The expression of gene in a cell or tissue sample can also be detected and quantified using in situ hybridization techniques according to, for example, Current Protocols in Molecular Biology, John Wiley and Sons, New York, 1997.

Protein levels can be detected by immunoassays using antibodies specific to protein. For example, western blot analysis using an antibody against Mra2 or the modified Mrap2 encoded by the transgene may be employed as an alternative or additional method for screening for the presence of the transgene product. Various immunoassays known in the art can be used, including but not limited to competitive and non-competitive assay systems using techniques such as radioimmunoassay, ELISA (enzyme linked immunosorbent assay), "sandwich" immunoassays, immunoradiometric assays, gel diffusion precipitin reactions, immunodiffusion assays, in situ immunoassays (using colloidal gold, enzyme or radioisotope labels), western blot analysis, precipitation reactions, agglutination assays (e.g., gel agglutination assays, hemagglutination assays), complement fixation assays, immunofluorescence assays, protein A assays, immunoelectrophoresis assays, etc. In one embodiment, antibody binding is detected by detecting a label on the primary antibody. In another embodiment, the primary antibody is detected by detecting binding of a secondary antibody or reagent to the primary antibody. In a further embodiment, the secondary antibody is labeled. Many means are known in the art for detecting binding in an immunoassay and are within the scope of the present invention.

Progeny of the transgenic animals may be obtained by mating the transgenic animal with a suitable partner, or by in vitro fertilization of eggs and/or sperm obtained from the transgenic animal. Where mating with a partner is to be performed, the partner may or may not be transgenic and/or a knockout; where it is transgenic, it may contain the same or a different knockout, or both. Alternatively, the partner may be a parental line. Where in vitro fertilization is used, the fertilized embryo may be implanted into a surrogate host or incubated in vitro, or both. Using either method, the progeny may be evaluated using methods described above, or other appropriate methods.

In some embodiments a cell and/or animal described herein can comprise one or more additional modifications or transgenes (e.g. an additional transgene and/or second knockout targeting a gene other than Mrap2). Cells and/or animals described herein containing more than one knockout construct and/or more than one transgene expression construct can be prepared in any of several ways. A typical manner of preparation is to generate a series of mammals, each containing one of the desired transgenic phenotypes. Such animals are bred together through a series of crosses, backcrosses and selections, to ultimately generate a single animal containing all desired knockout constructs and/or expression constructs, where the animal is otherwise congenic (genetically identical) to the wild type except for the presence of the knockout construct(s) and/or transgene(s).

Described herein are nucleic acid molecules comprising a modification of Mrap2 as described herein, e.g. a targeting vector comprising a modified variant of Mrap2 according to the embodiments described herein. Described herein are cells produced by the process of transforming the cell with any of the described nucleic acids. Described herein are cells produced by the process of transforming the cell with any of the non-naturally occurring described nucleic acids. Described herein are peptides produced by the process of expressing any of the described nucleic acids. Described herein are any of the non-naturally occurring peptides produced by the process of expressing any of the described nucleic acids. Described herein are any of the described peptides produced by the process of expressing any of the non-naturally occurring described nucleic acids. Described herein are animals produced by the process of transfecting a cell within the animal with any of the nucleic acid molecules described herein. Described herein are animals produced by the process of transfecting a cell within the animal any of the nucleic acid molecules described herein, wherein the animal is a mammal. Described herein are animals produced by the process of adding to the animal any of the cells described herein.

In one aspect, described herein is a method of producing an animal for use. An animal can be used for, e.g. competition, breeding, products obtained from a live animal (e.g. milk from dairy animals or fibers from, e.g. a sheep or an alpaca), or work (e.g. a draft animal). Regardless of what use the animal is to serve, there will be a desired minimum size for that animal to have achieved prior to being used. By minimizing the time it takes for the animal to grow to that size, resources and time are conserved, lowering the cost of producing the useful animal.

In some embodiments described herein, the carcass of the animal is to be used. The carcass of the animal can be used for human consumption as well as for other purposes (e.g. to produce leather, fertilizer, cosmetics, drugs, hair products, perfumes, gelatin, glue, etc). In some embodiments, described herein is a method of producing an animal for use of the carcass, the method comprising providing species-appropriate food to an animal as described herein and butchering the animal when it reaches the desired size for use. Species-appropriate foods and desired size(s) for use of any of the animals described herein, e.g. are well known in the art, see, e.g. Pond et al. "Basic Animal Nutrition and Feeding" 5th Edition, Wiley, Hoboken, NJ, 2004 or Marshall et al. "Animal Feeding and Nutrition" 11th Edition, Kendall Hunt Publishing 2012. Where the animal is to be butchered (i.e. "slaughtered"), the desired size can be referred to in the art as "slaughter weight" or "finish weight." By way of non-limiting example, a pig is typically butchered when it weighs from about 240 to about 280 pounds and dietary recommendations are well-known in the art, e.g. Albrecht et al. "Swine Feeding Suggestions" Circular 509; Clemson Extension; (1995). Alternatively, species-appropriate food can be purchased commercially, e.g. PURINA MILLS™ NATURE'S MATCH™ Sow and Pig Complete Feed, Cat No. 0034068; Purina, St. Louis, MO.

In some embodiments, the animal as described herein can reach the desired size for use as much as 80% earlier than a wild-type animal, e.g. 10% earlier, 20% earlier, 30% earlier, 40% earlier, 50% earlier, 60% earlier, 70% earlier, or 80% earlier. In some embodiments, the animal as described herein can reach the desired size for use as much as 50% earlier than a wild-type animal. In some embodiments, the animal as described herein can be butchered as much as 80% earlier than a wild-type animal, e.g. 10% earlier, 20% earlier, 30% earlier, 40% earlier, 50% earlier, 60% earlier, 70% earlier, or 80% earlier. In some embodiments, the animal as described herein can be butchered as much as 50% earlier than a wild-type animal. In some embodiments described herein, the animal can be provided feed substantially *ad libitum.* The animal is a pig.

In some embodiments, the pig can be butchered at from about 50 to about 150 days of age. In some embodiments, the pig can be butchered at from about 75 to about 125 days of age. In some embodiments, the pig can be butchered at from 75 to 125 days of age.

In some embodiments, the animal, the pig can be provided a restricted level of food as compared to a wild-type animal. On a restricted diet, an animal as described herein can reach a given desired size for use in the same amount of time as the wild-type animal. In some embodiments, the restricted level of food is from about 60% to about 95% of the level provided to a wild-type mammal. In some embodiments, the restricted level of food is from about 80% to about 95% of the level provided to a wild-type mammal.

For convenience, the meaning of some terms and phrases used in the specification, examples, and appended claims, are provided below. Unless stated otherwise, or implicit from context, the following terms and phrases include the meanings provided below. The definitions are provided to aid in describing particular embodiments, and are not intended to limit the claimed invention, because the scope of the invention is limited only by the claims. Unless otherwise defined, all technical and scientific terms used herein have the same meaning as commonly understood by one of ordinary skill in the art to which this invention belongs. If there is an apparent discrepancy between the usage of a term in the art and its definition provided herein, the definition provided within the specification shall prevail.

For convenience, certain terms employed herein, in the specification, examples and appended claims are collected here.

The terms "decrease", "reduced", "reduction", or "inhibit" are all used herein to mean a decrease by a statistically significant amount. In some embodiments, "reduce," "reduction" or "decrease" or "inhibit" typically means a decrease by at least 10% as compared to a reference level (e.g. the absence of a given treatment) and can include, for example, a decrease by at least about 10%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 98%, at least about 99% , or more. As used herein, "reduction" or "inhibition" does not encompass a complete inhibition or reduction as compared to a reference level. "Complete inhibition" is a 100% inhibition as compared to a reference level.

As used herein, a "transgenic animal" refers to an animal to which exogenous DNA has been introduced. In most cases, the transgenic approach aims at specific modifications of the genome, e.g., by introducing whole transcriptional units into the genome, or by up- or down-regulating pre-existing cellular genes. The targeted character of certain of these procedures sets transgenic technologies apart from experimental methods in which random mutations are conferred to the germline, such as administration of chemical mutagens or treatment with ionizing solution.

As used herein, "knock-out" refers to partial or complete suppression of the expression of a protein encoded by an endogenous DNA sequence in a cell. The "knock-out" can be affected by targeted deletion of the whole or part of a gene encoding a protein in an cell. In some embodiments, the deletion may prevent or reduce the expression of the functional protein in any cell in the whole animal in which it is normally expressed. For example, a "Mrap2 knock-out animal" refers to an animal in which the expression of functional Mrap2 has been reduced or suppressed by the introduction of a recombinant modifier that introduces a modification in the sequence of the Mrap2 gene. A knock-out animal can be a transgenic animal, or can be created without transgenic methods, e.g. by transient introduction of a TALENs molecule, such that a deletion of part or all of the Mrap2 gene occurs, but without the introduction of exogenous DNA to the genome.

The term "animal" is used herein to include all vertebrate animals, except humans. It also includes an individual animal in all stages of development, including embryonic and fetal stages. The term "germ cell line transgenic animal" refers to a transgenic animal in which the genetic alteration or genetic information was introduced into a germ line cell, thereby conferring the ability to transfer the genetic information to offspring. If such offspring in fact possess some or all of that alteration or genetic information, they are transgenic animals as well. The term "chimera," "mosaic," "chimeric animal" and the like, refers to a transgenic and/or knock-out animal with exogenous DNA and/or a modification of Mrap2 in some of its genome-containing cells.

The term "heterozygote," "heterozygotic mammal" and the like, refers to a transgenic and/or knock-out animal with exogenous DNA and/or a modification of Mrap2 on one of a chromosome pair in all of its genome-containing cells. The term "homozygote," "homozygotic mammal" and the like, refers to a transgenic mammal with with exogenous DNA and/or a modification of Mrap2 on both members of a chromosome pair in all of its genome-containing cells.

The term "homologous recombination" refers to the exchange of DNA fragments between two DNA molecules or chromatids at the site of homologous nucleotide sequences. The term "gene targeting" refers to a type of homologous recombination that occurs when a fragment of genomic DNA is introduced into a mammalian cell and that fragment locates and recombines with endogenous homologous sequences. Gene targeting by homologous recombination employs recombinant DNA technologies to replace specific genomic sequences with exogenous DNA of particular design and/or a modified sequence.

The term "native expression" refers to the expression of the full-length polypeptide encoded by a gene, e.g., a Mrap2 gene, at expression levels present in the wild-type cell and/or animal. Thus, a disruption in which there is "no native expression" of the endogenous gene, e.g., a Mrap2 gene, refers to a partial or complete reduction of the expression of at least a portion of a polypeptide encoded by an endogenous gene, e.g., an endogenous Mrap2 gene, of a single cell, selected cells, or all of the cells of an animal.

The expression "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers. Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

As used herein, the term "marker" refers to a reporter, a positive selection marker, and a negative selection marker. A reporter refers to any molecule the expression of which in a cell produces a detectable signal, e.g., detectable signal, e.g., luminescence. Exemplary markers are disclosed in, e.g., Sambrook, J., et al., (2001) Molecular Cloning: A Lbaroratory Manual, 2nd ed. (2001), Cold Spring Harbor Laboratory Press, Cold Spring Harbor, N.Y.; and, U.S. Pat. No. 5,464,764 and No. 5,625,048. Many procedures for selecting and detecting markers are known in the art. See, e.g., Joyner, A. L., (2000) Gene Targeting: A Practical Approach, 2nd ed., Oxford University Press, New York, N.Y. As used herein, the terms "selective marker" and "position selection marker" refer to a gene, the expression of which allows cells containing the gene to be identified, e.g., antibiotic resistance genes and detectable, e.g., fluorescent, molecules. In certain embodiments of the invention, the gene encoding a product enables only the cells that carry the gene to survive and/or grow under certain conditions. For example, plant and animal cells that express the introduced neomycin resistance (Neo^{r}) gene are resistant to the compound G418. Cells that do not carry the Neo^{r} gene marker are killed by G418. Other positive selection markers are known to, or are within the purview of, those of ordinary skill in the art. A "negative selectin marker" refers to a gene, the expression of which inhibits cells containing the gene to be identified, e.g., the HSV-tk gene.

As used herein, the terms "protein" and "polypeptide" are used interchangeably herein to designate a series of amino acid residues, connected to each other by peptide bonds between the alpha-amino and carboxy groups of adjacent residues. The terms "protein", and "polypeptide" refer to a polymer of amino acids, including modified amino acids (e.g., phosphorylated, glycated, glycosylated, etc.) and amino acid analogs, regardless of its size or function. "Protein" and "polypeptide" are often used in reference to relatively large polypeptides, whereas the term "peptide" is often used in reference to small polypeptides, but usage of these terms in the art overlaps. The terms "protein" and "polypeptide" are used interchangeably herein when referring to a gene product and fragments thereof. Thus, exemplary polypeptides or proteins include gene products, naturally occurring proteins, homologs, orthologs, paralogs, fragments and other equivalents, variants, fragments, and analogs of the foregoing.

As used herein, the term "nucleic acid" or "nucleic acid sequence" refers to any molecule, preferably a polymeric molecule, incorporating units of ribonucleic acid, deoxyribonucleic acid or an analog thereof. The nucleic acid can be either single-stranded or double-stranded. A single-stranded nucleic acid can be one nucleic acid strand of a denatured double- stranded DNA. Alternatively, it can be a single-stranded nucleic acid not derived from any double-stranded DNA. In one aspect, the nucleic acid can be DNA. In another aspect, the nucleic acid can be RNA. Suitable nucleic acid molecules are DNA, including genomic DNA or cDNA. Other suitable nucleic acid molecules are RNA, including mRNA.

An "isolated" nucleic acid molecule is a nucleic acid molecule that is identified and separated from at least one contaminant nucleic acid molecule with which it is ordinarily associated in the natural source of the nucleic acid. An isolated nucleic acid molecule is other than in the form or setting in which it is found in nature. Isolated nucleic acid molecules therefore are distinguished from the nucleic acid molecule as it exists in natural cells.

The term "gene" refers to (a) a gene containing a DNA sequence encoding a protein, e.g., Mrap2; (b) any DNA sequence that encodes a protein, e.g., Mrap2 amino acid sequence, and/or; (c) any DNA sequence that hybridizes to the complement of the coding sequences of a protein. In certain embodiments, the term includes coding as well as noncoding regions, and preferably includes all sequences necessary for normal gene expression.

The term "target gene" (alternatively referred to as "target gene sequence" or "target DNA sequence") refers to any nucleic acid molecule, polynucleotide, or gene to be modified by, e.g. homologous recombination or a meganuclease-mediated method of modification. The target sequence can include, e.g. an intact gene, an exon or intron, a regulatory sequence or any region between genes. The target gene may comprise a portion of a particular gene or genetic locus in the individual's genomic DNA.

The term "statistically significant" or "significantly" refers to statistical significance and generally means a two standard deviation (2SD) or greater difference.

Other than in the operating examples, or where otherwise indicated, all numbers expressing quantities of ingredients or reaction conditions used herein should be understood as modified in all instances by the term "about." The term "about" when used in connection with percentages can mean ±1%.

As used herein the term "comprising" or "comprises" is used in reference to compositions, methods, and respective component(s) thereof, that are essential to the method or composition, yet open to the inclusion of unspecified elements, whether essential or not.

The term "consisting of' refers to compositions, methods, and respective components thereof as described herein, which are exclusive of any element not recited in that description of the embodiment.

As used herein the term "consisting essentially of' refers to those elements required for a given embodiment. The term permits the presence of elements that do not materially affect the basic and novel or functional characteristic(s) of that embodiment.

The singular terms "a," "an," and "the" include plural referents unless context clearly indicates otherwise. Similarly, the word "or" is intended to include "and" unless the context clearly indicates otherwise. Although methods and materials similar or equivalent to those described herein can be used in the practice or testing of this disclosure, suitable methods and materials are described below. The abbreviation, "e.g." is derived from the Latin exempli gratia, and is used herein to indicate a non-limiting example. Thus, the abbreviation "e.g." is synonymous with the term "for example."

Definitions of common terms in cell biology and molecular biology can be found in The Encyclopedia of Molecular Biology, published by Blackwell Science Ltd., 1994 (ISBN 0-632-02182-9; Benjamin Lewin, Genes X, published by Jones & Bartlett Publishing, 2009 (ISBN-10: 0763766321); Kendrew et al. (eds.), , Molecular Biology and Biotechnology: a Comprehensive Desk Reference, published by VCH Publishers, Inc., 1995 (ISBN 1-56081-569-8) and Current Protocols in Protein Sciences 2009, Wiley Intersciences, Coligan et al., eds.

Unless otherwise stated, the present invention was performed using standard procedures, as described, for example in Sambrook et al., Molecular Cloning: A Laboratory Manual (3 ed.), Cold Spring Harbor Laboratory Press, Cold Spring Harbor, N.Y., USA (2001); Davis et al., Basic Methods in Molecular Biology, Elsevier Science Publishing, Inc., New York, USA (1995); Current Protocols in Protein Science (CPPS) (John E. Coligan, et. al., ed., John Wiley and Sons, Inc.), Current Protocols in Cell Biology (CPCB) (Juan S. Bonifacino et. al. ed., John Wiley and Sons, Inc.), and Culture of Animal Cells: A Manual of Basic Technique by R. Ian Freshney, Publisher: Wiley-Liss; 5th edition (2005), Animal Cell Culture Methods (Methods in Cell Biology, Vol. 57, Jennie P. Mather and David Barnes editors, Academic Press, 1st edition, 1998).

Other terms are defined herein within the description of the various aspects of the invention.

All patents and other publications; including literature references, issued patents, published patent applications, and co-pending patent applications; cited throughout this application are for the purpose of describing and disclosing, for example, the methodologies described in such publications that might be used in connection with the technology described herein. These publications are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

The description of embodiments of the disclosure is not intended to be exhaustive or to limit the disclosure to the precise form disclosed. While specific embodiments of, and examples for, the disclosure are described herein for illustrative purposes, various equivalent modifications are possible within the scope of the disclosure, as those skilled in the relevant art will recognize.

The technology described herein is further illustrated by the following examples which in no way should be construed as being further limiting.

### EXAMPLES

### EXAMPLE 1: Loss of Function of the Melanocortin 2 Receptor Accessory Protein 2 Is Associated with Mammalian Obesity

Melanocortin receptor accessory proteins (MRAPs) modulate signaling of melanocortin receptors *in vitro.* To investigate the physiological role of brain-expressed Melanocortin 2 Receptor Accessory Protein 2 (MRAP2), mice with whole body and brain-specific targeted deletion of *Mrap2* were characterized, both of which develop severe obesity at a young age. Mrap2 interacts directly with Melanocortin 4 Receptor (Mc4r), a protein previously implicated in mammalian obesity, and it enhances Mc4r-mediated generation of the second messenger cyclic AMP, suggesting that alterations in Mc4r signaling may be one mechanism underlying the association between *Mrap2* disruption and obesity. In a study of humans with severe, early-onset obesity, four rare, potentially pathogenic genetic variants in *MRAP2* were found, suggesting that the gene may also contribute to body weight regulation in humans.

Membrane-expressed G protein-coupled receptors (GPCRs) modulate cellular responses to numerous physiological stimuli. The melanocortin receptors (MCRs) are a subfamily of GPCRs that mediate signaling in response to the pro-opiomelanocortin-derived peptides, adrenocorticotropic hormone (ACTH) and α-melanocyte-stimulating hormone (αMSH) and their competitive antagonists, agouti and agouti-related protein. The MCRs mediate a diverse range of physiological functions: MC1R is involved in skin pigmentation, MC2R plays a critical role in the hypothalamic-pituitary-adrenal axis, MC3R and MC4R are involved in energy homeostasis and MC5R is implicated in exocrine function (*1*).

There is increasing recognition that accessory proteins can modulate GPCR trafficking, as well as ligand binding and signaling (*2*). An accessory protein for MC2R, MC2R accessory protein (MRAP), is required for the trafficking of MC2R to the surface of adrenal cells and for signaling in response to ACTH (*3, 4*). Loss of either MC2R or MRAP in humans causes severe resistance to ACTH, with resulting glucocorticoid deficiency (*5*, *6*).

All mammals have a paralogous gene, *MRAP2,* which, like *MC3R* and *MC4R,* is predominantly expressed in the brain (*7*), most prominently in the pons and cerebellum, but including in regions involved in energy homeostasis such as the hypothalamus and brainstem (Figs. 4A-4C). Within the paraventricular nucleus of the hypothalamus (PVN), Mrap2 and Mc4r mRNAs are co-expressed in many cells (data not shown). The inventor hypothesized that Mrap2 might modulate signaling through a melanocortin receptor and potentially affect energy homeostasis. Targeted deletion of *Mrap2* in mice was performed, using Cre-lox-mediated excision of the 100 bp exon 3 (which encodes the highly conserved transmembrane domain (*7*)) to create mice with normal levels of an mRNA predicted to encode a truncated protein that includes the first 55 amino acids of Mrap2, with the transmembrane domain replaced by 11 aberrant amino acids specified by the out-of-frame exon 4, followed by a stop codon (Figs. 4D-4G). Normal levels of the mutant mRNA indicate preservation of Mrap2-containing neurons in null mice, although these neurons likely do not express the predicted mutant protein, since mutant Mrap2 mRNA, but not protein, is present in cells transfected with the same *Mrap2* mutant construct used to create the null mice (Fig. 4H).

*Mrap2* null mice appeared normal at birth, with normal weight gain and post-weaning food intake during early life (0-32 days and 23-32 days, respectively), although young *Mrap2-*/*-* male mice trended toward greater weight and food intake with advancing age (Fig. 4I). However, null mice of both genders gradually became extremely obese on a diet of regular chow *ad libitum* (Fig. 1A, 5A). Heterozygous mice were significantly heavier than wild-type animals on standard chow (160-175 days; males, *Mrap2*^{+/+} 26.0±0.4 g, *Mrap2*^{+/-} 29.9±0.9 g; females *Mrap2*^{+/+} 24.5±0.9 g, *Mrap2*^{+/-} 28.1±0.7 g), and at younger ages (56-95 days) on a high fat diet (Fig. 1A). In addition, *Mrap2*^{-/-} mice had increased length (Fig. 4J) and per cent of weight due to fat, and decreased per cent of weight due to lean mass (Fig. 4K). Both genders of *Mrap2*^{-/-} mice had increased visceral adiposity, over twice the normal white adipose tissue cell size, enlarged brown adipose tissue depots, normal liver histology on a regular chow diet, but much greater hepatic steatosis compared with wild-type mice on a high fat diet (Fig. 1B, Fig. 5A).

Adult *Mrap2* null mice had, as expected, elevated leptin concentrations corresponding to their increased fat mass, which normalized with diet-induced weight normalization (Fig. 5B). Obese adult mice had normal fasting insulin (Fig. 5C) and normal tolerance to intraperitoneal glucose injection (Fig. 5D). Mrap2 has been postulated to play a role in the adrenal response to ACTH (*8*). Diurnal rhythmicity and stress responsiveness of the adrenal axis was measured in *Mrap2* null mice, which were normal (Fig. 5E). Thyroid hormone levels were also normal (Table 2). Epinephrine and norepinephrine excretion were reduced in male *Mrap2*^{-/-} mice only (Fig. 5F), but Ucp1 mRNA concentrations increased appropriately in both genders of null mice following exposure to 4°C for 18h (Fig. 5G). Hypothalamic Agrp mRNA concentration was reduced in Mrap2 null mice, whereas Pomc mRNA was normal (Fig. 5H).

To characterize the mechanisms underlying the obesity in these mice, food intake was measured under a variety of conditions. At 42 (Fig. 5I) and 84 (Fig. 5J) days of age, when *Mrap2*^{-/-} mice were clearly overweight, no difference in food intake was detected between the two genotypes when analyzed over a 4-day interval. Obesity was not caused by more efficient absorption of calories in null mice (Fig. 5K). Only when monitored daily over 50 days (ages 34-84 days) was a subtle increase in cumulative food intake discernable in the null animals (Fig. 2A), with the onset of obesity preceding hyperphagia (Fig. 2A and Fig. 5L). To further understand the contribution of hyperphagia to obesity in *Mrap2*^{-/-} mice, their food intake was limited to that amount consumed by their normal siblings (pair feeding). Even when fed the same amount of chow, null mice gained more weight than did wild-type mice (Figs. 5M-5N). Only when the amount of food intake in null mice was further restricted to 10% (females) and 13% (males) less than that of wild-type mice was there equivalent weight gain (Fig. 5O) in the two genotypes. To determine whether the late onset hyperphagia in *Mrap2*^{-/-} mice (Fig. 2A) could simply be the consequence of an increased body mass at this older age caused by a separate metabolic defect, null mice were switched to *ad libitum* access to chow after 40 days of restricted feeding (upward arrow, Fig. 5O). During the first 24 h of *ad libitum* feeding, food intake almost doubled in null mice (from 2.9±0.1 to 5.6±0.5 g/d in males, and from 2.8±0.1 to 5.3±0.2 g/d in females), with a corresponding marked increase in body weight. Thus, hyperphagia develops in an age-dependent manner in older mice, independent of body weight. Consistent with this, young (age 38-45 days) *Mrap2*^{-/-} mice had an intact anorectic response to the melanocortin receptor (Mc4r and Mc3r) agonist, MTII (Fig. 5P), corresponding to their normal *ad libitum* food intake at this age.

It was hypothesized that young *Mrap2*^{-/-} mice might display abnormal energy expenditure because obesity develops early during *ad libitum* feeding prior to the onset of hyperphagia, persists in mutant mice pair-fed to a normal dietary intake, and is abolished only by underfeeding. To explore this, energy expenditure and respiratory exchange ratio (RER) were measured by indirect calorimetry, as well as locomotor activity and core body temperature, in young (30-45 days of age) wild-type and *Mrap2* null mice, just as their weights began to diverge (Fig. 2A). Surprisingly, the wild-type and mutant mice had indistinguishable 24h total energy expenditure, as analyzed by ANCOVA (*9*) (Fig. 2B). There were also no differences between *Mrap2*^{+/+} and *Mrap2*^{-/-} mice in RER (Fig. 5Q), locomotor activity (Fig. 5R), or core body temperature at 22°C (Fig. 5S), with both genotypes exhibiting the expected increase in all three parameters during the active night period. Following exposure to 4°C for 18h, null and wild-type mice became significantly hypothermic to the same extent (Fig. 5S).

Since 1) MRAP is essential for signalling through MC2R (*3, 4*), 2) MRAP's paralog, *Mrap2,* is expressed principally in the brain, and 3) Mc2r's paralog, Mc4r, has a key role in energy balance in Sim1-containing neurons (*10*), it was asked whether deletion of *Mrap2* causes obesity in part by altering signaling through centrally-expressed Mc4r. We created a *Siml^{Cre}*::*Mrap2*^{*fox*/*flox*} mouse with conditional deletion of *Mrap2* exclusively in these neurons, and expression of Mrap2*^{Del}* mRNA only in hypothalamus and not cerebral cortex or brainstem (Fig. 3A). Like global null mice, conditional mutants were similarly obese (Fig. 3B), and pair-feeding to a normal dietary intake only partially reversed their obesity (Fig. 6A).

If Mrap2 facilitates the action of Mc4r, then Mc4r deficiency should create an equivalent or more severe obesity phenotype than does Mrap2 deficiency, depending on the degree to which Mrap2 interferes with Mc4r function. Supporting this, Mrap2^{+/-} mice of both genders were less obese than either Mc4r^{+/-} or doubly heterozygous mice (Fig. 6B). The differences between Mc4r^{+/-} and doubly heterozygous mice were not statistically significant, although the latter trended toward being heavier. Among homozygous knockouts, those with Mc4r deficiency alone were more obese than those with Mrap2 deficiency alone (Fig. 6B). The *Mc4r* knockout mice were more obese than mice with deletion of both *Mc4r* and *Mrap2* (in males, with a trend in females), suggesting that Mrap2 may promote weight gain through both Mc4r-dependent and Mc4r-independent actions.

To determine whether mouse Mrap2 and Mc4r can interact directly, transiently expressed, N-terminally Myc-tagged Mrap2 and N-terminally GFP-tagged Mc4r were co-immunoprecipitated in CHO cells (devoid of endogenous Mrap, Mrap2 and melanocortin receptors). It was found that mouse Mrap2 and Mc4r interact (Fig. 6C), consistent with previous data (*7*). The impact of Mrap2 on Mc4r (Fig. 3C) and Mc3r (Fig. 6D) signalling was investigated. The combined expression of Mc4r and Mrap2 in CHO cells suppressed basal PKA signaling compared with Mc4r alone (Fig. 3C, left panel), as previously reported with the human orthologs (*7*). But in contrast to that report (which used NDP-MSH), it was found that αMSH caused a 5-fold increase above basal PKA activity (Fig. 3C, right panel) compared with less than a 2-fold increase with Mc4r alone or Mc4r plus the *Mrap2* null construct, *Mrap2delE3* (*in vitro* model for *in vivo* disruption of *Mrap2*). The presence of Mrap2 increased signaling through Mc3r at the two highest αMSH doses (Fig. 6C). These findings suggest Mrap2 may alter signalling through Mc4r and perhaps other receptors.

To investigate whether alterations in MRAP2 are associated with human obesity, the coding region and intron/exon boundaries of *MRAP2* in obese and control individuals from the Genetics of Obesity Study (GOOS) cohort (*11*) and the Swedish obese children's cohort (*12*) were sequenced. Four rare heterozygous variants were found in unrelated, nonsyndromic, severely obese individuals, that were absent from cohort-specific controls and 1000 genomes (Table 1), with all but one in the C-terminal region of the protein (Fig. 7). In three of these subjects, no pathogenic variants were found in the coding region or intron/exon boundaries of all known nonsyndromic human obesity genes (Table 3). Only one of the variants (E24X) is clearly disruptive, and overall few rare variants were found in the obese cohorts, indicating that *MRAP2* mutations, if they contribute to severe human obesity, do so rarely.

In summary, it is demonstrated herein that global or brain-specific inactivation of Mrap2 causes obesity in mice and that rare heterozygous variants in *MRAP2* are associated with early-onset, severe obesity in humans. The mechanism(s) by which Mrap2 exerts its effects on body weight regulation remain to be firmly established but likely involve altered signaling through Mc4r and perhaps other melanocortin receptors.

### References and Notes:

1. R. D. Cone, Studies on the physiological functions of the melanocortin system. Endocrine reviews 27, 736 (Dec, 2006).
2. D. L. Hay, D. R. Poyner, P. M. Sexton, GPCR modulation by RAMPs. Pharmacology & therapeutics 109, 173 (Jan, 2006).
3. P. M. Hinkle, J. A. Sebag, Structure and function of the melanocortin2 receptor accessory protein (MRAP). Molecular and cellular endocrinology 300, 25 (Mar 5, 2009).
4. S. N. Cooray, A. J. Clark, Melanocortin receptors and their accessory proteins. Molecular and cellular endocrinology 331, 215 (Jan 15, 2011).
5. L. A. Metherell et al., Mutations in MRAP, encoding a new interacting partner of the ACTH receptor, cause familial glucocorticoid deficiency type 2. Nature genetics 37, 166 (Feb, 2005).
6. L. F. Chan, L. A. Metherell, A. J. Clark, Effects of melanocortins on adrenal gland physiology. Eur J Pharmacol 660, 171 (Jun 11, 2011).
7. L. F. Chan et al., MRAP and MRAP2 are bidirectional regulators of the melanocortin receptor family. Proc Natl Acad Sci U S A 106, 6146 (Apr 14, 2009).
8. J. A. Sebag, P. M. Hinkle, Regulation of G protein-coupled receptor signaling: specific dominant-negative effects of melanocortin 2 receptor accessory protein 2. Science signaling 3, ra28 (2010).
9. M. H. Tschop et al., A guide to analysis of mouse energy metabolism. Nature methods 9, 57 (Jan, 2012).
10. N. Balthasar et al., Divergence of melanocortin pathways in the control of food intake and energy expenditure. Cell 123, 493 (Nov 4, 2005).
11. S. Farooqi, S. O'Rahilly, Genetics of obesity in humans. Endocrine reviews 27, 710 (Dec, 2006).
12. L. E. Johansson et al., Genetic variance in the adiponutrin gene family and childhood obesity. PLoS One 4, e5327 (2009).
13. J. Y. Altarejos et al., The Creb1 coactivator Crtcl is required for energy balance and fertility. Nature medicine 14, 1112 (Oct, 2008).
14. J. T. Halley, T. S. Nelson, L. K. Kirby, Z. B. Johnson, Relationship between dry matter digestion and metabolizable energy. Poultry science 64, 1934 (Oct, 1985).
15. D. J. Adams et al., A genome-wide, end-sequenced 129Sv BAC library resource for targeting vector construction. Genomics 86, 753 (Dec, 2005).
16. P. Liu, N. A. Jenkins, N. G. Copeland, A highly efficient recombineering-based method for generating conditional knockout mutations. Genome research 13, 476 (Mar, 2003).
17. T. D. Schmittgen, K. J. Livak, Analyzing real-time PCR data by the comparative C(T) method. Nature protocols 3, 1101 (2008).
18. H. Liu et al., Transgenic mice expressing green fluorescent protein under the control of the melanocortin-4 receptor promoter. J Neurosci 23, 7143 (Aug 6, 2003).

**Table 1. MRAP2 variants detected in obese subjects and controls**

| **MRAP2 variant** | **Subjects with variant** | ***Subject Sex/ Age/BMI/BMI SDS** | **Controls with variant** | ****MAF: European American** | ****MAF: African American** | *****Polyphen prediction** |
|---|---|---|---|---|---|---|
| E24X | 1/488 | M/19/63/4.7 | 0/488 | 0.000% (0/8600) | 0.000% (0/4406) | damaging |
| N88Y | 1/376 | M/11/29.6/3.3 | 0/376 | 0.000% (0/8600) | 0.000% (0/4406) | possibly damaging |
| L115V | 1/488 | M/5/24/4.2 | 0/488 | 0.012% (1/8600) | 0.000% (0/4406) | benign |
| R125C | 1/488 | F/8/29/3.5 | 0/488 | 0.047% (4/8600) | 0.045% (2/4406) | possibly damaging |

| | | | | | | |
|---|---|---|---|---|---|---|
| *Subject Sex (Male[M], Female[F])/Age (y)/BMI (kg/m²)/SDS (standard deviation score) **MAF (minor allele frequency, NHLBI exome variant server http://evs.gs.washington.edu/EVS/) ***Polyphen (http://genetics.bwh.harvard.edu/pph2/) | | | | | | |

### EXAMPLE 2: Materials and Methods

*Animals.* Mice were housed at 22°C unless otherwise noted, on a 12 h:12 h light:dark cycle with lights on at 0700. Mice were fed either standard rodent chow (Isopro RMH 3000, PMI Nutrition International, St. Louis, MO) or high fat diet chow (D12451, Research Diets, New Brunswick, NJ). *Ad libitum* food intake was measured in individually-housed mice as the difference in weight between food in a suspended food bin, accounting for food spillage onto the cage floor on subsequent days, measured either daily for up to 55 days, or in group-housed mice (4/cage of the same gender) over 4 days. For pair feeding of individually-housed mice, the average amount of food consumed each day by wild-type mice was given to the experimental genotype of the same gender on the subsequent day. Body weights were measured at the same time as was food intake. For restricted feeding, between 10-15% less than the average amount of food consumed each day by wild-type mice was given to the experimental genotype of the same gender on the subsequent day, to maintain the same body weights between the two groups. The effect of melanotan II (MTII: acetyl-(Nle⁴, Asp⁵, D-Phe⁷, Lys¹⁰)-cyclo-□-MSH (4-10) amide acetate salt, Bachem Americas, Torrance, CA) on food intake was carried out in individually-housed mice as described (*13*). Food was removed from mice at 1800. Saline or MTII (2µg/g body weight) were administered by intraperitoneal injection at 1830. Pre-weighed food was provided at 1900, and was re-weighed at 2030. Feces (for energy measurement) were collected over a 24 h period from the cage floor of individually-housed mice and lyophilized at 22°C for 16-24 h until the feces weight had stabilized. Energy of feces and chow was measured by using a Parr Adiabatic Calorimeter (*14*) in two separate groups of animals and laboratories (University of Arkansas and North Carolina State University) with identical results. Urine (for catecholamine measurements) was collected into tubes cooled in Dry Ice over a 24 h period from mice individually-housed in metabolic cages. Response to intraperitoneal injection of glucose (2 g/kg body weight) was performed in the morning after a 12 h fast from 1900 to 0700 by measuring tail venous blood using a clinical glucometer. Core body temperature was measured using either a handheld rectal probe (YSI model 43 telethermometer with series 500 probe, Yellow Springs Instrument Co., Yellow Springs, OH) inserted 1 cm beyond the anus for 1 min in conscious mice following exposure to 4°C, or a subcutaneously-implanted microchip transponder (DAS-5002, BioMedic Data Systems, Seaford, DE) to measure the diurnal temperature rhythm during exposure to 22°C. Total energy expenditure and respiratory exchange ratio were measured in either *ad libitum* or food-restricted mice by indirect calorimetry using an Oxymax Comprehensive Lab Animal Monitoring System (CLAMS, Columbus Instruments, Columbus, OH). A 24 h acclimation period preceded 48 h of measurement (sampling once per 17 minutes) in the calorimetry cages, in which locomotor activity was also measured by the rate of interruption of a light-photocell circuit (sampling once per minute). Lean and fat mass were measured by dual energy xray absorptiometry (DEXA, Lunar PIXImus Densitometer, Fitchburg, WI). Phlebotomy was performed by retro-orbital venipuncture without anesthesia, with the interval between touching the mouse cage and phlebotomy being less than 60 sec. Diurnal rhythmicity and stress responsiveness of blood corticosterone was performed by sequential phlebotomy at 1900, 0800, and following 30 min of restraint stress, at 0830, in mice fed a high fat diet. All mouse protocols were approved by the Boston Children's Hospital Animal Care and Use Committee.

*Creation of Mrap2*^{-/-} *and Mrap2*^{*flox*/}*^{flox} mice.* Both *Mrap2*^{-/-} and *Mrap2*^{flox/flox} mice were generated using a common targeting vector and recombineering (*15, 16*). All the required plasmids (PL253, PL452, and PL451) and recombinogenic bacteria strains (EL350) were obtained from David Conner (Harvard Medical School Department of Genetics). The 129Sv BAC clone (bMQ-392015) containing *Mrap2* (accession number: XM_147017, which had been replaced by NM_001101482) in DH10B was obtained from the Sanger Institute (http://www.sanger.ac.uk/). Primers were designed to create miniarms for PL253 to retrieve the *Mrap2* genomic region, miniarms for PL452 for the upstream LoxP insertion, and miniarms for PL451 for the downstream FRT-Neo-FRT-LoxP insertion. The final targeting vector was linearized with NotI, electroporated into 129Sv ES cells, and selected with G418 (Invitrogen, Carlsbad, CA). G418-resistant ES cell lines were isolated and analyzed by long range PCR using LA Taq™ (TAKARA, Otsu, Japan), plus external *Mrap2* genomic primers and internal vector primers at both 5' and 3' ends to confirm by DNA sequence analysis the correct 5' and 3' incorporation of the construct into the proper *Mrap2* genomic locus. To generate *Mrap2*^{+/-} ES cells, clones were transfected with pOG231 that encodes CMV-Cre. To generate Mrap2^{+/flox} ES cells, clones were transfected with pCAGGS-Flpe. Validated ES cells were karyotyped and surgically implanted into C57BL/6 pseudo-pregnant female mice. Male chimeric animals were obtained and crossed with 129S6/SvEvTac female mice (Taconic, Hudson, NY). F1 heterozygous male mice were crossed with F1 heterozygous female mice to generate F2 colonies on a pure 129 background. These mice were then bred to produce wild-type (*Mrap2*^{+/+}), heterozygous (*Mrap2*^{+/-}) and null (*Mrap2*^{-/-}*)* mice for subsequent studies, at the expected frequencies of 1:2:1. The *Mrap2* null allele was also backcrossed for 10 generations onto a C57BL/6J (Jackson Laboratories, Bar Harbor, ME) background. *Mrap2* null mice have normal fertility.

*Creation of Sim1^{CreBAC}::Mrap2*^{*f*/}*^{f} mice. Mrap2*^{flo/flox} mice were mated with *Siml^{CreBAC}* mice (006451, Jackson Laboratories, Bar Harbor, ME) to produce *Sim1^{CreBAC}::Mrap2*^{*f*/*f*} mice. These mice were bred to *R26*^{*flox*-*stop*-*tdTomato*} reporter mice (007576, Jackson Laboratories, Bar Harbor, ME), in which tdTomato expression is induced by Cre-mediated recombination, to map Sim1-Cre expression in the brain. Site-specific deletion of *Mrap2* exon 3 in brain was also assessed by *Mrap2*-specific PCR of genomic DNA and RT-PCR of RNA in parietal cortex, hypothalamus, and brain stem (Table 2). Body weight and food intake of *Mrap2*^{flox/flox} and *Sim1^{CreBAC}::Mra2*^{*f*/f} siblings were measured daily from ages 35-89. Mice were fed *ad libitum* until age 70 days, when one half of the *Sim1^{CreBAC}::Mrap2*^{*f*/f} mice were switched to be pair-fed to the food intake of the *ad libitum-*fed *Mrap2*^{flox/flox} mice.

*Creation of mice with combined Mrap2 and Mc4r deficiency. Mc4r* null mice (006414, mixed C57BL/6-129 background, Jackson Laboratories, Bar Harbor, ME) were mated with *Mrap2* null mice to create mice with heterozygous and homozygous combinations of knockout of both genes. Body weights were measured daily from ages 28-84 days in littermate siblings as described above.

*Histology.* Tissues were collected and immersed into 4% paraformaldehyde. Sections (5-7 µm) of paraffin-embedded tissues were cut and stained with hematoxylin and eosin. Images were captured using a Nikon Eclipse 800™ microscope. All images of a given tissue were recorded at the same magnification and exposure conditions, without subsequent manipulation.

*RT-PCR.* Total RNA was isolated from approximately 50 mg of tissue/cells using TRIZOL™ Reagent (Invitrogen, Carlsbad, CA). RNA samples were treated with DNase (6.8 Kunitz units/sample) (Qiagen, Valencia, CA) to eliminate genomic DNA contamination and were purified using the RNeasy™ Mini kit (Qiagen) according to the manufacturer's protocol. Purified RNA samples were subjected to reverse-transcription using iScript™ cDNA Synthesis kit (Biorad, Hercules, CA). For conventional RT-PCR, 50 ng RNA equivalent-cDNA was PCR-amplified for 35 cycles using gene-specific primer sets following the program: 94°C 2 min, (94 °C 10 sec, 60 °C 30 sec) x 35 cycles, 72 °C 7 min. PCR products were electrophoresed on 2.0 % agarose gels stained with ethidium bromide. Real-time qPCR was carried out using the same gene-specific primer sets and using iQ SYBR green Supermix (Biorad) following the program: 95 °C 3 min, (95 °C 10 sec, 60 °C 40 sec) x 45 cycles and the ΔΔCT method (*17*). Gene-specific primer sets are listed in Table 4.

*Double-label ³⁵S*/*digoxigenin (DIG) in situ hybridization.* Tissue sections (10 µm) were thaw-mounted on SuperFrost™ slides (Fisher Scientific, Pittsburgh, PA) and stored at -20°C. Before hybridization, sections were fixed in 4% formaldehyde in phosphate buffered saline (PBS) for 15 min on ice. The 521 bp *Mc4r* template plasmid for *in situ* hybridization (*18*) and cRNA was labeled with ³⁵S-UTP (Perkin Elmer, Waltham, MA). For the *Mrap2* probe template, a 443 bp-*Mrap2* cDNA PCR fragment (Table S1) was cloned into PCRII TOPO™ (Invitrogen, Carlsbad, CA), and a cRNA antisense probe was labeled with digoxigenin (DIG) using DIG RNA Labeling Mix™ (Roche) and T7 RNA polymerase (Roche) according to the manufacturer's protocol. cRNA probes were denatured at 90°C for 3 min before adding to the hybridization solution containing 50 % deionized formamide, 10 % dextran sulfate, 0.5 M NaCl, 1x Denhardt's solution, 10 mM Tris (pH 8), 1 mM EDTA (pH 8), 500 µg/ml yeast tRNA, 10mM DTT. Sections were pretreated with 2x SSC (0.3 M sodium chloride, 0.03 M sodium citrate) for 2 min, 0.1 M triethanolamine (TEA) for 2 min, 0.25% acetic anhydride in 0.1 M TEA for 10 min, and 2x SSC for 2 min in order, all at room temperature (RT). Sections were dehydrated in ascending concentrations of ethanol, and were subjected to de-lipidation in chloroform for 5 min. Sections were rinsed in 95% ethanol for 2 min and air-dried. Approximately 150 µl of the hybridization solution was spread over the sections and incubated overnight at 65°C. After hybridization, sections were soaked in 2x SSC/1 mM dithiothreithol (DTT) for 15 min at 22°C twice, followed by RNase digestion solution (20 µg/ml RNAase A (Roche), 0.5 M NaCl, 10 mM Tris (pH8), and 1 mM EDTA) for 30 min at 37 °C. Sections were washed in 2x SSC/1 mM DTT for 10 min at 22°C, 0.5x SSC/1 mM DTT for 10 min three times at RT, 0.5x SSC/1 mM DTT at 65 °C for 30 min, 2x SSC for 2 min at 22°C. Sections were incubated in blocking solution (2x SSC, 2 % normal sheep serum, 0.05 % Triton X-100) for 2h at 22°C, rinsed in GB1 buffer (100 mM Tris-HCl, pH 7.5, 150 mM NaCl) for 5 min twice, and incubated in anti-DIG-Alkaline Phosphatase antibody solution (100 mM Tris-HCl, pH 7.5, 150 mM NaCl, 1 % normal sheep serum, 0.3 % Triton X-100, anti-DIG-Alkaline Phosphatase, Roche, Indianapolis, IN) overnight. Sections were washed with GB1 for 10 min, GB3 (100 mM Tris-HCl, pH 9.5, 100 mM NaCl, 50 mM MgCl₂-6H₂0) for 10 min, and incubated in levamisole solution (250 mg levamisole hydrochloride (Sigma, St. Louis, MO) in 250 ml GB3 for 5 min. Chromagen solution was prepared by mixing 7.5 ml GB3, 2.5 ml levamisole solution, 33.8 ml of 100 mg/ml Nitroblue tetrazolium (NBT) chloride solution (Roche), and 35 ml of 50 mg/ml 5-Bromo-4-chloro-3-indolyl-phosphate (BCIP) solution (Roche). Sections were soaked in chromagen solution in a glass Coplin jar wrapped with aluminum foil for 48 hours. The color reaction was stopped by rinsing with 10 mM Tris-HCl, pH 8.0, 1 mM EDTA, pH8.0 twice, and 4 % PFA in 1x PBS, and distilled water. Sections were air-dried completely, coated with 2 % Parlodion in amyl acetate (SPI Supplies, West Chester, PA) and air-dried. Sections were dipped in pre-warmed silver emulsion NTB2 diluted 1:1 with distilled water (Kodak, Rochester, NY) at 42°C. Dipped and air-dried sections were placed in slide boxes, wrapped with aluminum foil, and stored for 1 month at 4°C. Sections were developed by soaking the slides in developer D-19 (Kodak, Rochester, NY) for 4 min, by rinsing in distilled water for 10 sec and by fixing with Professional Fixer (Kodak) for 5 min. Sections were rinsed in distilled water, dried and coverslipped.

*Hormone Measurements.* Leptin and insulin were measured by ELISA (Crystal Chem, Downers Grove, IL) using the manufacturer's protocol. Corticosterone was measured by radioimmunoassay (MP Biomedicals, Santa Ana, CA). Urine and norepinephrine were measured by HPLC using a C-18 reverse phase column followed by electrochemical detection.

*Co-immunoprecipitation and Western blotting.* CHO cells (ATCC, Manassas, VA) were grown in F12 media supplemented with 10% fetal bovine serum and 1% penicillin/streptomycin. Plasmids (Mrap2-pCDNA-Myc-His and Mc4r-pEGFP-N1) were introduced into CHO cells by transient transfected using FuGENE 6™ (Promega, Madison, WI). Lysates were prepared 24 hours after transfection by washing cells in phosphate-buffered saline and sonicating them in RIPA buffer (40mM Hepes pH 7.4, 2mM EDTA, 10mM sodium pyrophosphate, 10mM sodium glycerophosphate, 1% sodium deoxycholate, 0.3% CHAPS, 0.1% SDS with protease inhibitors). For immunoprecipitation, lysates were incubated overnight at 4°C with anti-GFP (290, Abcam, Cambridge, MA) or anti-Myc (9106, Abcam, Cambridge, MA) antibodies. Protein A Sepharose beads (CL-4B17-0963-03, GE Healthcare Biosciences, Pittsburgh, PA) were added and incubated for 2 hours at 4°C. Beads were washed with high salt (150mM NaCl) lysis buffer, supernatant was removed, and beads were resuspended in SDS loading buffer (Laemmle). To detect the Myc epitope, samples were heated for 5 min at 100°C. To detect Mc4r-GFP, samples were not heated. Proteins were resolved by SDS-PAGE. Western blotting was performed using anti-GFP or anti-Myc primary antibodies and HRP-linked Protein A secondary antibody (NA9120, GE Healthcare Biosciences, Pittsburgh, PA).

*Mc4r bioassay for Mrap2 effect.* The coding sequences of mouse *Mrap2* and *Mc4r* were isolated by PCR of brain cDNA (*Mrap2*) or genomic DNA (*Mc4r*) (Table S4), subcloned into TOPO-pCRII™ (Invitrogen, Carlsbad, CA), sequence-verified, and ligated into pRC/CMV (Invitrogen, Carlsbad, CA). *Mrap2delE3,* identical to the product of *Mrap2*^{-/-} mice, was also created and ligated into pRC/CMV. A 6:1 ratio of *Mrap2:Mc4r* DNA (3µg *Mrap2*:0.5µg *Mc4r*) was transfected into 0.5 million CHO cells (ATCC, Manassas, VA) in a 60mm dish, along with 1µg *pCRE-Luc,* (Clontech, Mountain View, CA) and 05µg of *pRL-TK* (renilla luciferase, Promega, Madison, WI) using 20µL Fugene HD™ (Promega). On the next day, transfected cells (0.12 million cells / well) were seeded on 12-well plates. 24 h later, following 7 h of serum starvation, cells were treated with α-melanocyte stimulating hormone (αMSH, M4135-1MG, Sigma, St. Louis, MO), 0-10 nM, for 5 hours followed by luciferase assay.

*Expression of Mouse Mrap2 in HEK293 cells.* HEK293 cells (ECACC, DS Pharma Biomedical, Osaka, Japan) were transfected with nothing, empty vector, *Mrap2,* or *Mrap2delE3* expression constructs (Table 4), followed by RT-PCR of *Mrap2* mRNA (using the same *Mrap2-*specific RTPCR primers used in Fig.1A) and 18S rRNA, or western blotting of Mrap2 protein using either anti-mouse Mrap2³⁻¹² mouse monoclonal antibody (Mrap2 mAb-NH₂) directed against the amino-terminus (amino acids MSAQRLASNR (SEQ ID NO: 64)) or anti-rat Mrap2¹⁸⁹⁻²⁰⁴ rabbit polyclonal antibody (Mrap2 rAb-COOH) directed against the carboxyl-terminus (amino acids LLENKPVSQTARTDLD (SEQ ID NO: 65)) (*7*). Western blots were then stripped and reprobed with anti-beta actin antibody (Sigma, A1978) at a dilution of 1:5,000.

*Human Studies.* The Genetics of Obesity Study (GOOS) **(*11*)** protocol was approved by the Anglia and Oxford multiregional ethics committee. Each subject, or parent (children <16 years), provided written informed consent (oral consent was obtained from the minors themselves). Clinical studies were conducted in accordance with the principles of the Declaration of Helsinki. The Swedish obese children cohort has been described previously (*12*) and consists of a group of 376 obese children and adolescents (age 6-21 years, BMI >30 kg/m² or +3 SDS) referred to the National Childhood Obesity Centre at the Karolinska University Hospital and Karolinska Institute, and 376 non-obese adolescents (age 15-20 years, BMI 20-25 kg/m²) recruited from 17 upper secondary schools around Stockholm to match the obese group with respect to ethnicity and socioeconomic status and asked to participate by their school nurse. The study was approved by the Regional Committee of Ethics, Stockholm, and all subjects gave their informed consent. DNA sequence analysis for *MRAP2* and nine other genes associated with severe obesity (*LEP, LEPR, POMC, MC4R, PC1*/3, *BDNF, TRKB, SIM1,* and *SH2B1*) was performed using di-deoxynucleotide (Sanger) methodology.

*Statistical Analysis.* Data were analyzed by Student's t-test or by ANOVA followed by Bonferroni's post hoc test, as appropriate for comparison or two or more samples respectively (Statview 5.0, SAS Institute Inc.). For energy expenditure, the effect of body weight on total energy expenditure in *Mrap2*^{+/+} and *Mrap2*^{-/-} mice of each gender was compared using analysis of covariance (SAS, SAS Institute Inc.) (*9*). All data are shown as the mean ± SEM, with p< 0.05 considered statistically significant.

**Table 2. Thyroid hormone concentrations in Mrap^{+/+} and Mrap2^{-/-} mice**

| | **Male** | | **Female** | |
|---|---|---|---|---|
| | ***Mrap2*^{+/+}** | ***Mrap2*^{-/-}** | ***Mrap2*^{+/+}** | ***Mrap2*^{-/-}** |
| **T4 [µg/dL]** | 3.3±0.3 | 3.2±0.6 | 2.5±0.3 | 3.2±0.4 |
| **T3 [ng/dL]** | 8.5±1.1 | 10.5±1.6 | 11.7±4.8 | 11.1±4.4 |

**Table 3. Variants in other obesity-associated genes in obese subjects with rare MRAP2 variants**

| | **E24X (het)** | **L115V (het)** | **R125C (het)** |
|---|---|---|---|
| **LEP** | WT | WT | WT |
| **LEPR** | c.668A>G, p.Q223R (het), rs1137101; c. 1029T>C, p.S343S (het), rs1805134 | c.668A>G, p.Q223R (het), rs1137101 | c.668A>G, p.Q223R (het), rs1 137101; c.3057G>A, p.P1019P (het), rs1805096 |
| **POMC** | WT | WT | WT |
| **MC4R** | WT | WT | WT |
| **PC1/3** | c. 1650 T>C, p. N550N, rs6233 (hom) | WT | c. 1650 T>C, p. N550N, rs6233 (Het); c. 1993 C>G, p. Q665E, rs6234 (Het); c. 2069 G>C, p. S690T, rs6235 (Het) |
| **BDNF** | c. 196 G>A, p. V66M (het) rs6265; c. 450 A>G, p. A150A, (hom) rs2353512 | c. 196 G>A, p. V66M (het), rs6265 | c. 450 A>G, p. A150A (hom) rs2353512 |
| **TRKB** | WT | WT | WT |
| **SIM1** | c.1054C>A, p.P352T (het), rs3734354; c.1112C>T, p.A371V (het), rs3734355 | WT | WT |
| **SH2B1** | c. 1450 A>G, p. T484A (hom), rs7498665 | c. 1450 A>G, p. T484A (hom), rs7498665 | WT |

**Table 4. Oligonucleotide primers used in this study**

| **Function of oligonucleotide** | **DNA sequence** | **SEQ ID NO:** |
|---|---|---|
| | | |

| ***1. Generation of Mrap2 null and flox mice*** | | |
|---|---|---|
| *1.1 Retrieving vector* | | |
| PL253 miniarm1 F | 5' -GCGGCCGCCAAACAAAAAAAGGTATTTGG-3' | 5 |
| PL253 miniarm1 R | 5'-ACTAGTCTACAGTATGTATTAC-3' | 6 |
| PL253 miniarm2 F | 5'-ACTAGTTACTATAGTATCATTA-3' | 7 |
| PL253 miniarm2 R | 5'-GGATCCGTTCTTGGTCTTGCTCAGAAA-3' | 8 |

| *1.2 Upstream loxP insertion* | | |
|---|---|---|
| PL452 miniarm1 F | 5' -GTCGACA TGTGAAGCGTGGACCAAAA-3' | 9 |
| PL452 miniarm1 R | 5'-GAATTCATTCATGTTCATGTTGACAC-3' | 10 |
| PL452 miniarm2 F | 5'-GGATCCGAAGTGGAGTAAATTCAATC-3' | 11 |
| PL452 miniarm2 R | 5' -GCGGCCGCGCACAGGTGCCCCGCCCACC-3' | 12 |

| *1.3 Downstream loxP insertion* | | |
|---|---|---|
| PL451 miniarm1 F | 5'-GTCGACCGCGATGTTTCTTTAGCATA-3' | 13 |
| PL451 miniarm1 R | 5'-GAATTCTCACGAGGGCAAAAACCAGG-3' | 14 |
| PL451 miniarm2 F | 5'-GGATCCAATTAGGTGTTTTGGTTCTT-3' | 15 |
| PL451 miniarm2 R | 5'-GCGGCCGCCTTTTCTTACTATTAAAAAT-3' | 16 |

| *1.4 PL253 multiple cloning site (MCS) sequencing* | | |
|---|---|---|
| PL253 MCS seq F | 5'-GCAAGGCGATTAAGTTGGG-3' | 17 |
| PL253 MCS seq R | 5'-GGAAACATTCCAGGCCTGG-3' | 18 |

| *1.5 Neo ES cells long PCR validation* | | |
|---|---|---|
| *1.5.1 5'-end* (7109 bp) | | |
| 5P external F | | 19 |
| 5P PL452 internal R | | 20 |

| *1.5.2 3'-end* (8954 bp) | | |
|---|---|---|
| 3P Neo internal F | | 21 |
| 3P external R | | 22 |

| *1.6 Null mouse screening PCR* (1200 bp for WT allele, and 789 bp for null allele) | | |
|---|---|---|
| Null screen F | 5'-ATGTGAAGCGTGGACCAAAATGCTGAGA-3' | 23 |
| Null screen R | 5'-TTCTCTAGCTCCGGACATCTCAAGCACA-3' | 24 |

| *1.7 Flox mouse screening PCR* | | |
|---|---|---|
| *1.7.1 Upstream loxP* (814 bp for WT allele, and 912 bp for flox allele) | | |
| Flox screen up F | 5'-GTCGACATGTGAAGCGTGGACCAAAA-3' | 25 |
| Flox screen up R | 5'-GCGGCCGCGCACAGGTGCCCCGCCCACC- 3' | 26 |

| *1.7.2 Downstream loxP* (814 bp for WT allele, and 918 bp for flox allele) | | |
|---|---|---|
| Flox screen down F | 5'-GTCGACCGCGATGTTTCTTTAGCATA-3' | 27 |
| Flox screen down R | 5'-GCGGCCGCCTTTTCTTACTATTAAAAAT-3' | 28 |

| *1.8 Null mouse long PCR validation* | | |
|---|---|---|
| *1.8.1 5'-end* (7100 bp) | | |
| 5P external F | | 29 |
| 5P null internal R | | 30 |

| *1.8.2 3'-end* (8734 bp) | | |
|---|---|---|
| 3P null internal F | | 31 |
| 3P external R | | 32 |

| *1.9 Flox mouse long PCR validation* | | |
|---|---|---|
| *1.9.1 5'-end* (7109 bp) | | |
| 5P external F | | 33 |
| 5P PL452 internal R | | 34 |

| *1.9.2 3'-end* (8745 bp) | | |
|---|---|---|
| 3P PL451 internal F | | 35 |
| 3P external R | | 36 |

| *1.10 Null*/*Flox mice genotyping* (210 bp for WT allele, 314 bp for flox, and 400 bp for null) | | |
|---|---|---|
| Mrap2 genotype F1 | 5'-TCAGCCTCCGTGTCAGAGCTGG-3' | 37 |
| Mrap2 genotype F2 | 5'-GGCATGGATTGCTACCCAGATGCAGGT-3' | 38 |
| Mrap2 genotype R | 5'-GGCTCAACTCTGCAAACACGTCAGCGT-3' | 39 |
| | | |

| ***2. Mrap2* / *Mc4r RT-PCR and qRT-PCR*** | | |
|---|---|---|
| *2.1 Mrap2 and Mc4r* | | |
| Mouse Mrap2 (exon4-5 spanning), amplicon 150 bp, accession number NM 001101482.2 | | |
| Mrap2 e4-5 F | 5'-TGCTGACTTTGCTGACGAAGACAGGTG-3' | 40 |
| Mrap2 e4-5 R | 5'-TGGACTCCTCGTTGCCCTGACG-3' | 41 |
| | TAGGCCATGAGGCCCATCCAGT | |
| Mouse Mc4R, 171 bp, NM 016977.3 | | |
| Mc4r F | 5'-AGGCGGGTCGGGATCATCATAAGTTGTA-3' | 42 |
| Mc4r R | 5'- CCTCGCCATCAGGAACATGTGGACATAGA -3' | 43 |

| Mouse/Human 18S ribosomal RNA, 70 bp, NR 003278 | | |
|---|---|---|
| 18S F | 5'-AGTCCCTGCCCTTTGTACACA-3' | 44 |
| 18S R | 5'-CGATCCGAGGGCCTCACTA-3' | 45 |

| *2.2 Mrap2 null validation* (Fig. 1C) | | |
|---|---|---|
| Mouse Mrap2 (exon3-4), 247 bp for WT, 147 bp for null, NM 001101482.2 | | |
| Mrap2 nul val F | 5'-GTCTGCCCAGAGGCTGGCTT-3' | 46 |
| Mrap2 nul val R | 5'-CCTCTCTGAGGACTCCGCGT-3' | 47 |

| *2.3 Mrap2 exon1a identification* | | |
|---|---|---|
| Mouse Mrap2 (exon1-2), 175 bp for variant 1, NM 001101482.2 | | |
| Mrap2 e1a F | 5'-AGCACCGGAGGAGCTGAACC-3' | 48 |
| Mrap2 e1a R | 5'-CAGTCCTTCAAAGGAAACTGGTCCG-3' | 49 |
| | | |

| ***3. Ucp1, Agrp, Pomc qRT-PCR*** | | |
|---|---|---|
| *3.1 Mouse uncoupling protein 1 (Ucp1), 89 bp, NM 009463.3* | | |
| Ucp1 F | 5'- GGATTGGCCTCTACGACTCA -3' | 50 |
| Ucp1 R | 5'-TAAGCCGGCTGAGATCTTGT -3' | 51 |

| *3.2 Mouse agouti related protein (Agrp), 100 bp, NM 007427.3* | | |
|---|---|---|
| Agrp F | 5'-TGTGTAAGGCTGCACGAGTC-3' | 52 |
| Agrp R | 5'-GGCAGTAGCAAAAGGCATTG-3' | 53 |

| *3.3 Mouse proopiomelanocortin (Pomc), 150 bp, NM 008895* | | |
|---|---|---|
| Pomc F | 5'-GAGTTCAAGAGGGAGCTGGA-3' | 54 |
| Pomc R | 5'- GGTCATGAAGCCACCGTAAC -3' | 55 |
| | | |

| ***4. Mrap2* / *Mc4r in situ hybridization*** | | |
|---|---|---|
| Mrap2 probe template plasmid for nonradioactive ISH: 443 bp-Mrap2 cDNA: | | |
| Mrap2 ISH probe F | 5'-TTTGCTGACGAAGACAGGTG-3' | 56 |
| Mrap2 ISH probe R | 5'-CAGTCCAGGTCTATGCGTGA-3' | 57 |
| | | |

| ***5. Mouse Mrap2 luciferase assay*** | | |
|---|---|---|
| *4.1 Mouse Mrap2 assay* | | |
| Mouse Mrap2 wild type, NM_001101482.2 | | |
| Mrap2 exp F | 5'-AAGCTTGCCACCATGGAGATGTCTGCCCAGAG-3' | 58 |
| Mrap2 exp R | 5'-GCGGCCGCTCAGTCCAGGTCTATGCGTG-3' | 59 |

| Mouse Mrap2 del exon3 mutant | | |
|---|---|---|
| Mrap2 delE3 F | | 60 |
| Mrap2 delE3 R | | 61 |
| Mouse Mc4r, NM_016977.3 | | |
| Mc4r exp F | 5'-AAGCTTGCCACCATGAACTCCACCCACCACCA-3' | 62 |
| Mc4r exp R | 5'-GCGGCCGCTTAATACCTGCTAGACAACT-3' | 63 |

## Claims

1. A method of producing a carcass of a pig, the method comprising:
providing pig-appropriate food to a knockout pig comprising an engineered modification of the Mrap2 gene, wherein the pig does not express a detectable level of functional Mrap2;
butchering the pig when it reaches slaughter weight.

2. The method of claim 1, wherein the pig is butchered as much as 80% earlier than a wild-type pig or wherein the pig is butchered as much as 50% earlier than a wild-type pig, and/or wherein the pig is provided feed substantially ad libitum.

3. The method of claim 1, wherein the pig is provided a restricted level of food compared to a wild-type pig, optionally wherein the restricted level of food is from about 60% to about 95% of the level provided to a wild-type pig or wherein the restricted level of food is from about 80% to about 95% of the level provided to a wild-type pig.

4. The method of any of claims 1-3, wherein the pig is butchered at from about 75 to about 125 days of age.

5. The method of any of claims 1-4, wherein the engineered modification is:
a deletion of at least exon 3 of the Mrap2 gene; or
a deletion of at least the transmembrane domain of Mrap2.

6. The method of any of claims 1-5, wherein the pig is heterozygous for the modification, or wherein the pig is homozygous for the modification.

## Patentansprüche

1. Verfahren zur Herstellung eines Schlachtkörpers eines Schweins, das Verfahren umfassend:
Versorgen eines Knockout-Schweins umfassend eine konstruierte Modifikation des Mrap 2 Gens mit an Schweine-angepasstem Futter, wobei das Schwein kein detektierbares Level an funktionalem Mrap 2 exprimiert;
Schlachten des Schweins, wenn es Schlachtgewicht erreicht.

2. Verfahren nach Anspruch 1, wobei das Schwein so viel wie 80 % früher geschlachtet wird als ein Wildtyp-Schwein oder so viel wie 50 % früher geschlachtet wird als ein Wildtyp-Schwein, und/oder wobei das Schwein im Wesentlichen ad libitum mit Futter versorgt wird.

3. Verfahren nach Anspruch 1, wobei das Schwein mit einer begrenzten Menge an Futter verglichen mit einem Wildtyp-Schwein versorgt wird, optional wobei die begrenzte Menge an Futter ungefähr 60 % bis ungefähr 95 % der Menge entspricht, mit der ein Wildtyp-Schwein versorgt wird oder wobei die begrenzte Menge an Futter ungefähr 80 % bis ungefähr 95 % der Menge entspricht, mit der ein Wildtyp-Schwein versorgt wird.

4. Verfahren nach einem der Ansprüche 1 bis 3, wobei das Schwein in einem Alter von ungefähr 75 bis ungefähr 125 Tagen geschlachtet wird.

5. Verfahren nach einem der Ansprüche 1 bis 4, wobei die konstruierte Modifikation:
eine Deletion von mindestens Exon 3 des Mrap2 Gens; oder
eine Deletion von mindestens der Transmembrandomäne des Mrap 2 ist.

6. Verfahren nach einem der Ansprüche 1 bis 5, wobei das Schwein für die Modifikation heterozygot ist, oder wobei das Schwein für die Modifikation homozygot ist.

## Revendications

1. Procédé de production d'une carcasse de porc, le procédé comprenant :
une fourniture d'une nourriture appropriée au cochon à un cochon knock-out comprenant une modification du gène Mrap2, dans lequel le cochon n'exprime pas un niveau détectable de Mrap2 fonctionnel ;
un abattage du cochon lorsqu'il atteint le poids d'abattage.

2. Procédé selon la revendication 1, dans lequel le cochon est abattu jusqu'à 80% plus tôt qu'un cochon de type sauvage ou dans lequel le cochon est abattu jusqu'à 50% plus tôt qu'un cochon de type sauvage, et/ou dans lequel il est fourni au cochon une alimentation sensiblement *ad libitum.*

3. Procédé selon la revendication 1, dans lequel il est fourni au cochon une teneur réduite en nourriture comparée à un cochon de type sauvage, optionnellement dans lequel la teneur réduite en nourriture est d'environ 60% à environ 95% de la teneur fournie à un cochon de type sauvage ou dans lequel la teneur réduite en nourriture est d'environ 80% à environ 95% de la teneur fournie à un cochon de type sauvage.

4. Procédé selon l'une quelconque des revendications 1 à 3, dans lequel le cochon est abattu à partir d'environ 75 à environ 125 jours d'âge.

5. Procédé selon l'une quelconque des revendications 1 à 4, dans lequel la modifcation est :
une délétion d'au moins de l'exon 3 du gène Mrap2 ; ou
une délétion d'au moins du domaine transmembranaire de Mrap2.

6. Procédé selon l'une quelconque des revendications 1 à 5, dans lequel le cochon est hétérozygote pour la modification, ou dans lequel le cochon est homozygote pour la modification.
